(19) Europäisches Patentamt
European Patent Office
Office européen des brevets

(11) EP 3 162 361 A1

(12) **EUROPEAN PATENT APPLICATION**

(43) Date of publication:
**03.05.2017 Bulletin 2017/18**

(51) Int Cl.:
***A61K 9/127*** *(2006.01)*

(21) Application number: **15192570.8**

(22) Date of filing: **02.11.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Universität für Bodenkultur Wien**
**1180 Wien (AT)**

(72) Inventors:
- **BIXNER, Oliver**
  **1190 Vienna (AT)**
- **SHIRMARDI SHAGHASEMI, Behzad**
  **1190 Vienna (AT)**
- **REIMHULT, Erik**
  **1190 Vienna (AT)**

(74) Representative: **Sonn & Partner Patentanwälte**
**Riemergasse 14**
**1010 Wien (AT)**

(54) **IMPROVED MAGNETICALLY REACTIVE VESICULAR BODIES**

(57) The invention provides a method of preparing a vesicular particle having at least in part a lipid and/or polymeric membrane that is a barrier between the interior and exterior of said vesicular particle, wherein said membrane comprises at least one inorganic core nanoparticle embedded in said membrane, said method comprises the steps of i) providing a first dispersion with one or more inorganic core particles having a hydrophobic dispersant shell, in a solution of membrane forming lipids and/or polymers in a non-aqueous solvent; and ii) introducing the first dispersion into a non-solvent for the membrane forming lipids and/or polymers, wherein the volume of the non-solvent exceeds the volume of the first dispersion, thereby forming the vesicular particles; the produced particle preparations and their uses.

EP 3 162 361 A1

## Description

### Field of the invention

The present invention relates to the field of nanoparticles embedded in membranes or coatings in vesicular structures.

### Background of the invention

Nanoparticle containing capsules have been proposed for many uses, including triggered drug delivery and imaging. Combining superparamagnetic iron oxide nanoparticles (SPIONs) with existing liposome drug delivery technology is an enticing prospect, but it requires efficient methods of synthesis and formulation compatible with pharmaceutical applications.

Large unilamellar liposomes (~100-200nm in diameter) comprise some of the most successful drug delivery systems in clinical use and are heavily researched for development of new drug delivery systems. The advantages of liposomes are manifold. Foremost, they possess a natural excellent biocompatibility; by virtue of their lipid composition they can be recycled by the body. Moreover, their vesicular structure enables transport of hydrophilic cargo in their large aqueous lumen as well as hydrophobic and amphiphilic drugs in the lipid bilayer.

The lipid membrane provides an effective impermeable barrier to charged or polar molecules and liposomes are therefore very efficient means of encapsulating a multitude of drugs over long time scales. The composition of the lipid membrane can be easily tuned, including addition of charged lipids for transfection and PEG-lipids to create so-called stealth liposomes with strongly reduced clearance rates in vivo. Additionally, easy functionalization of liposomes with bioactive tags can drastically increase the specificity to particular tissues or cells thereby significantly enhancing the range of therapeutic applications over traditional passive targeting mechanisms. The biocompatibility, however, brings about inherently low blood circulation times owing to rapid cleavage by phospholipases and a short shelf life, which debatably can be increased by PEGylation of a fraction of the lipids. This approach has been combined with bioactive labeling to demonstrate enhanced targeting through longer circulation and by augmenting vesicles with specific biological interactions.

An important consideration when applying liposomes and stealth liposomes for drug delivery is that efficient encapsulation and circulation can lead to inefficient or slow release. Rapid release at the site of action is desired to reach a concentration within the therapeutic range. Destabilizing the lipid membrane to increase its permeability, however, leads to premature drug release during circulation and short shelf life. The self-assembled nature of lipid membranes offers many possibilities to make the release profile dependent on changes in the environment, thereby utilizing them for stable encapsulation and circulation and letting a local change in the physical environment increase the release rate at the target.

Liposomes structurally including biocompatible superparamagnetic iron oxide nanoparticles (SPIONs) are an attractive alternative for such strategies. SPIONs, in contrast to most other nanoparticles, offer the advantage of being hydrolytically degraded into constituent nontoxic ions and are highly compatible with in vivo applications due to the low susceptibility of tissue to magnetic fields. Additionally, they offer the possibility to simultaneously image and remote control biodistribution via magnetic field gradients which makes them attractive as multipurpose tools for guided drug delivery and bioimaging.

US 2002/103517 A1 describes the use of magnetic nanoparticles to a patient to induce hyperthermia in a cell or tissue by applying a electromagnetic radiation. A treatment of cancer is proposed.

WO 2006/072943 relates to methods of forming metal particles in the lumen of a liposome. The lipid membranes of the liposomes do not contain metal particles. Various methods of creating liposomes are disclosed.

US 2007/154397 teaches polymer nanostructures with magnetic nanoparticles encapsulated in the polymer structure.

US 6,251,365 describes a magnetosome (magnetic liposome) with a magnetic monocrystal surrounded by a phospholipid membrane.

WO 2007/021236 describes a superparamagnetic core encapsulated in a heat sensitive coating with membrane disruptive agents for heat-induced delivery of a co-encapsulated substance to a cell.

US 2009/004258 describes a thermosensitive liposome for drug delivery containing paramagnetic iron oxide particles to generate heat and thereby cause leakage in the membrane.

WO 2012/001577 describes the formation of superparamagnetic iron particles from an oleate complex.

WO 2011/147926 describes stabilized magnetic nanoparticles embedded in a lipid bilayer membrane.

To date various preparation methods have been described for producing magnetoliposomes (or magnetosomes). Co-incorporation of water soluble SPIONs and pharmaceutical agents in the liposome lumen was first demonstrated. Major drawbacks have been shown for this approach. First, SPIONs that are not properly stabilized interact with

the liposome membrane and causes leakage, but properly stabilized SPIONs take up large volume. Second, heating by SPIONs in the lumen to induce a thermal transition requires heating of the entire environment to change the permeability of the membrane due to the high thermal transport of water.

In contrast, hydrophobic SPIONs embedded in the lipid bilayer were shown to directly act on the capsule wall rather than on the aqueous bulk, thereby allowing for effective release without strong heating of the surrounding environment when actuated by alternating magnetic fields. The drawback, however, is that the embedding efficiency heavily depends on particle size and density in the membrane, which, however, when lowered too much may adversely affect interaction with magnetic fields. Optimal magnetic liposome preparations therefore aim for high loading of monodisperse SPIONs, as large as can fit in the membrane, to maximize the efficiency of actuation; this requires a dense and stable hydrophobic coating. To date, control over high loading of monodisperse hydrophobic SPIONs in the membrane of liposomes has not been achieved.

Prior magnetosomes suffer from lack of stability both with and without heat induction (leakiness) of both the active agents and the magnetic particles themselves, or the triggered release have been weak. In addition, prior magnetosome preparations suffer from inhomogenous size distribution that hampers a physiological use, which requires that the magnetosomes have a homogeneous size, about 100 nm in diameter with little variation. The invention therefore has the goal to improve magnetosomes in these aspects.

## Summary of the invention

The invention relates to a method of preparing a vesicular particle having at least in part a lipid and/or polymeric membrane that is a barrier between the interior and exterior of said vesicular particle, wherein said membrane comprises at least one inorganic core nanoparticle embedded in said membrane, said method comprises the steps of i) providing a first dispersion with one or more inorganic core particles having a hydrophobic dispersant shell, in a solution of membrane forming lipids and/or polymers in a non-aqueous solvent; and ii) introducing the first dispersion into a non-solvent for the membrane forming lipids and/or polymers, wherein the volume of the non-solvent exceeds the volume of the first dispersion, thereby forming the vesicular particles.

The invention further relates to a composition of a plurality of vesicular particles each having at least in part a lipid and/or polymeric membrane that is a barrier between the interior and exterior of said vesicular particle, wherein said membrane comprises inorganic core nanoparticles embedded in said membrane, characterized in that A) said embedded inorganic core nanoparticles are in a concentration of at least 0.5 % (w/w per lipid and/or polymer), and wherein said concentration is constant or decreases by less than 25 % (percentage of w/w concentration) at least during 24 hours at standard conditions in an aqueous dispersion with physiological buffer; and/or B) said vesicular particles are formed by the inventive method.

Also provided is the use of the inventive composition in cosmetics, in medicine or as a contrast agent, by administration to a subject or to a cell or tissue culture.

The invention provides a facile way of producing small and large, unilamellar, and homogeneously sized magnetosomes with high content of monodisperse, hydrophobic inorganic core nanoparticle, such as SPIONs, integrated in the lipid or polymeric membrane by use of a simple bulk solvent inversion technique.

The following detailed disclosure reads on all aspects and embodiments of the present invention, irrespective of relating to a method, composition or use. E.g. described method steps also disclose that the resulting product can result in an element of the particle or composition, such as specific reagents used in the method may lead to a chemical group or moiety bound to the particle of the composition. Elements described for the composition can read on steps in the inventive manufacturing method that provides such elements. Also, the invention relates to a composition and all descriptions of particles also read on particles of said composition.

## Detailed description of the invention

The present invention provides an improved method to create vesicular particles with embedded nanoparticles inside the membrane and a composition of such improved particles. These particles have an improved stability and reduced loss of loading content and/or nanoparticles, and enhanced homogeneity. Furthermore, these particles can be easily re-sized, e.g. by sonication, to a similarly homogeneous yet smaller size (the latter especially in the case of mostly lipid membranes). In particular, they excel at their long-term stability with no or little loss of incorporated inorganic core nanoparticles over time. The vesicular particles with the magnetic nanoparticles are also referred to herein as "magnetosomes". However, the invention is not limited to magnetic nanoparticles and everything disclosed for the use of magnetic nanoparticles reads also on any other inorganic core nanoparticle to be embedded in the membrane, except where stated otherwise.

In addition to the novel full control over vesicle structure and nanoparticle loading, a major advantage of the method for vesicle assembly is that it is easily scalable while simultaneously compatible with direct drug encapsulation

methods. Vesicles in the ideal 100-200nm size range which provide a large lumen can be directly obtained; the large lumen is important for drug delivery efficiency. Furthermore, the much higher than previously obtained number of nanoparticles per vesicle that could be achieved is important for all applications. Magnetic contrast and susceptibility to magnetically triggered release is enhanced in direct proportion to the order of magnitude of higher loading, in case of magnetosomes.

The method uses a solvent inversion step, wherein a solution of the membrane forming components (lipids, amphiphilic polymers or both - in case of hybrid vesicles) and with dispersed inorganic core nanoparticles (hence this mixture is called dispersion) are introduced into a non-solvent of the membrane forming component. The non-solvent is preferably an aqueous solution, especially for physiological or biological applications. The non-solvent is also a non-solvent for the nanoparticles having a hydrophobic dispersant shell to improve localization into the membrane.

Herein, the inventive vesicular particles are also referred to as vesicles (even though not necessarily of biological substances), liposomes (if comprised of lipids) or polymersomes (if comprised mostly of polymers). They may be a lipid/polymer hybrid (of the same substances as described for lipids and polymer vesicles). In such a hybrid, the ratio of lipids to polymers may be 5:95 to 95:5, or 10:90 to 90:10, 20:80 to 80:20, 30:70 to 70:30, 40:60 to 60:40 (all w/w ratios). Also pure liposomes and pure polymersomes (i.e. without membrane forming polymers or lipids, respectively) are possible.

In step i) the inorganic core nanoparticles the lipids and/or polymers are mixed to form a mixture, called "first dispersion". Preferably, the inorganic core nanoparticles are magnetic core nanoparticles. In a preferment for all embodiments and aspects of the invention, the inorganic particle core comprises preferably a metal responsive to an external magnetic field. It is preferably selected from the group consisting iron, cobalt, zinc, cadmium, nickel, gadolinium, chromium, copper, manganese, terbium, europium, gold, silver, titanium, platinum, or any other element of the fourth row of the periodic table, or alloys thereof. In further embodiments the inorganic particle core comprises a metalloid, a semiconductor or consists of a non-metal material. Examples are Al, Si, Ge, or silica compounds. The inorganic nanoparticle core can be a nanocrystal or a multidomain crystallised nanoparticle composed of more than one nanocrystal. Preferably the core comprises an oxide any thereof, preferably a Fe oxide, such as $Fe_2O_3$ and/or $Fe_3O_4$. In a further embodiment, the inorganic nanoparticle core comprises a hydride nitride or an iron sulfide, preferably mixed oxide/hydroxide, nitride or sulfide of Fe (II) and/or Fe (III), e.g. in the form of a nanocrystal. Preferably, the inorganic nanoparticle core is $Fe_3O_4$ (magnetite) or comprises $Fe_3O_4$ spiked with any other metal, preferably those mentioned above. "Metal" as used herein refers to the element, not to the state. The metal may be metallic (with neutral charge) or, as in most case of the present invention, non-metallic, especially in case of crystallized cationic metals.

The inorganic core nanoparticles are particles with an inorganic core having a hydrophobic dispersant shell. The hydrophobic dispersant shell mediates localization in the membrane of the vesicular particle.

In further preferments of all inventive aspects and embodiments, the inorganic core is magnetic, especially paramagnetic, preferably superparamagnetic. This property can be achieved by using metal nanoparticles of a material as described above, especially selected from the group consisting of iron, cobalt or nickel, alloys thereof, preferably oxides or mixed oxides/hydroxides, nitrides, carbides or sulfides thereof. In a preferred embodiment the stabilized magnetic nanoparticles are superparamagnetic iron oxide nanoparticles (SPIONs). Magnetic particles allow controlled mobility, such as for separation of enrichment of particles in a non-accessible compartment, e.g. in a patient's body by applying a magnetic field, or the capability to heat the particles by applying an oscillating field, in particular by radio wave irradiation, e.g. in the range of 10 kHz to 1000 kHz, e.g. 400 kHz.

Such particles with the dispersant shell can be provided according to PCT/EP2015/068253 or Bixner et al., Langmuir, 2015, 31, 9198-9204, both incorporated herein by reference. Briefly, the inorganic core particles can be produced with a dispersant shell with dispersant molecules in a high surface covering density on the inorganic core, by the steps of: · providing one or more inorganic particles, · ligating at least one organic linker onto the inorganic particle, thereby obtaining an inorganic core linker coated particle, · providing at least one fluidized dispersant, preferably in form of a melt, suspension or solution, · binding the at least one fluidized dispersant to the at least one organic linker, thereby obtaining the inorganic core particles comprising a dispersant shell. Optimal reaction conditions aim at conditions to: (1) dissolve the reversibly bound surfactant (e.g. oleic acid), (2) maintain conditions for binding of the linker to the inorganic core, (3) fluidize the dispersant, e.g. PEG, (4) while keeping the dispersant in a low $R_G$ (low solubility or low coil volume) conformation. Such conditions are disclosed in the above cited references. Preferably, in this method, the temperature of the dispersant is raised above its melting temperature and binding is above the melting temperature. The dispersant can be a macromolecule, such as a macromolecule comprising a polymer, e.g. poly(N-isopropylacrylamide), polyisobutylene, caprolactone, polyimide, polythiophene, polypropylene, polyethylene, polyvinylpyrrolidone. The inorganic core particles may have an average size between 1 nm to 15 nm in diameter, especially preferred of 1.5 nm to 13 nm, or of 2 nm to 10 nm or of 2.2 nm to 8 nm or of 2.5 nm to 6 nm. In a further combinable preferment, the nanoparticles (the core or the core with the dispersant shell) are smaller than twice the length of the membrane forming amphiphiles in stretched conformation, preferably the nanoparticles are smaller than twice the length of the equilibrium size of the hydrophobic block of core of the membrane. Smaller particles help to avoid the formation of core-shell micelles, which do not possess

the lumen for encapsulation of compounds soluble in the bulk solvent.

The dispersant is preferably a macromolecule providing steric/osmotic colloidal stability in the preferred environment of the application, e.g. a polymer, such as polyisobutylene (PIB; e.g. in applications as polymer filler materials such as to produce impact resistant polypropylenes) or a hydrocarbon chain (for a lipid environment). Further dispersant polymers with preferred properties, uses and utilities are: polyoxazolines (including different thermoresponsive derivatives, for biomedical applications), poly(N-isopropylacrylamide) (thermoresponsive polymer, for biotechnological applications, separation, responsive membranes and drug delivery capsules), polyisobutylene (in applications as polymer filler materials such as to produce impact resistant polypropylenes), caprolactone (low melting point, biodegradable, biomedical applications), polyimide (very resistant, KEVLAR, filler material impact resistant materials), polythiophene (conductive polymers, smart materials applications), polypropylene/polyethylene (filler materials). A macromolecule is a very large molecule commonly, but not necessarily, created by polymerization of smaller subunits. The subunits of the macromolecule or polymer may be homogenous or heterogenous. Preferred dispersants comprise hydrocarbon groups, which encompass any polymers soluble in organic solvents. Typically, "hydrocarbon chains" include linear, branched or dendritic structures. Different forms of hydrocarbon chains may differ in molecular weights, structures or geometries (e.g. branched, linear, forked hydrocarbon chains, multifunctional, and the like). Hydrocarbon chains for use in the present invention may preferably comprise one of the two following structures: substituted or unsubstituted $-(CH_2)_m-$ or $-(CH_2)_n-Het-(CH_2)_o-$, dendrimers of generations 1 to 10 where m is 3 to 5000, n and o are independently from another 1 to 5000 and Het is a heteroatom, wherein the terminal groups and architecture of the overall hydrocarbon chains may vary. E.g. in the final particle there will be an anchor group which is formed by the linker molecule. This description includes any linear or branched hydrocarbon chains with ratios of unsaturated : saturated bonds varying from 0 : 100 to 100 : 0. In some embodiments the hydrophobic spacer comprises e.g. > 50% of subunits that are $-CH_2-$. In alternative or combined embodiments at least 10% of the carbon atoms, e.g. 10% to 50%, more preferred 20% to 40%, of the hydrocarbon chains are substituted by a heteroatom. Heteroatoms may be selected from O, N, S or N, preferably O. Side chain substitutions can be at a C or at Het with the substituents being selected independently from heterosubstituted or non-heterosubstituted, branched or unbranched, saturated or unsaturated hydrocarbons with 1 to 20 atoms, preferably 2 to 10, especially preferred 2 to 6 atoms in length.

The dispersant may have an average mass of 50 Da to 30 kDa, preferably of 200 Da to 1 kDa, especially preferred of 250 Da to 400 Da.

Preferably, high surface densities of bound dispersant molecules on the nanoparticle are used, e.g. at least 1.1, preferably at least 1.2, even more preferred at least 1.3, at least 1.4, at least 1.5, at least 2, at least 2.5, at least 2.8, at least 2.9, at least 3, at least 3.1, at least 3.2, at least 3.3 or at least 3.4, dispersant molecules per $nm^2$ of the inorganic core surface.

Preferably the inorganic core nanoparticles have a homogenic size wherein the mean standard deviation of said average size is at most 10%, preferably at most 5%, even more preferred at most 2% of the particle's average size, such as at most 0.8 nm, preferably at most 0.5 nm. Such particles may be synthesized as is (e.g. the cores provided without size separation) or selected after size separation.

The standard deviation (SD) measures the amount of variation or dispersion from the average. The standard deviation of size distribution is the square root of its variance.

In preferred embodiments the inorganic core particles comprise dispersant molecules bound to the particle surface, that (a) are at an average density of at least 1.1, preferably at least 3.0, dispersant molecules per $nm^2$ of the inorganic core surface, and/or (b) form a shell of constant dispersant density and a further shell of gradually reduced dispersant density with increasing distance from the inorganic core surface. Such a shell is obtained by the methods described in PCT/EP2015/068253 or Bixner et al., 2015, supra). The shell structure can be identified by small angle x-ray scattering in a solvent, e.g. water, and a solvable shell, distinct from the dense inner polymer shell.

The inorganic core nanoparticles can be labelled, either at the core or in the dispersant shell or at both sites. Such a label may be a radiolabel an electromagnetic responding label (e.g. if the core is not by itself magnetic) or a photoreactive label, preferably a chromophore or fluorescent label.

The lipids and/or polymers of the first dispersion, that later form part of the vesicular particle's membrane, can be any known in the art for vesicles. Preferably lipids are used, at least in part, such as at least 10%, at least 20%, at least 30%, at least 40%, at least 50%, at least 60% or at least 70% (w/w) of the membrane forming parts of the dispersion or in the membrane of the vesicles. The lipid can be zwitterionic. As used herein a lipid may be a neutral lipid, a cationic lipid or an anionic lipid, preferred are anionic lipids. Preferably the lipids comprise one or more saturated or mono- or polyunsaturated free fatty acids of 10-24, more preferably 16-18, carbon atoms, and esters and amides thereof. Example fatty acid ester groups are selected from palmitoyl-, lauryl-, myristoyl-, stearoyl-, oleoyl-, decyl-, arachidyl- groups or linolenic acid or linoleic acid esters. Preferably the lipid is a triglyceride. It may comprise one or two fatty acid esters or the like (e.g. sphingosine) and a phosphor ester group. The two fatty acid groups can be selected independently from the above. They may be different or the same. Preferably the lipids are phospholipids, e.g. selected from the group consisting of phosphatidylcholines, cardiolipins, phosphatidylethanolamines, spingomyelin, lysophosphatidylcholine,

phosphatidylserine, phosphatidylinositol, phosphatidylglycerol, and phosphatidic acid. Especially preferred, the lipids comprise a phosphor ester group, that is in most preferred embodiments selected from phosphatidylcholine (phosphocholine) or phosphatidylethanolamine. Preferred lipids are selected from monopalmitoylphosphatidylcholine, monolaurylphosphatidylcholine, monomyristoylphosphatidylcholine, monostearoylphosphatidylcholine, dipalmitoylphosphatidylcholine, 1-palmitoyl-2-oleoyl-sn-glycero-3-phosphocholine (POPC), 1-myristoyl-2-palmitoyl-sn-glycero-3-phosphocholine (DMPC), 1-myristoyl-2-palmitoyl-sn-glycero-3-phosphocholine (MPPC), 1,2-dipalmitoyl-sn-glycero-3-phosphocholine (DPPC).

Preferably at least one of the lipids, e.g. if a mixture of lipids or a uniform lipids are used, has a melting transition above 38°C, or above 40°C. Preferably the melting transition is in the range of 38°C to 60°C, preferably 40°C to 55°C. Lipids do not truly solidify but have a phase transition from a gel-like state to a liquid. For simplicity, this phase transition is referred to herein as melting transition. In the preferred embodiment, this phase transition is noticeable at the indicated temperatures at ambient conditions. Ambient conditions are standard conditions. A melting transition temperature above such ambient temperatures helps to control triggered release by heating the membrane, e.g. by applying an electromagnetic field to excite magnetic nanoparticles. Such release triggering by lipids in transition phase may or may not occur in the entire membrane. It is also possible that only parts of the membrane have these lipids and other parts have lipids with higher transition temperatures or polymers (hybrid vesicles). In this case, parts of the membrane will gain a higher permeability than other parts, which serves to maintain higher structural stability of the entire vesicle. Preferably, 10% to 60% (v/v) of lipids with the above identified transition temperature are used, especially preferred 20% to 50% or 25% to 40% (all v/v).

Any of the typically used lipids as mentioned hereinabove may be incorporated in this way into liposomes to tune the vesicle mechanical, physical and chemical properties, including phospholipids, sphingolipids, lysolipids, glycolipids, saccharolipids, glycophospholipids, cholesterol, PEG-lipids and others using standard procedures see for example formation of phospholipid unilamellar vesicles of various charge. In other embodiments, the lipid membrane of the vesicles may be free of cholesterol. A fraction of the lipid is preferably PEGylated to form stealth liposomes and counteract aggregation of liposomes not in the liquid membrane phase. Modifications like PEGylation can be introduced before or after formation of the inventive vesicular particles. Preferably, the modifications are after formation.

Polymer amphiphiles comprising the membrane can have structural transitions that change their shape and propensity to form a membrane. Such structural changes triggered by an increasing in temperature can use the desolvation of the hydrophilic block above the LCST to increase the permeability of the membrane due to loss in membrane integrity.

The lipids and/or polymers comprising the membrane can be labelled. Such a label may be a radiolabel or a photoreactive label, preferably a chromophore or fluorescent label. It can also be a biochemical label that confers high affinity to biological markers for targeting such as cationic charge, a peptide, antibody, antibody fragment or aptamer.

According to the invention, a solution of the lipids and/or polymers is formed with dispersed nanoparticles. As (first) solvent preferably an organic solvent of the lipids and/or polymers and nanoparticles is used such as tetrahydrofuran (THF), 1,4-dioxane, acetic acid/ethanol mix (EtOAc/EtOH) or dimethylformamide (DMF). Preferably the solvent comprises a non-aqueous organic small molecule, e.g. with a size of 3 to 10 carbon or heteroatoms such as (O, N, S, P). "Non-aqueous" indicated that the solvent - or at least one component of the solvent in case of mixtures - is not water. Preferably a cyclic compound is used. Preferably the solvent comprises at least one oxygen atom. The solvent can be a mixture but preferably it is free of water or has less than 1% (v/v) water content. Especially preferred, the solvent comprises tetrahydrofuran (THF). THF yielded the best results, especially with regard to vesicle stability (long term) and homogeneity (size distribution). The solvent is preferably a water-miscible solvent. Furthermore, the solvent is preferably volatile at ambient conditions, e.g. with a boiling point below 100°C, preferably below 90°C or below 80°C or even below 70°C, e.g. of between 30°C to 100°C. Preferably a volatility, preferably also at one of these temperatures, is maintained when mixed with the non-solvent of step ii) so that the solvent can evaporate after or during the introduction step ii). Preferably an evaporation step of the solvent is performed after step ii). This volatility and evaporation after mixing helps during liposome formation, to avoid too stable droplets to begin with and undesired phase separation.

Step ii) comprises introducing the first dispersion into a fluid that is a non-solvent for the membrane forming lipids, wherein the volume of the non-solvent exceeds the volume of the first dispersion (per introduction step), thereby forming the vesicular particles. This step essentially replaces the solvent conditions from a solvent to a non-solvent condition (solvent inversion). A mixture of the first dispersion and the non-solvent is formed, wherein preferably the solvent of the first dispersion and the non-solvent are miscible. This mixture, due to the larger presence of the non-solvent leads to the aggregation of lipid or polymer molecules to self-aggregate into the vesicular particles together with the dispersed nanoparticles. Preferably the non-solvent is in excess with regard to the solvent of the first dispersion, preferably by a factor of at least any one of 2x, or 3x, 4x, 5x, 6x, 7x, 8x, 9x, 10x or more (all volume multiplicities). The introduced volume of the non-aqueous solvent is preferably less than half of the volume of the non-solvent. The non-solvent is preferably aqueous, preferably with a water content of at least 60%, at least 70%, at least 80%, at least 90% or at least 95% of the liquid, non-solid matter. It may comprise any drugs or compounds that should be encapsulated

into the vesicular particles. The drugs or compounds may however also be present in the first dispersion, especially if better solubility can be achieved there. The non-solvent may also comprise common slats and buffer substances, e.g. to a pH of 5-9, preferably pH 6-8.

The first dispersion can be introduced into the non-solvent continuously or intermittently, e.g. dropwise, essentially by multiple steps ii).

Preferably the introducing step(s) is/are turbulent or under agitation, preferably by stirring, shaking or sonication of the non-solvent or by injection or dripping of the non-aqueous solvent into the non-solvent. With such turbulence or agitation, a faster mixing of the fluids is achieved, which controls vesicle formation and especially their size. Preferably introducing or mixing step ii) is under agitation so that vesicles with an average diameter of 20 nm to 400 nm form, preferably vesicles with an average diameter of 30 nm to 200 nm, most preferred of 35 nm to 100 nm, e.g. of 40 nm to 60 nm, form.

Especially preferred the inventive method is a combination of the above preferred elements, especially it comprises the steps of i) providing a first dispersion with one or more inorganic core particles having a hydrophobic dispersant shell and an inorganic paramagnetic or superparamagnetic core of between 1 to 15 nm in diameter, in a solution of membrane forming lipids in tetrahydrofuran; and ii) mixing the first dispersion into an aqueous fluid under rapid conditions and/or with agitation, thereby forming the vesicular particles, optionally further in combination with any other preferred elements disclosed herein.

One benefit of the inventive vesicular particles is that they can be reproducibly resized and still yield stable and homogenous vesicular particles - usually of smaller size than obtained in step ii). Preferably the inventive method further comprises sonicating the vesicular particles of step ii). To a reduced size, e.g. reduced average size (vesicle diameter) of by at least 10% or by at least 20%.

Due to the hollow sphere morphology, vesicles can be applied for encapsulation of various agents within the vesicle core and their further delivery in both synthetic and living systems. Additionally, vesicles have already been exploited as nanoreactors for controlled processes, which take place within their aqueous core. Since the first observation of vesicular structure with lipids, there have been many studies to test the feasibility of such applications with lipid vesicles (liposomes). Lipids are biocompatible, naturally occurring compounds and are ideally suited for investigation in biological systems. However, lipid vesicles have a very poor stability and high membrane permeability, which are considerable limitations in applied science. In this context, it is important to note that block copolymer vesicles have enhanced toughness and reduced water permeability. The limitations of lipid vesicles can be addressed by introducing polymer 'scaffolding' for both liposomes and planar lipid membranes, which has a stabilizing effect on the membrane (Kita-Tokarczyk et al., Polymer 46 (2005) 3540-3563).

Vesicles can be individually fabricated from lipid or synthetic block copolymer molecules via self-assembly in aqueous solutions; the blending of both vesicle forming amphiphiles leads to the formation of hybrid membranes as disclosed in Schulz et al., Soft Matter 2012, 8, 4849. Upon merging the best properties of lipo- and polymersomal membranes, hybrid lipid/polymer vesicles represent a new scaffold for medical applications combining, e.g., the biocompatibility of liposomes with the high thermal and mechanical stability and functional variability of polymersomes within a single vesicular particle. Such hybrid vesicles with both polymers and lipids may have a largely polymeric vesicular structure with island or patches of lipid membranes. According to the invention, nanoparticles are embedded into the lipid membranes of such hybrid vesicles, but of course nanoparticles may also be present in the polymeric area. The localization of the nanoparticles can be controlled by the nature of the hydrophobic part of the polymer and of the hydrophobic dispersant shell of the nanoparticle.

Therefore, the present invention also includes the use of polymers as membrane scaffold or partly membrane replacement in the inventive vesicular particles. All methods reported for liposome preparation are in general also valid for self-assembled vesicular structures of amphiphilic polymers (polymersomes) (Kita-Tokarczyk et al., supra).

Similarly to lipids, amphiphilic block copolymers aggregate in solution to produce vesicular structures. Even though the stability of lipid and polymer vesicles will inevitably vary due to their extremely different chemical composition, the principle of their formation remains essentially the same: both are held together solely by noncovalent interactions.

In polar media, such as water, the block copolymer macromolecules merge by their non-polar parts to form directly vesicles.

The polymer can be an amphiphile with a hydrophilic part and a hydrophobic part. Wherein the hydrophobic part aggregates to form a membrane by a bilayer - similar to a lipid bilayer membrane - wherein two layers of polymers form the membrane with polymer molecules joining in the middle of the membrane. The polymer may have a structure: hydrophilic part, a hydrophobic part and again a hydrophilic part. In this case, one molecule takes the form of both layers, side-by-side arrangement of the central (hydrophobic) part establishes the membrane center and each hydrophilic part reaches to either one of the opposing sides of the membrane. To accommodate both features of the polymer, it is usually a copolymer, especially preferred a diblock or triblock copolymer.

In some embodiments the hydrophilic block is selected from a polymer group consisting of polyoxyalkylene, polymethacrylate, poly(methacrylic acid), polyacrylic acid, polyacrylate, poly(alkylacrylic acid), poly(alkylacrylate), polyacrylamide, poly(N-isopropylacrylamide), poly(2-ethyl-2-oxazoline), polyethylenimine, poly(vinyl alcohol), poly(vinylpyrrolidone), poly(styrenesulfonate), poly(vinyl acid), poly(allylamine), poly(diallyldimethyl ammonium chloride), poly(methyl vinyl ether), poly(2-methyloxazoline), polyethylene glycol (PEG), or copolymer combinations thereof. Prominent examples for hydrophilic blocks of non-responsive block-co-polymers well suited for polymersome formation especially but not exclusively in the biomedical field are poly(ethylene glycol) (PEG) (also called poly(ethylene oxide) (PEO)), poly(2-methyl-2-oxozaline) (PMOXA) and poly(lactic-co-glycolic acid) (PLGA).

Preferably, the amphiphilic polymer comprises a hydrophilic block of 20 to 60 %v/v, especially preferred 30-50%v/v.

In some embodiments the hydrophobic block is selected from a polymer group consisting of poly(lactide-co-glycolic acid (PLGA), polylactide (PLA), polyglycolide (PGA), polycaprolactone (PCL), poly(methyl methacylate) (PMMA), polydimethylsiloxane (PDMS), Poly(N,N-diethylacrylamide) (PDEAAm), poly(oxazoline) (PEOz), poly(butylmethacrylate) (PBMA), polyethylene (PE) and polystyrene (PS).

In some embodiments described above or below of an aqueous soluble polymersome, the hydrophilic block has a number average molecular weight of about 1,000 to about 10,000 Daltons. In some embodiments described above or below of an aqueous soluble polymersome, the hydrophilic block has a number average molecular weight of about 5,000 Daltons. In some embodiments described above or below of an aqueous soluble polymersome, the hydrophilic block has a number average molecular weight of about 2,000 Daltons.

In some embodiments described above or below of an aqueous soluble polymersome, the hydrophobic block has a number average molecular weight of about 2,000 to 20,000 Daltons. In some embodiments described above or below of an aqueous soluble polymersome, the hydrophobic block has a number average molecular weight of about 10,000 Daltons. In some embodiments described above or below of an aqueous soluble polymersome, the hydrophobic block has a number average molecular weight of about 5,000 Daltons.

An especially preferred block copolymer is poly(isoprene-b-N-isopropylacrylamide) (PNIPAM). Prominent examples for thermoresponsive blocks of block-co-polymers are polymers, where the hydrophilic block consists of poly(2-dimethyl amino ethyl) methacrylate (PDMAEMA), Poly(N-isopropylacrylamide) (PNIPAAM) or other thermoresponsive polymers. Hydrophilic blocks can also be pH-sensitive such as poly(acrylic acid) (PAA), poly(L-lysine) (PLL) and poly(L-glutamic acid) (PGA) resulting in pH responsive polymersomes. Next to poly(methyl carpolactone) (PMCL) and poly(carpolactone) (PCL), poly(ethylethylene) (PEE), poly(dimethyl siloxan) (PDMS), polystyrole (PS), poly(N-vinyl 2-pyrrolidone) (PVP), poly(propylene oxide) (PPO) and polybutadiene (PBD) are prominent examples for hydrophobic blocks of responsive and non-responsive polymersomes. Prominent examples of block-co-polymers are poly(butadiene)-PEO (PB-PEO), poly(D, L-lactide)-PEG (PDLLA-PEG), PEG-PLA, PEG-poly(propylene sulfide)-PEG (PEG-PPS-PEG), PEG-disulfide poly(propylene sulfide) (PEG-SS-PPS), PEO-PCL, PEG-PLGA-PEG, PEO-PCL-PLA, PEO-PDEAMA, PEO-PNIPAm, PEO-PCL-PAA, PLA-PEG-PLA, PMOXA-PCL, PMOXA-PDMS-PMOXA or poly(2-methacryloyloxy)ethyl-phosphorylcholine)-poly(2-(diisopropylamino)-ethyl methacrylate) (PMPC-PDPA) (WO 2011/147926).

In preferred embodiments the first dispersion and/or the vesicular particles comprise an amphiphilic polymer. The polymer can be added to the solution of step i) or to the forming vesicular particles of step ii).

Preferably the copolymer exhibits a low critical solution temperature of about 39-55 °C, preferably of about 40-47 °C.

Compounds or drugs inside the vesicular particle can be released by electromagnetic heating that induce a reversible structural change in the lipid or polymersome membrane. The release in polymersome is usually controlled but less efficient compared to liposomes; this could possibly be improved by optimizing the structure using a higher MW block copolymer for which the hydrophilic block undergoes a more drastic volumetric change upon dehydration than is the case for, e.g. short PNIPAM blocks. A correspondingly higher MW hydrophobic block also allows for incorporation of large SPIONs that provide more efficient heating. Nevertheless, the use of lipids as membrane part for nanoparticle embedding is preferred due to better release characteristics.

The invention also provides a composition of a plurality of vesicular particles each having at least in part a lipid membrane that is a barrier between the interior and exterior of said vesicular particle, wherein said membrane comprises inorganic core nanoparticles embedded in said membrane, characterized in that A) said embedded nanoparticles are in a concentration of at least 0.5 % (w/w per lipid or polymer), and wherein said concentration is constant or decreases by less than 25 % (percentage of w/w concentration) at least during 24 hours at standard conditions in an aqueous dispersion with physiological buffer; and/or B) said vesicular particles are formed by the inventive method. The individual parts, such as the composition of the lipids, the polymer and or of the nanoparticles may be selected as described above, e.g. the nanoparticles comprise preferably magnetic core, especially a superparamagnetic core, of between 1 to 15 nm in diameter and a hydrophobic dispersant shell.

The amount of embedded nanoparticles can be determined by determining the composition as such, without individual vesicle isolation. According to the invention very high nanoparticle loading rates are possible, which are surprisingly stable at high concentrations. Preferably the concentration of nanoparticles is at least 0.5 % (w/w per lipid or polymer, i.e. membrane forming component). Preferably, especially in case of hybrid vesicles, the concentration is

determined per lipid only, e.g. if the nanoparticles aggregate in the lipid membrane part, or (less preferred) per polymer only, e.g. if the nanoparticles aggregate in the polymer part.

Especially preferred, the concentration of nanoparticles is at least 0.5 %, more preferred at least 1 %, or at least 2%, at least 5%, at least 7%, at least 8%, at least 10% (w/w per lipid or polymer, preferably per lipid only). The concentration can be about 0.5% to 25%, preferably 1% to 20%, e.g. 2% to 15% (w/w as above). Depending on the size of the vesicular particles, preferably a concentration is used wherein at least 50% of the vesicles of a composition, or plurality of vesicles, contain a nanoparticle. Preferably the concentration is 5% or greater to ensure that most vesicles have at least one nanoparticle even in case of small vesicles.

A "plurality" as used herein refers to several vesicular particles, which may differ within certain parameter thresholds in parameters such as size. The amount of the particles can be at least 100, at least 1000, at least 10000, at least 100000, at least 1 Mio., at least 10 Mio. etc.. Preferred ranges are e.g. 100 to 100 Mio.

Surprisingly the vesicles are homogeneous within the composition and predominantly unilamellar when prepared by the inventive method. Preferably the provided composition contains a plurality of vesicular particles with an average diameter of 20 to 400 nm, preferably of 30 to 200 nm, preferably 30 nm to 100 nm. As a homogeneity criterion, the standard deviation of the size distribution can be used. The standard deviation of said average size is at most 75%, preferably at most 50%, even more preferred at most 40% of the particle's average size. Preferably, the standard deviation is at most 80 nm, preferably, at most 70 nm, more preferred at most 60 nm, or at most 50 nm, at most 40 nm or even at most 30nm.

The vesicular particles can be unilammelar or multi-lammelar. Since multi-lammelar liposomes have reduced release, unilammelar vesicles are preferred. Especially preferred at least 60%, or at least 70%, at least 80% or at least 90%, of the particles in the composition are unilammelar.

Also preferred, the vesicular particles are non-porous or have a continuous surface over their entirety. Porosity means that pores of holes in the membrane are present. These should be avoided for a tighter sealing of the vesicular particles. Non-porous vesicles may not be entirely tight since leakage through the membrane may exist but by avoiding pores, systematic leakage may be avoided. Such pores that shall be avoided may have a coating of the lipids (or polymer) with the hydrophilic side or block facing the pore, i.e. the pores have a hydrophilic interior. This means that the pores may facilitate a continuous arrangement of the hydrophilic side connecting the inside and the outside of the membrane. Preferably this is not the case and hence the inside and outside of the vesicular particle is separated by the hydrophobic portion of the lipids or polymers over the entire surface of the non-porous vesicle.

Preferably a pharmaceutical agent or other loading compounds is contained in the lumen or in the membrane of the vesicular particles. The drug or compound can be incorporated in the lumen of the vesicles or in the membrane. They may be added during step i) or step ii), either in the first dispersion or in the non-solvent.

Small unilamellar liposomes/vesicles (SUVs) have sizes up to 100 nm; large unilamellar liposomes/vesicles (LUVs) may have sizes more than 100 nm up to few micrometers (μm). There are giant unilamellar liposomes/vesicles (GUVs), which have an average diameter of 100 μm. GUVs are mostly used as models for biological membranes in research work. Each lipid bilayer structure is comparable to lamellar phase lipid organization in biological membranes, in general. In contrast, multilamellar liposomes (MLVs), consist of many concentric amphiphilic lipid bilayers analogous to onion layers, and MLVs may be of variable sizes up to several micrometers.

The particles may comprise a release rate modifying agent. Such agents are e.g. selected from the group consisting of nitric acid, perchloric acid, formic acid, sulfuric acid, phosphoric acid, acetic acid, trichloroacetic acid, and trifluoroacetic acid, and salts or combinations thereof. Release rate modifying agent change the permeability of the lipid or polymer membrane either in ambient conditions or upon irradiation and hence excitation of the inorganic core nanoparticles, which may lead to increased temperature of the membrane. The release rate modifying agent can be incorporated in the lumen of the vesicles or in the membrane. They may be added during step i) or step ii), either in the first dispersion or in the non-solvent.

The invention also provides the use of the inventive composition for administration to a subject or to a cell or tissue culture. In preferred embodiments, the composition is administered to a subject and said subject is irradiated so that the inorganic core nanoparticles are excited and/or heated. This allows localized heating of the particles, at e.g. a location of interest, for release of any drugs or compounds carried by the vesicular particles. The inventive particles, during preparation or in the composition of the invention can be loaded with pharmaceutical agents. For cell or tissue culture treatment, the vesicular particles can be loaded with any component that serves to influence the culture, be it a growth factor, toxin or an expression stimulus.

The administration can be for cosmetic or medical purposes or for use as a contrast agent. The metal particles themselves can be used as contrast agent. Otherwise, the vesicular particles can be loaded with another contrast agent. Further uses of the vesicular particles are for inclusion in bandages and in tissue culture scaffolds.

The vesicular particle can be loaded with a small molecule drug, a nucleic acid or a polypeptide.

Such drugs or agents loaded into the inventive vesicular particles are usually with an atomic mass of 75 g/mol to 1000 g/mol, preferably of 85 g/mol to 700 g/mol, especially preferred of 100 g/mol to 500 g/mol, even more preferred

120 g/mol to 400 g/mol, such as 140 g/mol to 300 g/mol.

The surface of the vesicle may comprise a delivery ligand, such as an immobilized ligand for a cellular receptor that can mediate binding to a particular type of cell or tissue of interest (such as the therapeutic target cells, e.g. cancer cells). For example, the vesicles can be loaded with a chemotherapeutic reagent or with a metabolic substitute (or encoding nucleic acids therefore), such as for use in enzyme replacement therapy.

The vesicular particles comprise a biocompatible coating thereon. Such a coating is e.g. PEG as described above.

Also provided is the inventive composition or its particles for use in therapy.

The present invention is further illustrated by the following figures and examples, without being necessarily limited to these embodiments of the invention. Each step or element taken alone described in the examples is a preferred feature in combination with the invention in general as described above and in the claims.

**Figures:**

**Fig. 1.** Effect of POPC concentration on liposome formation via solvent inversion at constant THF:$H_2O$ inversion ratio of 1:10. A) DLS shows similar size distributions for the vesicles formed in the investigated concentration range (circles - 0.5mg/ml, squares - 1mg/ml, stars - 2mg/ml lipid), whereas (b) measurements of the optical density (squared solid line) demonstrate progressive deviation from values for unilamellar, extruded 100nm POPC vesicles (dashed line). The inset shows the respective preparations via solvent inversion exhibiting enhanced turbidity with increasing lipid concentration.

**Fig. 2.** TEM images of pure POPC liposomes formed via solvent inversion at constant THF:$H_2O$ inversion ratio of 1:10. Pt/C replicas of 0.5mg/ml preparation obtained by freeze-fracture/- etching TEM (a) give an overview of the morphology and size distribution of the obtained suspension in the native state. (b) Liposomes obtained at 2mg/ml frequently exhibit multilamellar membranes in freeze-fracture-TEM. Trehalose-fixed preparations of the same samples at 0.5mg/ml embedded in a sugar matrix after air drying (c) yield similar results. The obtained size distributions (blue - freeze-fracture TEM and red - trehalose fixation) are shown for comparison in (d).

**Fig. 3.** Loading content determination of liposome preparations (0.5mg/ml POPC) with different input weight fractions of spectroscopically clean 3.5nm P-NDA-SPIONs. a) DLS size distributions of a 1-10%w/w loading series, b) the corresponding $OD^{350}$ quantification curves (note that the offset at zero is due to vesicle scattering) c) representative TGA graphs of the preparations (from bottom to top: 0% (grey), 1% (black), 5% (red), 10% (green) and 20%w/w (blue) SPION input; the 20% sample is shown for impure SPIONs to illustrate their upper loading limit which is not accessible to UV determination because of higher scattering due to increased polydispersity) and d) table of loading contents evaluated by UV/VIS and TGA compared to nominal SPION weight percentage. 20%w/w SPION input is split into samples with spectroscopically clean P-NDA coated SPIONs and P-NDA coated SPIONs with residual oleic acid.

**Fig. 4.** TEM micrographs of POPC liposomes loaded with 5%w/w 3.5nm P-NDA-SPIONs. (a) overview and b) magnified vesicles depicting the nanoparticle distribution. Samples were prepared via solvent inversion (THF:H2O=1:10) at 0.5mg/ml lipid and fixed in 1% trehalose by air drying.

**Fig. 5.** (a) DLS and (b) ATR-FTIR of POPC liposomes prepared by solvent inversion and loaded with different contents of 3.5nm P-NDA-SPIONs purified by standard methods (light color) leading to residual oleic acid in the sample (red- 1%, green - 5%, blue - 10% and magenta 20% SPION input; black - incompletely purified SPIONs with residual OA and grey - pure P-NDA SPIONs are shown for reference). Preparations with clean SPIONs are shown as overlay (dark color).

**Fig. 6.** Phase diagram of the prepared nanoparticle-lipid assemblies. The grey region depicts formation of LUVs, the white indicates formation of polydisperse MLVs by only solvent inversion. The shaded region highlights structural changes through association of the vesicles with surfactant remnants from incomplete SPION purification ultimately leading to a loading cut-off around 10%w/w. MLVs with less than 10%w/w loading can be resized to LUVs without significant SPION loss by extrusion.

**Fig. 7.** TEM images of assemblies from POPC ($c_{lipid}$=0.5mg/ml) with 5% SPIONs of different sizes (a) 4.5nm and (b) 8.3nm, fixed by trehalose. 4.5nm SPIONs are incorporated while assemblies with 8.3nm SPIONs exclusively yielded unloaded lipid vesicles coexisting with nanoparticle loaded lipid droplets.

**Fig. 8.** Stability of POPC liposomes loaded with 5%w/w 3.5nm SPIONs stored in water at room temperature (red symbols) or at 4°C (blue symbols). The hydrodynamic diameter d (intensity weighted average) and polydispersity index PDI of the distributions are indicated by filled and empty squares respectively over the time-course of 1 month.

**Fig. 9.** $^1H$ NMR spectra of POPC in $D_2O$ containing 1mg/ml DSS as reference standard. Liposomes were formed at 0.5mg/ml via 1:10 solvent inversion. (a) NMR spectrum right after dropwise addition of THF at t=0h and (b) after

24h of evaporation. The size of the formed vesicles was around 200nm. DSS signals are found at 2.9ppm (t, 2H, $-CH_2SO_3^{l}$), 1.75ppm (p, 2H, $-CH_2-$), 0.65ppm (t, 2H, $-CH_2SiR_3$) and 0ppm (s, 9H, $-SiMe_3$).

**Fig. 10.** OD curves of POPC liposomes formed via 1:10 solvent inversion (THF into water) at 0.5mg/ml (black), 1mg/ml (blue), 1.5mg/ml (green) and 2mg/ml (red) total lipid concentration.

**Fig. 11.** (a) DLS size distributions of DMPC (dashed) and MPPC (full lines) formed at T<Tm (blue) and T>Tm (red) via solvent inversion (0.5mg/ml lipid; THF:H2O=1:10). (b) shows DLS curves for DPPC assemblies formed via the same conditions and a stability series for 1-20%w/w SPION loaded assemblies at selected times (t=0 directly after THF addition, t=12h after evaporation of THF and t=24h after overnight storage at RT).

**Fig. 12.** DLS size distributions of DPPC liposomes ($c_{lipid}$=0.5mg/ml; THF:H2O=1:10) formed in presence of various chemical inhibitors of interdigitation fusion below the lipid $T_m$. Color coding: blue - 20%n/n Chol (cholesterol), red - 55%w/w treh (trehalose 1.5M) and black - 20%v/v DMSO (dimethylsulfoxide).

**Fig. 13.** (a) DLS scattering curves for DPPC liposomes(red) loaded with 5%w/w SPION exhibiting a similar size distribution as loaded POPC liposomes (black, dash). Samples were prepared via solvent inversion (THF:H2O=1:10) above the $T_m$ of DPPC (T=55°C) by adding 20%v/v of DMSO to the aqueous phase prior to addition of DPPC in warm THF. After evaporation of THF, the sample was dialysed (Novagen D-tube, 12-14kDa MWCO, RC) for 12h against Milli-Q water to remove residual DMSO. (b) OD curves of the same samples. The SPION-loaded vesicles show a characteristic increase in OD.

**Fig. 14.** Comparison of loading methods for 5%w/w 3.5nm P-NDA-SPION input (POPC). (a) DLS curves (1:10 dil) of the preparations. The inset shows the following preparations (left to right): first - rehydration (supernatant after 12h resting) at 5mg/ml lipid, second - rehydration plus extrusion through 100nm PVP coated track-etched PC-membranes at 5mg/ml lipid and third - THF-$H_2O$ solvent inversion at 0.5mg/ml. (b) OD curves (1:10 dil) of the preparations. POPC vesicles formed via solvent inversion and 3.5nm P-NDA-SPIONs in MeOH:THF=10:1 are shown for comparison.

**Fig. 15.** OD curves of 3.5nm P-NDA-SPIONs at different concentrations in THF (a) and (b) calibration curves at various wavelengths.

**Fig. 16.** OD curves of POPC vesicles (0.5mg/ml) loaded with 1-10, 15 and 20%w/w (1:1 diluted) 3.5nmP-NDA-SPIONs.

**Fig. 17.** OD curves of POPC preparations with different weight fractions of 3.5nm P-NDA SPIONs containing residual physisorbed oleic acid (THF:$H_2O$=10:1; $c_{lipid}$=0.5mg/ml) The inset shows the following SPION weight fractions: 1, 5, 10, 20% (left to right)

**Fig. 18.** (a) DLS graphs and (b) OD curves of POPC liposomes ($c_{lipid}$=0.5mg/ml) in different buffers loaded with 5%wt 3.5nm P-NDA-SPIONs via solvent inversion. 1x PBS (10mM $Na_2HPO_4$ / 2.7mM KCl / 137mM NaCl) and 1x TBS (50mM Tris / 150mM NaCl).

**Fig. 19.** (a) DLS scattering curves and (b) OD curves of POPC vesicles ($c_{lipid}$=0.5mg/ml) containing 5%w/w improperly purified SPIONs1x PBS (10mM $Na_2HPO_4$ / 2.7mM KCl / 137mM NaCl), 1x TBS (50mM Tris / 150mM NaCl) and isotonic NaCl (140mM)

**Fig 20.** (a) DLS graphs and (b) OD curves of POPC preparations ($c_{lipid}$=0.5mg/ml) containing 5%w/w SPION formed at different THF:$H_2O$ ratios.

**Fig. 21.** (a) DLS curves of POPC vesicles formed at 5mg/ml before (dashed lines) and after post-extrusion (solid lines) loaded with 5% (red) and 10% SPION (black). (b) UV/VIS quantification of SPION loss by extrusion. Samples were prepared by solvent inversion (THF:$H_2O$=1:10) into Milli-Q water and extruded 31 times through 100nm track-etched polycarbonate filters. The loss of SPIONs was evaluated at 350nm by comparing the filter absorption (polycarbonate membrane after extrusion in 1ml THF) to the input SPION absorption (in 1ml THF) at 1:16 dilution. The UV absorption of the plain PC membrane is shown for reference.

**Fig. 22.** (a) DLS and (b) OD measurements before (straight lines) and after (dashed lines) passing 5%w/w SPION loaded vesicle suspensions (0.5mg/ml lipid) over a magnetic column (dimensions: height x diameter = 3.5cm x 1cm; 0.5g ultrafine steel wool). The slightly altered UV absorption of the 3nm loaded sample post elution is attributed to co-eluted material from the column.

**Fig. 23.** TEM micrographs of trehalose-fixed liposomes loaded with SPION. (a) Spherical structures of high contrast with associated nanoparticles (red, arrows) were observed for some magnetoliposome preparations at high content of 3.5nm SPION. Similar features (red, dashed circles) were however also seen for low loading contents in (b) and in samples where exclusively 8nm lipid droplets were observed (see lower panel). It is likely that the observed features result from trehalose fixation, since such structures were not indicated in other experiments, such as DLS or magnetic chromatography.

**Fig. 24.** TEM images of co-existing empty liposomes and lipid coated SPION aggregates formed at (a) high concentration of 3.5nm SPION and (b) with 8nm SPION. Similar features (red dashed circles) as in the case of 3.5nm loaded vesicles are sometimes observed in the background..

**Fig. 25.** Temperature-dependent DLS size distributions (left) at 25-70°C in 5°C steps of the crude PI-*b*-PNIPAM

assemblies at 1mg/ml prepared by THF solvent inversion into Milli-Q water. B) TEM after trehalose fixation of the sample shows spherical objects with a similar size distribution as obtained from room-temperature DLS. The lower contrast of the vesicular structures are due to that water in the lumen of the vesicles is not replaced by trehalose.

**Fig. 26.** A) DLS size distribution and B) TEM micrograph of calcein-loaded, extruded PI-*b*-PNIPAM polymersomes at 1mg/ml with 20%w/w 3.5nm hydrophobic SPION input. Samples were prepared by THF solvent inversion into 5mg/ml calcein solution to form polydisperse, large polymersomes and subsequent extrusion through 100nm track-etched polycarbonate membranes after evaporation of the organic solvent. A high SPION content is seen from the high contrast of most vesicles and the cores are directly visualized in the inset. C) Optical density curves of extruded PI-*b*-PNIPAM vesicles without nanoparticles (red), SPION loaded polymersomes before (black) and after (blue) homogenization by 10 passes through 100nm track-etched polycarbonate membranes. The inset shows a digital image of the preparations before and after extrusion. D) TGA curves (20-650°C) of BCP **2** and BCP **2** extruded with 20%w/w P-NDA-coated iron oxide nanoparticles. Iron oxide content (taking inorganic residue of BCP into account) is estimated to be ~9 %w/w, which is significantly higher than for prior pure liposomes.

**Fig 27.** A) Release kinetics of calcein encapsulated in 3.5nm hydrophobic SPION-loaded PI-*b*-PNIPAM polymersomes. The samples were actuated with 10min AMF pulses followed by a 5min cool-down period. B) The hydrodynamic size distribution of the polymersomes measured before (blue) and after (red) actuation by AMF is almost unchanged, indicating increased permeability without destruction of the vesicles.

**Fig 28.** DLS size distributions of various magnetopolymersomes (10%w/w SPION) prepared via solvent inversion at 2mg/ml and homogenization by extrusion through 100nm PC membranes. (A) PBD-b-PEO-OH, (B) PBD-b-PEO-COOH / b-PEI (100%n/n), (C) PBD-b-PEO-DEDETA (50%n/n) and (D) PBD-b-PEO / $DOPC^+$ (30%n/n)

**Fig 29.** TEM micrographs of ultrathin sections of nanoparticle loaded PBD-b-PEO polymersomes (10%w/w SPIONs) prepared via solvent inversion at 1mg/ml. Hydrophobic SPIONs (black granular objects) are homogeneously embedded in the polymer membrane. The lower contrast of the vesicular structures is due to that the fixing matrix did not replace the hollow interior of the vesicles.

**Fig. 30.** Confocal images of (a) HeLa cells (negative control), (b) HeLa cells after 12h incubation with PBD(1200)-b-PEO(600) polymersomes (1% DEAC labeled), (c) HeLa cells after 12h incubation with cell light stain expression a red fluorescent protein (RFP) in lysosomes, (d) HeLa cells after 12h incubation with cationic b-PEI adsorbed to the polymersomes (positive control), (e + f) co-localization of the fluorescently labeled cationic polymersomes (green) within lysosomes (red). Neutral polymersomes exhibit slow uptake kinetics while those modified with cationic b-PEI show an increased frequency of internalization and localization within lysosomes.

**Fig. 31.** Confocal images of (a) HeLa cells after 12h incubation with polymersomes containing 50% DEDETA (1% DEAC; green) and (b + c) the corresponding lysosome (red) co-localization images. Image (d) shows HeLa after 12h uptake with 20% DOPC+-blended lipopolymersomes (green) and (e + f) show the co-localization within lysosomes (red).

**Fig. 32.** TEM micrographs of (A) as-synthesized monodisperse SPIONs with (B) a size distribution of 5 $\pm$ 0.4 nm. (C) Overview of ultra-thin sections of membrane embedded hydrophobic SPIONs in PBD(1200)-b-PEO(600) polymersomes prepared by solvent inversion at 0.5-1mg/ml amphiphile concentration. (D) Close-up TEM of same sample showing the SPION distributed inside the membrane of the vesicles. The low contrast in the center demonstrates the empty lumen which could not be filled by the fixing solution.

**Fig. 33**. TEM ultrathin sections of HeLa cells ($OsO_4$ stained) after 12h incubation with fluorescent magnetopolymersomes (PBD(1200)-b-PEO(600), 10% SPION, 1% DEAC). (a) Overview of a typical preparation showing internalized polymersomes as dark spherical objects and (b) peripheral cell region with an internalized polymersome. The inset depicts a close-up of a stealth SPION loaded multilamellar structure.

**Fig. 34.** TEM ultrathin sections of HeLa cells ($OsO_4$ stained) after 12h incubation with cationic fluorescent magnetopolymersomes (50% PBD(1200)-b-PEO(600), 50% PBD(1200)-b-PEO(600)-COOH, 10% SPION, 1% DEAC, 50% b-PEI). Black objects in (a) represent elevated levels of uptake of magnetopolymersomes after cationic modification. The sequence (b-f) shows the hydrolytic degradation of SPION loaded polymersomes after internalization.

**Fig. 35**. TEM ultrathin sections of HeLa cells ($OsO_4$ stained) after 12h incubation with fluorescent magnetopolymersomes (50% PBD(1200)-b-PEO(600), 10% SPION, 1% DEAC) blended with DOPC (30%n/n).

**Examples:**

**Example 1: General Material and Methods**

**Reagents**

All reagents were purchased from Sigma Aldrich and used as received without further purification.

Ultrapure water (Millipore USA, R=18M$\Omega$cm); THF (Chromasolv plus for HPLC, inhibitor free) $\geq$99%; 1,4-Diox-

ane (anhydrous) 99.8%; EtOAc (anhydrous) 99.8%; DMF (ACS reagent) ≥99.8%; EtOH (Chromasolv for HPLC, absolute) ≥99.8%; PBS tablets (0.01 M phosphate buffer, 0.0027 M potassium chloride and 0.137 M sodium chloride, pH 7.4, at 25 °C); TBS BioUltra tablets (0.05 M TRIS-HCl buffer ; 0.15 M sodium chloride; pH 7.6 at 25°C)

All employed P-NDA-coated magnetite nanoparticles originated from the same batches.

All lipids were obtained dissolved in Chloroform from Avanti Lipids Inc. and high-vacuum dried for at least 24h before further use.

1-palmitoyl-2-oleoyl-sn-glycero-3-phosphocholine (POPC) >99%, 1-myristoyl-2-palmitoyl-sn-glycero-3-phosphocholine (DMPC) >99%, 1-myristoyl-2-palmitoyl-sn-glycero-3-phosphocholine (MPPC) >99%, 1,2-dipalmitoyl-*sn*-glycero-3-phosphocholine (DPPC) >99%. Where nothing else is stated, POPC was used as lipid.

For polymersomes:

4-Cyano-4-(phenylcarbonothioylthio)pentanoic acid >97%; Isoprene 99% (contains <1000 ppm *p*-*tert*-butylcatechol as inhibitor); *N*-Isopropylacrylamide 97%; 2,2'-Azobis(2-methylpropionitrile) 98%; *S*-Methyl methanethiosulfonate 97%; *N,N*-Dimethylethylenediamine 95%; 5-(Dimethylamino)naphthalene-1-sulfonyl chloride BioReagent, powder and chunks ≥99% (HPLC); Ethanolamine ACS reagent ≥99.0%; *N,N'*-Dicyclohexylcarbodiimide puriss ≥99.0% (GC); 4-(Dimethylamino)pyridine ReagentPlus ≥99%; Milli-Q water (R=18MΩcm); Methanol anhydrous ≥99.8%; Acetone Chromasolv for HPLC ≥99.9%; Dichloromethane anhydrous ≥99.8% (contains 50-150 ppm amylene as stabilizer); Chloroform ≥99.5% (containing 100-200 ppm amylenes as stabilizer); Tetrahydrofuran Chromasolv Plus for HPLC ≥99.9% (inhibitor-free); 1,4-Dioxane ACS reagent ≥99.0%; Toluene anhydrous 99.8%; n-Hexane anhydrous 95%; Aluminium oxide activated, basic, Brockmann I (150 mesh); (+)-D-Trehalose dihydrate from corn starch >99%. *N*-isopropylacrylamide (NIPAM) was recrystallized from hexane/toluene : 1/1 v/v. 2,2'-Azobis(2-methylpropionitrile) (AIBN) was recrystallized from methanol. Isoprene was purified by passing through a column of basic alumina.

**Measurement conditions**

**TEM and analysis:** TEM studies were performed on a FEI Tecnai G2 20 transmission electron microscope operating at 120 kV or 200 kV for high resolution imaging. Samples were prepared by drop-casting aqueous vesicle dispersions onto 300-mesh carbon-coated copper grids. Size distributions were evaluated using PEBBELS.

**Dynamic Light Scattering:** Hydrodynamic size distributions were measured on a Malvern Zetasizer Nano-ZS (Malvern UK) in Milli-Q water or buffer at 25°C in 173° backscattering mode. Samples were equilibrated for 120s each and the autocorrelation function was obtained by averaging 3 runs. Samples were measured as-prepared without further dilution.

**OD measurements:** UV-Vis spectra were collected at a scan speed of 400nm/min on a Hitachi UV-2900 spectrophotometer referenced against pure solvent.

**TGA/DSC measurements:** Thermograms were recorded on a Mettler-Toledo TGA/DSC 1 STAR System in the temperature range 25-650°C with a ramp of 10K/min in synthetic air ($O_2$). 70μl aluminum oxide crucibles were filled with 0.5-2mg sample and the rest mass was evaluated at 500°C. The mass loss was obtained by placing horizontal steps to the TGA curves.

**ATR-FTIR measurements:** Mid-IR powder spectra of the lyophilized samples were collected on a single reflection Bruker Platinum Diamond ATR at a resolution of $4cm^{-1}$ by averaging 32 scans.

**$^1$H-NMR measurements:** $^1$H -solution spectra were collected on a Bruker DPX operating at 300MHz in D2O using 1mg 4,4-dimethyl-4-silapentane-1-sulfonic acid (DSS) as an internal standard.

**Sample preparation**

**Trehalose fixation:** An aliquot of 10%w/v trehalose stock solution was added to the vesicle suspension to give a final trehalose concentration of 1-2%w/v. The sample was gently vortexted for 1min before a drop of the sugar-vesicle solution was placed onto a carbon-coated copper grid. The vesicles were allowed to adsorb for 30min before the grid was cautiously washed with a drop of Milli-Q water. Samples were dried for several hours in air before examined in TEM.

**Freeze-fracture/-etching:** 5μl vesicle suspension was loaded onto a gold specimen holder and shock-frozen by quickly immersing it into dichlorofluoromethane (Freon R22) at -196°C. The fixed sample was mounted onto a sample holder under cryogenic temperatures and transferred to a Balzer BAF400 freeze-etching system. After an initial equilibration period of 10min at -150°C, the sample was slowly warmed to -100°C for fracturing. The sample top was stripped off with a N2(1)-cooled microtome and the exposed surface etched by sublimation of 40nm ice in high-vacuum (90sec at -100°C and $10^{-6}$ mbar). A 2nm Pt-shadowing was evaporated from a 45° angle followed by a carbon support layer of 20nm. The sample was subsequently warmed to room-temperature and cleaned in 70% H2SO4 overnight to digest all organic material. The cleaning medium was exchanged five times for Milli-Q water, the washed replicas loaded onto 300mesh copper grids and dried overnight before imaged in TEM.

**NMR determination of residual solvent:** Vesicles were prepared by the standard 1:10 solvent inversion procedure to 0.5mg/ml POPC in 10ml $D_2O$ containing 1mg/ml DSS and withdrawing 1ml aliquots right after THF addition and after 24h of evaporation. The size of the formed liposomes was determined to be around 200nm.

**Vesicle preparation by rehydration plus extrusion:** 5mg POPC mixed with 5%w/w SPIONs in 3ml $CHCl_3$ were dried on the rotary evaporator and lyophilized in high vacuum for 12h. The dry lipid-nanoparticle film was rehydrated in 1ml Milli-Q water for 2h at 50°C and detached from the flask wall by gentle sonication (3x 30sec). The rehydrated sample was subsequently extruded 31 times through 100nm track-etched polycarbonate (PC) membranes (Avanti Lipids) or PVP-coated PC membranes (Whatman).

**Magnetic column separation:** A perforated Eppendorf tube was packed with 0.5g of ultrafine steel wool and flushed thrice with ultrapure water. The column was attached to a 1T Nd/Fe/B-magnet and the sample of SPION-loaded lipid vesicles/aggregates was passed through the column. UV/VIS quantification after up-concentration to the initial volume (speed-vac) was used to access the amount of aggregates formed.

**Example 2: SPION preparation**

Monodisperse 3.5nm N-palmityl-6-nitrodopamide (P-NDA) capped superparamagnetic iron oxide nanoparticles (SPIONs) were synthesized as reported previously (PCT/EP2015/068253 or Bixner et al., Langmuir, 2015, 31, 9198-9204). In brief, 200mg as-synthesized SPIONs were purified by repeated pre-extraction in hot MeOH containing 1mM oleic acid as stabilizer before exchanged in a mixture of 150mg P-NDA in DMF:$CHCl_3$:MeOH=6:3:1 for 3h under nitrogen gas. Newly capped SPIONs were evaporated to the DMF fraction, precipitated by adding excess MeOH and collected via magnetic decantation. The particles were purified by threefold extraction in hot MeOH. Mixed dispersant SPIONs were post-coated with 100mg P-NDA in minimal 2,6-lutidine for 48h at 50°C under inert atmosphere, evaporated to dryness and purified by hot MeOH extractions. SPIONs were lyophilized from THF:$H_2O$ (5:1).

**Example 3: Vesicle preparation by solvent inversion**

The respective amount of high-vacuum dried lipid (usually 5mg) or respective nanoparticle-lipid mixes were dissolved in 1ml anhydrous THF and dropwise (approx. 1 drop per second) added into 10ml aqueous phase (ultrapure water or buffers) under constant magnetic stirring (400rpm). THF was evaporated for 24h under air circulation or $N_2$ flow. The vesicle suspension was refilled with water or buffer to the original concentration. Where nothing else is specifically stated 1-palmityl-2-oleoyl-sn-glycero-phosphatidyl choline (POPC) was used as lipid.

**Example 4: Calculations**

$M_w$ calculation of core-shell SPIONs (d=3.5nm)

$$m_{core-shell} = m_{core} + m_{shell}$$

$$m_{core} = \rho_{core} * V_{core}(r) = \rho_{Fe_3O_4} * \frac{4\pi}{3} r_{core}^3$$

$$m_{shell} = \frac{M_{shell}}{N_A} = \frac{N_{lig}(r) * M_{lig}}{N_A} = \frac{4\pi r_{core}^2 \rho^{graft} * M_{lig}}{N_A}$$

$$m_{core-shell} = 1.91 * 10^{-19} g$$

$$M_{core-shell} = m_{core-shell} * N_A$$

$$M_{core-shell} \sim 1.15 * 10^5 g/mol$$

$M_w$ calculation of liposomes (d=100nm)

$$N_{lipids}(r) = \frac{4\pi\left(\frac{d}{2}\right)^2 + 4\pi\left(\frac{d}{2} - h\right)^2}{a}$$

$$M_{liposome}(r) = N_{lipids}(r) * M_{lipid}$$

$$M_{liposome}(50nm) = 6.08 * 10^7 g/mol$$

$$m_{liposome}(r) = \frac{M_{liposome}(r)}{N_A}$$

$$m_{liposome}(50nm) = 1.01 * 10^{-16} g$$

estimation of maximum SPION loading per liposome (d=100nm)

$$S_{liposome}(r) = 4\pi r_{liposome}^2$$

$$A_{core-shell\,SPION} = \pi r_{total}^2$$

$$r_{total} = r_{core} + l_{ligand}$$

$$N_{max}^{SPION} = \frac{S_{liposome}}{A_{core-shell\,SPION}} * 0.74\ (hcp - packin$$

$$N_{max}^{SPION} = 328\ SPIONs\ /100nm\ liposome$$

$$m_{max}^{SPION} = N_{max}^{SPION} * m_{core-shell}$$

$$w_{max}^{SPION} = \frac{m_{max}^{SPION}}{m_{liposome}} * 100 = 62\%$$

The calculated %w/w refers to weight-% SPION per lipid to ensure easy comparability to the SPION input values given in the main manuscript.

**Example 5: Vesicle formation and lamellarity**

It is most important to assemble large (~100nm in diameter), monodiperse and unilamellar vesicles to optimize loading and control rapid triggered release. Large unilamellar vesicles composed of 1-palmityl-2-oleoyl-sn-glycero-phosphatidyl choline (POPC) were prepared using solvent inversion. The non-polar, water miscible solvent THF was used as carrier/transfer fluid for the mix of monodisperse N-palmityl-6-nitrodopamide (P-NDA) coated magnetite particles and lipids; the mix is rapidly diluted upon injection into a larger volume of aqueous phase. During the assembly process THF is thought to behave as a co-solvent scaffold for both species followed by progressive dialysis. In this sense THF serves as a fluidizer that provides the system with a combination of solvation and flexibility to rearrange while being slowly forced into the final assembly. THF itself is a high vapor pressure solvent and is readily evaporated under continuous

nitrogen flow until a homogeneous suspension of lipid vesicles containing SPIONs is achieved. An efficient removal of solvents is especially important with respect to delivery applications as remnants render liposomes leaky and might induce toxicity. The amount of residual THF in the preparations was quantified by NMR to be 0.05‰ or 50ppm of its initial value (see Figs. 9 and 10). Such minimal traces of THF retained after 24h of evaporation are far below any toxic level and suitable for biological and medical applications.

Attempts to replace THF by other commonly used organic solvents or solvent mixtures like 1,4-dioxane, EtOAc/EtOH or DMF, resulted in weaker structure of magnetoliposomes and/or reduced nanoparticle dispersion.

Figure 1 demonstrates the formation of POPC vesicles at different lipid concentrations for a constant THF:$H_2O$ inversion ratio of 1:10. DLS demonstrates the spontaneous formation of monodisperse liposomes with approximately 100nm in diameter. The size distribution hardly changed when the lipid concentration was increased from 0.5 to 2mg/ml (Fig. 1a), but the turbidity increased drastically (Fig 1b). Measurements of the dependence of the optical density at 436nm ($OD_{436}$) on the lipid concentration ([L]) allow for discrimination between uni- and oligolamellar vesicles. The measured $OD_{436}$ vs [L] curve for vesicle solutions prepared by solvent inversion in Fig. 1b suggests that the observed increase in turbidity with increasing input lipid concentration is mainly related to an increase in lamellarity of the vesicles. The $OD_{436}$/[L] ratio matches a homogeneous sphere model of the respective diameters at low concentrations while better agreement to optically denser oligolamellar vesicles is obtained for liposomes prepared at higher lipid concentrations.

The size distribution, morphology and lamellarity of the liposomes were additionally checked by freeze-fracture/-etching TEM and by trehalose fixation of the preparations (Figure 2). Lipid suspensions of 0.5mg/ml exhibited spherical, monodisperse and unilamellar vesicles of uniform morphology. The dispersity obtained from DLS (PDI=0.21) matches commonly used homogenization methods in the same liposome size range such as extrusion (PDI=0.14) through polycarbonate membranes (see Fig. 1, 2 and 14). Replicas obtained by freeze-fracture/-etching on liposomes solutions with 2mg/ml of lipid, frequently revealed multiple layers on the fractured liposome surface (cf. Fig. 2b). The results qualitatively confirmed the results from DLS and OD measurements, by demonstrating similar size and high monodispersity, but an increased frequency of multilamellar membranes for liposomes formed at higher lipid concentration.

### Example 6: Magnetosome formation in various media

Two common buffer systems were tested: 1x PBS (140mM NaCl, 10mM $Na_2HPO_4$, pH=7.4) and 1x TBS (140mM NaCl, 10mM Tris, pH=7.4). PBS is commonly used to mimic intracellular fluids but is particularly incompatible with surface modified iron oxide nanoparticles since phosphate ions can displace dispersants from the particle surface and reduce colloidal stability. TBS is in this respect less challenging but has the same ionic strength. Typical preparations of SPION-loaded vesicles (0.5mg/ml POPC; 1:10 inversion; w/wo 5%w/w SPION;) exhibited monomodal size-distributions with a scattering maximum slightly above 100nm in both buffers. Slightly larger average hydrodynamic diameters and broader distributions were observed in PBS than in TBS (see Fig. 18 and 19). The cosmotropic or H-bond breaking character of phosphate ions favors the hydrophobic effect and in turn causes enhanced aggregation of lipid acyl chains; this is consistent with the larger assemblies observed in PBS by DLS. The salting out effect also correlates with increasingly polydisperse assemblies at higher ionic strengths due to poor solubilities of organic solvents in phosphate buffers.

### Example 7: Dependence of vesicle formation on lipid species and temperature

While unsaturated lipids easily assembled into the desired LUVs through solvent inversion from THF in water, formation of saturated lipid vesicles was highly dependent on the chain length of the employed lipid. Saturated lipids such as DMPC, MPPC or DPPC were insoluble in THF at room-temperature and required gentle heating in order to be dissolved prior to inversion into aqueous medium. Saturated lipid samples were assembles by dropwise addition of the warm THF solutions (around $T_m$ of the lipid species) into the stirred, equilibrated aqueous phases immersed in a thermostated water bath ($T = T_m \pm 10°C$).

For 1,2-dimyristoyl-*sn*-glycero-3-phosphocholine (DMPC; $T_m$=24°C), exhibiting the shortest symmetric acyl chain length that was tested and therefore the lowest melting temperature, no marked differences in size distribution were observed for preparations below (water-bath, 15°C) or above the $T_m$ (37°C). All preparations yielded stable vesicles with rather broad distributions (PDI= 0.36-0.48) centered around 89 nm (Fig. 11). In contrast its higher analogue 1,2-dipalmitoyl-sn-glycero-3-phosphocholine (DPPC; $T_m$=41°C) resulted in rather ill-defined micron-sized assemblies that became unstable after removal of THF and precipitated within hours. Initially DPPC formed a clear dispersion in THF-water which became turbid and ultimately lead to flocculation of the sample (Fig. 11). The longer the alkyl chains the less soluble the lipids are in THF and the less defined are the resulting assemblies in terms of size and stability.

A slightly different behavior was observed for lipids with unsymmetric acyl chain lengths. 1-myristoyl-2-palmitoyl-*sn*-glycero-3-phosphocholine (MPPC; $T_m$=35°C) preparations formed at $T>T_m$ resulted in size distributions at slightly larger diameters than at $T<T_m$ (Fig. 11). Both preparations resulted in vesicles that were stable over weeks.

While the as-prepared DMPC and MPPC assemblies generally were sub-micron sized and stable for a few weeks, DPPC assemblies were distributed over a wide size range and colloidally unstable. For the latter, precipitation of excess SPIONs occurred during evaporation of the organic solvent accompanied by a significant increase in turbidity of the solutions. Storage at room-temperature led to the formation of flakes and precipitation of the sample (Fig. 11).

The difference in formation behavior among saturated and unsaturated lipids can be due to solvent influence on lipid interdigitation. It is generally accepted that a chemical inducer such as organic solvent adsorbs to the interfacial region of the lipid where it causes an increase of the head-group volume of the amphiphile. This in turn alters the head-group tilt causing the formation of unfavorable voids to lamellar phases, which rearrange to a more stable interdigitated structure in which the lipids adopt a transverse stacked, alternating monolayer pattern. The latter is stabilized by organic solvent capping of the terminal methyl groups which face the aqueous bulk and persist until a lower critical solvent concentration is reached upon which interdigited structures coalesce into large, vesicular arrangements. Saturated phospholipids are prone to interdigitation fusion in aqueous solvent mixtures, notably THF-$H_2O$, whereas unsaturated lipids are strongly disfavored to interdigitate by virtue of their molecular geometry and lower phase transition temperatures.

Insensitivity of vesicle quality to temperature is surprising since the lipid phase state is known to have significant influence on interdigitation. Generally the liquid crystalline state, which the lipids are in due to dissolution in THF around their $T_m$, is highly unfavorable for interdigitation, because disordered acyl chains are more accessible to water. However the steric effect of solvent adsorption to the interface was reported to dominated at room temperature which also seems to hold in our case.

The much lower dispersity achieved for long-chain phospho-choline-lipid assemblies seems to correlate with their overall lower solubility in THF. Quick dilution upon injection triggers rapid loss of co-solvency causing large interdigitated assemblies to be formed for which the rate of fusion is accelerated because reduced curvature provides less steric hindrance to glide fusion of opposing monolayers.

Magnetosomes with high $T_m$ and therefore composed of saturated lipids with long fatty acid chains are, however, required for in vivo applications exploiting the difference between body temperature and magnetosome $T_m$ for stable circulation and magneto-thermally triggered release. We therefore strived to further refine the vesicle assembly of DPPC by investigating the action of chemical inhibitors of interdigitation such as cholesterol, trehalose or DMSO. Admixture of cholesterol at 20%n/n readily gave rise to monomodal size distributions centered at 100nm (Fig. S-4). Yet cholesterol is known to fill up voids in the bilayer and thereby strongly reduce the co-encapsulation of hydrophobic agents such as our SPIONs. Trehalose (1.5M) yielded a bimodal size distribution of SUVs and a broad distribution of LUVs (Fig 12).

Addition of 20%v/v DMSO below Tm also resulted in a bimodal size distribution (Fig. 12) and low stability. A monomodal size distribution with hydrodynamic diameter of 110nm and PDI comparable to that of POPC vesicles could be prepared with or without SPION loading in 20%v/v DMSO at a temperature of 55°C, which is above $T_m$ (Fig. 13). Neither DMSO nor elevated temperature on their own resulted in an improvement of DPPC liposome assembly, but a combination of both yielded well-defined vesicles with excellent stability that could be purified from DMSO by overnight dialysis. Thus, this combination yields a general way to form magnetosomes also from long-chain saturated lipids without affecting the co-assembly of SPION into the membrane interior that can be used for all solvent inversion preparation of magnetosomes compared below.

**Example 8: Comparison of nanoparticle loading methods**

Previously, rehydration of SPIONs together with lipids from a dried film followed by extrusion has been used to load SPIONs into membranes of small to large unilamellar vesicles (Amstad et al., Nano Lett., 2011, 11, 1664-1670; WO 2011/147926). This benchmark was compared to the solvent inversion method for 3.5nm in diameter $Fe_3O_4$ nanoparticles coated with N-palmityl-6-nitrodopamine at a grafting density of 2.7/$nm^2$. The DLS data is summarized in Fig. 14. For comparison, a fixed amount of 5%w/w SPIONs was added to 5mg lipids and subjected to the respective preparations. The concentration of SPIONs in solution and thus their embedding efficiency can be determined by UV/Vis spectroscopy (see Fig. 3, 15 and 16) as nanoparticle absorption dominates the transmitted light spectrum.

Pure rehydration gave rise to broad, polydisperse size distributions with a time-dependent loading content of embedded nanoparticles. SPIONs precipitated over time, which led to a decrease in particle content with time. OD measurements showed that a minimal amount of SPIONs was retained after overnight suspension at room temperature (see Fig. 14, 20, 21).

Subsequent extrusion of rehydrated dispersions through polycarbonate membranes reconfirmed previous observations of significant loss of lipid and nanoparticle material. An almost colorless suspension is obtained, for which the embedding of SPION was pushed below the UV/VIS detection limit (Fig. 14-16). Hydrophilic extrusion filters (track-etched PVP-coated PC, Whatman) performed better than standard polycarbonate membranes (Avanti); however, after 31 passes the amount of incorporated SPIONs was still below detection limit by UV/VIS.

Solvent inversion to 0.5mg/ml of final lipid concentration yielded markedly colored, clear suspensions with an OD at 350nm corresponding to the calibrated extinction of 5%w/w addition of SPION (cf. Fig. 3b and 14-16). Such

magnetoliposome suspensions were long-term stable. Size distributions of unloaded and SPION-loaded preparations can be found in Figs. 1 and 3, respectively. Thus, the advantage of accurate and high SPION-loading using the solvent inversion methods compared to previous preparation methods is evident.

**Example 9: Determination of loading content**

The influence of the nanoparticle mass fraction on SPION loading and vesicle morphology was investigated at a fixed lipid concentration of 0.5mg/ml. The SPION concentration was varied from 1 to 20%w/w and prepared by solvent inversion. The lipid-nanoparticle mixture was dissolved in 1ml THF and added dropwise into 10ml of Milli-Q water under magnetic stirring at room-temperature. THF was evaporated for 12h in an open vial under constant magnetic stirring in a well-ventilated area. The as-prepared magnetoliposome suspensions were stable for >3 weeks in plastic cuvettes at both 4°C and RT.

Figure 3a shows the DLS curves and Fig. 3b the UV/VIS spectra after evaporation of the organic solvent. The SPION loading content of the LUVs was evaluated by comparing the obtained OD values at 350nm to those of the calibration curves of pure SPIONs in THF (Figs. 15-16). The linear range of the calibration curve was limited to 0.1 mg/ml (20%w/w) SPIONs by strong absorption of the iron oxide cores. As shown in the table in Fig. 3d, excellent agreement was found between the input of SPION and the weight fractions measured by UV/VIS. Precipitation was also not observed for 20%w/w SPION input, which indicates that all SPIONs were loaded into the vesicles and quantitative loading was achieved.

The amount of incorporated SPIONs was cross-evaluated by thermogravimetric analysis (TGA) and resulted in close agreement with the results obtained by UV/VIS (Fig. 3c). Samples were lyophilized after overnight evaporation of THF and taking precautions to exclude any precipitated/non-incorporated particles. The residual inorganic content after thermal decomposition of dry samples between 25-500°C in synthetic air was analyzed. TGA could be used also to quantify the loading content of polydisperse samples, e.g. those obtained at high impure SPION input (see next section) which were inaccessible to UV/VIS determination; TGA is, however, less accurate especially at low inorganic fractions. The incremental steps of increasing nanoparticle concentrations were well reflected by the residual masses at 500°C although the amount of remaining, non-combusted lipid was substantial (Fig. 3c). Fatty acids, and therefore lipids, do not fully combust under inert atmosphere but yield carbonaceous residue which also causes reduction of the iron oxide core at elevated temperatures. Heating SPIONs in air yields more complete organic combustion but oxidizes magnetite at around 520°C. Lipid combustion was incomplete even in oxygenic atmosphere at 500°C with a significant residual mass of 19%w/w found for lipid samples without SPION. The embedded SPION fractions reported by TGA therefore relate to the observed mass excess above the background level of remaining lipid. The deviation from the input concentrations was most pronounced for the lowest SPION ratio, which we attribute to these errors. Higher SPION input yielded more emphasized multistep TGA profiles (cf. Fig 3c). Multistep profiles can be caused by the presence of species with different decomposition temperatures or by pronounced interactions of lipids with the nanoparticle shell.

Representative TEM images of the 5%w/w loaded vesicles fixed in 1% trehalose exhibit spherical morphology with a size distribution that agrees well with that obtained by DLS (Fig. 4). The hydrophobic SPIONs are distributed within the observed vesicles rather than dispersed in the background. The employed nanoparticle input of 5%w/w corresponds to 0.3‰n/n or 26 SPIONs per 100nm liposome (see Example 4). It is challenging to determine the number of SPIONs per liposome from TEM images, since the actual size of the vesicles is slightly altered by fixation (partial collapse) and because the observed number of particles depends on the focus, but the obtained micrographs are in general agreement with the expected SPION to lipid ratio. The number of SPIONs per liposome is significantly higher than previously estimated by TGA and SANS for rehydrated and extruded liposomes.

**Example 10: Influence of residual oleic acid from SPION synthesis on assembly behavior**

The size distributions of the SPION-lipid assemblies prepared by solvent inversion were significantly influenced by the purity of the SPIONs. The presence of residual impurities was confirmed for magnetoliposome preparations with incompletely purified SPION samples by recording ATR-FTIR spectra of the lyophilized preparations. Freely associated or physisorbed oleic acid shows up for samples prepared with incompletely purified SPIONs at higher input concentrations as a shoulder at 1705$cm^{-1}$ (Fig. 5). No such bands were observed in the case of stringently purified P-NDA SPIONs or their magnetolipos0me preparations (Fig.5). An estimate of the free oleic acid content of the employed SPIONs was obtained according to Klokkenburg et al. (supra) (Fig. 17-19). Evaluation of the relative IR peak intensities yielded 11%w/w physisorbed oleic acid or 29 molecules per SPION. This corresponds to a significant mole-fraction of free oleic acid per liposome of around 1.5%n/n for a 5%w/w SPION input. For the same preparation conditions, the addition of incompletely purified hydrophobic SPIONs containing residual physisorbed oleic acid initially only slightly shifted the scattering maximum of the formed vesicles to higher diameters than in the unloaded case but gave rise to a bimodal distribution above 5%w/w input (see Fig. 5a). At further increased SPION input the size distributions became increasingly

ill-defined with intense polydisperse micron-sized contributions.

This polydispersity with different types of aggregates could explain the quantitatively different OD curves for standard and spectroscopically pure SPIONs, where for the latter only P-NDA could be identified on the particles by IR spectroscopy (Fig. 5b and 17). A significant increase in OD as compared to unloaded and clean reference samples was observed for impure SPION input above 5-10%w/w. This matches the concentration range above which increasingly polydisperse morphologies were observed in DLS.

For spectroscopically pure SPIONs the resulting assemblies showed similar size distributions as for the unloaded case up to >20%w/w SPION input. The PDI for loaded and unloaded preparations were comparable at 0.2. In contrast we observed an upper loading limit for impure SPIONs at around 10%w/w (Fig 3), which could only be verified by TGA due to the increasingly polydisperse samples at higher concentration that precluded quantification by UV/VIS (Fig. 17). While impure nanoparticles tended to precipitate at input contents approaching 10%w/w, clean SPIONs did not show any visual precipitation in the investigated range (Fig 3).

Another striking difference was observed in relation to formation of magnetoliposomes in different buffers (Fig. 18). PBS showed a feature-less, polydisperse distribution of large aggregates from 100-10000nm when SPIONs containing residual OA were employed (Fig. 19). In contrast, TBS yielded quasi-monodisperse vesicles with a major component around 250nm, similar to preparations in $H_2O$. In the case of isotonic NaCl solutions (140mM) a narrow bimodal DLS distribution with the main populations around 100nm and 250nm was found, similar to for TBS. For spectroscopically clean SPIONs overlapping monodisperse size distributions were observed of vesicles slightly larger than 100nm for PBS and TBS (Fig. 18). Thus, the destabilizing effect of the phosphate ions on assembly is higher when there is potential free oleic acid in the liposome membranes.

The demonstration of the strong influence of the partitioning of small amounts of residual oleic acid from the particles to the lipid membrane on the magnetoliposome assembly and stability underscores how important well-defined and characterized starting materials will be for production of, for example, triggered drug delivery liposomes. Accumulation and partitioning of amphiphilic solutes is determined by the molecular structure of the detergent and the phase state of the lipid membrane.

**Example 11: Controlling magnetoliposome size Low concentration regime - THF:$H_2O$ ratio**

A variation of the inversion ratio between 1:5 to 1:20%v/v (THF:$H_2O$) showed that the obtained size distribution of formed vesicles can be tuned by adjusting the solvent-to-water ratio for lipid concentrations below 1 mg/ml (Fig. 20). The resulting average size was ~150nm for high THF-to-$H_2O$ ratio (1:5) and ~90nm for lower ratios (1:10 and 1:20). Differences in size were also observed for SPION-loaded versus unloaded vesicles when formed at constant inversion ratio of 1:10. Loaded assemblies were slightly larger than their unloaded counterparts with diameters of 110nm compared to 89nm.

**High concentration regime - post-extrusion**

Assemblies formed above a lipid concentration of a few mg/ml were characterized by poor control over size, lamellarity and long-term stability, but formation of concentrated vesicle samples is preferred for applications. Post-extrusion through 100nm pore-size track-etched polycarbonate membranes after complete evaporation of the organic solvent resulted in unilamellar preparations of controlled size also at high lipid concentrations. Surprisingly this approach allowed producing magnetoliposomes with higher SPION content compared to rehydration and extrusion (Fig. 21). Loss of nanoparticulate material is presumably minimized by the more similar and homogeneous size and loading of vesicles formed by solvent inversion compared to by rehydration. At high SPION input (>10%w/w) the loss of nanoparticles through extrusion became more pronounced also for vesicles pre-formed by solvent inversion. The loss of SPIONs upon post-extrusion were 8% and 24% of the total 5 and 10%w/w SPION inputs respectively (Fig. 21).

**Influence of SPION size on assemblies**

Differently sized SPIONs (3.5, 4.5 and 8.3nm) were tested for loading into POPC vesicles by solvent inversion. The SPIONs of different sizes exhibit similar grafting densities but vastly different chain end densities at the outer particle surface due to the increasing free volume at the outer shell for higher particle curvature (decreasing size). This particle size dependent thinning of the ligand shell yields 3nm SPIONs with light shells and large interaction volumes for surrounding solutes whereas 8nm SPIONs show roughly three times higher shell density at the outer surface.

Solvent inversion with all SPION sizes yielded markedly colored suspensions without precipitation. DLS showed comparable size distributions with scattering maxima around 100nm for all preparations (Fig. 22). TEM of 4.5nm SPION-loaded liposomes (Fig. 7) showed homogeneous distribution of nanoparticles among the lipid vesicles, similar as often observed for 3.5nm SPIONs (Fig. 4). Including the expected thickness of the P-NDA shell, this size is likely at the border

of what can be fitted into a lipid bilayer.

Addition of 8.3nm SPIONs to lipids via solvent inversion resulted in dispersed nanoparticles. However, a closer inspection in TEM of samples with 8.3nm SPION showed exclusive nanoparticle localization in lipid droplets, i.e. SPION aggregates surrounded by a lipid (mono-)layer. These SPION-lipid droplets co-exist with unloaded vesicles (Figs. 7 and 24). In the literature it is often suggested that micelle formation occurs around single SPIONs too large to fit into a lipid membrane due to unfavorable bending energy. However, in our 8nm SPION sample we only observed formation of droplets seemingly containing multiple cores, which have strong similarities with the aggregated nanoparticle inclusions in vesicle membranes.

To investigate the fraction of particle aggregates we employed absorption spectroscopy before and after magnetic chromatography of the samples on a magnetic column. UV/VIS was used to assess the amount of aggregates formed (Fig. 22). Dilute and non-aggregated SPIONs, for example well dispersed in vesicle membranes, are not possible to retain in such columns.

Samples prepared with 8.3nm SPIONs lead to almost complete removal of SPIONs during magnetic chromatography even at 5%w/w input (Fig. 22). Unloaded liposomes with identical size distributions measured by DLS before and after elution from the column could be detected. This behavior clearly correlates with TEM observations of nanoparticle aggregates in lipid droplets. Vesicles loaded with 5%w/w of 3.5nm SPIONs were eluted, indicating magnetoliposomes. Higher SPION fractions (e.g. 20%w/w) did not pass the magnetic column. This either indicates SPION clustering in lipid droplets or that a high SPION-loading in the membrane induced strong magnetic interactions with the column material, which could occur either through aggregation or by the high number of SPION per vesicle. 20%w/w SPION could be accommodated in liposome membranes, since it corresponds to approximately a third of a hexagonally closed packed SPION monolayer (~60%w/w) within 100nm liposome membranes (see Example 4). TEM inspection of samples fixed in trehalose showed loaded liposomes with indications of spherical areas containing nanoparticles. Droplet formation for small core sizes could however not unequivocally be identified in TEM since similar features (high contrast areas) were also observed for samples of lower loading content that easily passed the magnetic column just as for preparations with large SPIONs (Fig. 23). Moreover bursting of vesicles and spreading of nanoparticles during transfer to a high vacuum system is commonly observed for fixed vesicles. Additionally, we also could not detect vesicles by DLS in the eluate for high loading contents of small SPIONs, which showed monodisperse LUV by DLS before the column. Thus, all vesicles remained trapped on the magnetic column. The most plausible interpretation is therefore that at high loading of small SPIONs predominantly LUVs are formed with sufficient net inducible magnetic moment to allow facile magnetic extraction of the magnetosomes.

**Example 12: Magnetosome stability**

Magnetoliposomes (5%w/w 3.5nm SPION with POPC lipids) were stored in PMMA cuvettes at room temperature and at 4°C under ambient atmosphere. Sample integrity was confirmed at various time intervals using DLS and found to be preserved for at least one month (Fig. 8). The hydrodynamic size (intensity weighted average diameter) varied by less than 5% during storage at both room temperature and at 4°C while the PDI varied by 0.1 for the narrow distributions (Fig. 8).

**Example 13: Polymer synthesis**

Polyisoprene macroRAFT agent (1): HOOC-PI(1300)-DTB

Polyisoprene macroRAFT agent (1) was prepared as in reference with slight modifications. RAFT agent (81 mg, 0.29 mmol) and AIBN (24 mg, 0.146 mmol) were weighed into a thick walled glass tube. $N_2$-saturated anhydrousTHF (5.5 mL) and isoprene (6 mL, 59.9 mmol) were added and the resulting mixture was sealed under inert atmosphere. The glass tube was placed in a preheated oil bath (T = 125°C) and polymerized for 2 h. The tube was then allowed to cool down to room temperature the content was concentrated in vacuo. The resulting red-pinkish viscous oil was taken up in minimal DCM and precipitated in methanol. Compound (1) was collected by centrifugation (5000rpm/10min/rt), washed with methanol and dried in vacuo. Yield: 225 mg (5.5 %). The macro RAFT agent was dissolved in $N_2$-saturated, anhydrous dioxane at a concentration of 75 mg/mL and stored at -20°C until further use. $^1$H-NMR (300 MHz, $CDCl_3$, $\delta$): 7.98 (d, 2H, *J* = 7.5 Hz, Ph), 7.51 (t, 1H, *J* = 7.3 Hz, Ph), 7.37 (t, 2H, *J* = 7.4 Hz, Ph), 5.76 (1H, 1,2-PI), 5.12 (1H, 1,4-PI), 4.90 (2H, 1,2-PI), 4.69 (2H, 3,4 PI), 4.01 (t, 2H, *J* = 7.9 Hz, $CH_2$-S-C(S)), 1.5-2.3 ($CH_2$, $CH_3$ PI). UV/VIS (1,4-dioxane, $\lambda_{abs}$, nm): 280 (Ph-), 296 (C=S), 334 (sh), 500 (Ph(C=S)S)

The PI-block of the macro-RAFT agent displayed the following microstructure: 90 % 1,4-addition (cis/trans ~ 2/1), 5 % (1,2-addition) and 5 % (3,4-addition).

**Polymerization of *N*-isopropylacrylamide ([M]/[macroRAFT agent]: 167/1): HOOC-PI(1300)-*b*-PNIPAM(1000)-DTB**

The thermoresponsive PNIPAM blocks were prepared similar to Shan et al.(Macromolecules 2009, 42, 2696.). Macro-RAFT agent **1** (1.33 mL, 75 mg/ml) was added to a solution of NIPAM (1.52 g, 13.4 mmol) and AIBN (0.82 mg, 0.005 mmol) in anhydrous dioxane (6.4 mL). After purging the solution with nitrogen for 20 min, the flask was immersed into a preheated oil bath (70 °C) for 20 h. After cooling down, the flask was attached to a high vacuum system to remove dioxane and sublimate residual monomer. The crude residue was washed with hot water several times and subsequently freeze-dried.

**RAFT head group removal: HOOC-PI(1300)-*b*-PNIPAM(1000)-SSMe (2)**

Cleavage of the DTB headgroup was conducted according to a modified procedure of Roth et al.(Macromolecules 2008, 41, 8316.). The light orange polymer was dissolved in anhydrous THF (4 mL) and mixed with *S*-methyl methanethiosulfonate (188 μL, 2.25 mmol). After purging the resulting solution with nitrogen, (2-dimethylamino) ethylamine was dropwise added via a syringe (110 μL, 1 mmol). Discoloration to a faint-yellow solution is indicative of dithioester removal and was observed within 3h. To assure complete conversion, the reaction was allowed to stir overnight. The solution was concentrated and the residue was washed with water and methanol. After drying, the crude product was purified via silica gel column chromatography. First, residual polyisoprene was eluted using DCM/MeOH 100/1, then block copolymer **2** was obtained using DCM/MeOH 6/1 as eluent. Yield: 38 mg (21%).

$^{1}$H-NMR (300 MHz, $CDCl_3$, δ): 6.90 (1H, NH, PNIPAM), 5.76 (1H, 1,2-PI), 5.12 (1H, 1,4-PI), 4.90 (2H, 1,2-PI), 4.69 (2H, 3,4-PI), 4.00 (1H, s, C*H*$(CH_3)_2$ PNIPAM), 0.8-2.2 ($CH_2$, $CH_3$ PI, $CH_2$, CH, PNIPAM), calculated from the $M_n$ (MALDI-TOF MS) the block copolymer composition is $PI_{17}$-*b*-$PNIPAM_{8.5}$

$^{13}$C-NMR (75 MHz, $CDCl_3$, δ): 174.6 (C=O, PNIPAM), 135.1 (1,4 C=C, PI), 125.0 (1,4 C=C, *cis,* PI), 124.2 (1,4 C=C, *trans,* PI), 111.2 (1,2 and 3,4 C=C, PI), 41.6 (CH-CO, PNIPAM), 39.8 ($CH_2$, PI), 38.5 ($CH_2$, PNIPAM), 32.0 ($CH_2$, PI), 29.7 ($CH_2$, PI), 28.3 (PNIPAM), 26.7 ($CH_2$, PI), 23.5 ($CH_3$, PNIPAM), 22.5 ($CH_3$, 1,4-*cis*, PI), 16.0 ($CH_3$, 1,4-*trans,* PI).

MALDI-TOF MS (DHB, no salt added) $M_n$: 2337 g/mol, polydispersity: 1.14. For [M]/[Macro RAFT agent]: 167/1 a BCP with 40 vol-% PNIPAM was obtained.

ATR-FTIR (powder, $cm^{-1}$): 3600-3200 (b, -OH), 3300 (NH, amA), 3070 (=$CH_2$, 3,4-PI), 2966 ($CH_3$), 2924 ($CH_2$), 2874 ($CH_3$), 2854 ($CH_2$), 2234 (CN), 1715 ((C=O)OH), 1642 (C=O, amI + C=C, 3,4 & 1,2 PI), 1540 (NH, amII), 1453 ($CH_3$, PNIPAM), 1383 ($CH_3$, t-1,4-*trans* PI + PNIPAM), 1368 ($CH_3$, PNIPAM), 1264 (NH, amIII), 1172, 1130 (C-C, *c-1,4-cis* PI), 1098, 1027 (=C-$CH_3$, *c-1,4-cis* PI), 1004 (C-C, 3,4-PI), 909 (=$CH_2$, 1,2-PI), 886 (=$CH_2$, 3,4-PI), 840 (-CH=CH-, c,t-*1,4-cis,trans* PI), 690 (NH, amV), 510

UV/VIS (MeCN, $\lambda_{abs}$, nm): 208 (CONH), 272 (sh, -SSMe))

**Example 14: Polymer vesicle formation and release study**

**Solvent inversion:** Magnetic polymersomes were prepared by self-assembly of the amphiphilic block copolymer poly(isoprene-b-N-isopropylacrylamide) (PNIPAM) with monodisperse hydrophobic superparamagnetic iron oxide nanoparticles (SPION). A PI-b-PNIPAM block copolymer (BCP 2) with thermoresponsive volume fractions of 40%v/v was prepared by sequential RAFT polymerization. Multilamellar vesicles (MLVs) were formed by a protocol modified from Dorn et al.(Macromol. Biosci. 2011, 11, 514). Typically 4mg block copolymer were mixed with the respective weight percentage of hydrophobic SPIONs and dissolved in 200μl THF. The mixture was dropwise added into 2ml aqueous medium (buffer or ultrapure water) containing 5mg/ml calcein (0.2μm filtered) under magnetic stirring. The solvent was evaporated at room-temperature under a constant $N_2$ stream for 3h and while adding Milli-Q to keep the original total volume. The as-prepared vesicle suspension was extruded 10-times through 100nm track-etched polycarbonate membranes in a hand-held extruder (Avanti) to increase the encapsulation efficiency and improve lamellarity.

**Release assays:** Removal of nonencapsulated dye and free nanoparticles from the extruded samples was performed on a Bio Logic Duo Flow chromatography system equipped with a UV-detector, a Knauer Smartline RI 2300 detector and a Bio Logic BioFrac collector. In detail, the samples (2mL, 2mg/ml) were purified by passing over a FPLC-column (length × diameter: 60cm × 3cm, stationary phase: Superdex 75) in Milli-Q water with a flow rate of 0.75 mL/min. Fractions of 2mL containing the desired sample (usually 4 fractions) were identified by UV and RI detection. The sample concentration decreased to 0.5 mg/mL by the purification process.

**Magnetic Actuation:** The as-prepared sample was filled in a PMMA cuvette which was placed in an Ambrell Easy Heat LI magnetic heater, with a current of 438.9A and a frequency of 228kHz, coil dimension (height × outer diameter × coil thickness × number of turns = 37 mm × 37 mm × 2 mm × 6). Magnetic actuation was performed in 8 or 10 min cycles, with a delay of 5 min between the cycles for recording of the released amount of calcein via fluorescence spectroscopy.

**Fluorescence measurements:** Fluorescence spectra were collected with a PerkinElmer LS 55 luminescence

spectrometer at an excitation wavelength of 495nm and an emission wavelength of 515nm with a scan speed of 100nm/min and a slit width of 2.5nm. In some cases, the sample was diluted further in order to be within the optimal working range of the photo detector. Release of calcein was calculated according to the formula

$$Release\% = \frac{I_i - I_{AMF\,/\,PL}}{I_i - I_{tot}}$$

where $I_i$ is the initial fluorescence intensity measured immediately after column purification, $I_{AMF}$ is the fluorescence intensity measured after the sample was subjected to individual AMF treatments and $I_{PL}$ is the fluorescence intensity measured at different times without applying any AMF in order to calculate passive leakage. $I_{tot}$ is the total fluorescence intensity measured after complete lysis of the vesicles by addition of Triton X100 (10%v/v of 20% Triton in MQ water).

**Example 15: Determination of the iron oxide nanoparticle loading**

For TGA determination of the effective SPION content of the polymersome membranes the lyophilized samples were burnt under oxidative conditions (synthetic air) to yield near complete combustion. Yet a considerable residue (~11% w/w) remained even in the case of polymersomes containing no SPIONs. The reported final SPION loading content therefore refers to the non-combusted material at 650°C in excess of the residue for samples not containing nanoparticles, which amounts to approximately 9%w/w for extruded SPION loaded samples.

Optical density (OD) values at 350nm ($OD^{350}$) were used for spectroscopic quantification of the SPION embedding efficiency. The $OD^{350}$ values were obtained by dilution of the respective suspensions to match the amide absorptions at 208nm. Background spectra of the plain extruded PI-*b*-PNIPAM vesicles were recorded to account for vesicular scattering. The $OD^{350}$ value of the initial SPION loaded suspension was assigned to the input SPION weight fraction (20%) and the final loading content was determined by evaluating the $OD^{350}$ decrease upon extrusion. In this way we estimate an effective loading content of around 9%w/w which is similar to the one obtained by TGA.

**Example 16: Comparison of polymeric vesicles**

Vesicle formation of SPION-loaded BCP **2** depended on experimental conditions such as preparation method, temperature, aqueous phase composition and additional energy input (e.g. sonication). Initial attempts to produce loaded vesicles via standard rehydration in Milli-Q / calcein (5mg/ml; 0.2μm filtered) or phosphate buffered saline (1x PBS; 10mM $NaHPO_4$ / 150mM NaCl) / calcein solution required improvement because of minimal dispersion of the nanoparticle / BCP **2** film into those phases at ambient conditions. Neither gentle temperature variations nor sonication improved on vesicle formation.

Vesicles of BCP **2** ($M_n$ ~ 2300g/mol, Đ = 1.14, Φ (PNIPAM) = 40% v/v) were instead prepared at 1 mg/mL by solvent inversion into ultrapure water and calcein.

Dynamic light scattering (DLS) showed structures with a broad distribution of hydrodynamic sizes of 0.1-1 μm for the turbid as-prepared suspension (Figure 25A). TEM of the same sample showed spherical structures with a size distribution similar to the one obtained by DLS, further supporting successful formation of polydisperse block copolymer vesicles (Figure 25B). Figure 1A also shows the results of temperature-dependent DLS in the range of 25-75°C in 5°C steps. During temperature cycling, the initial broad distribution sharpened at 30°C to a maximum centered at 250nm. In the range from 35 to 70°C the hydrodynamic diameters only shifted slightly to approximately 200nm but steadily increased in intensity to ultimately settle at 7-fold of the initial value at 50°C. No further change in size distribution up to 70°C was observed. This result demonstrates the thermoresponsiveness of BCP **2** vesicles with a transition temperature range of 35-50°C; this is higher than the typical literature value of 32°C, but an increased LCST and even suppression of the collapse of the coil is expected for low molecular weight PNIPAM in an amphiphilic environment.

Multilamellar large vesicles are of limited use for release applications. Standard methods to enforce unilamellarity and decrease vesicle size are sonication and extrusion. Sonication at constant *T*=20°C led to polymer and nanoparticle precipitation. Extrusion through track-etched polycarbonate membranes caused loss of hydrophobic SPIONs and some polymer but did not lead to precipitation. The measured DLS curves and $OD^{350}$ values (Fig. 26) of the extruded preparations matched the expected changes based on similar preparations of liposomes, for which the lamellarity is known to be reduced. The solution became clearer, which indicates a reduction in size but primarily a lower fraction of multilamellar vesicles. We therefore used extrusion (10x, 100nm polycarbonate membranes) to create monodisperse unilamellar thermoresponsive polymersomes encapsulating calcein in the lumen, while retaining a high SPION content.

DLS size distributions (159±66 nm) and TEM of extruded SPION-loaded vesicles with encapsulated calcein are shown in Figure 26A-B. The orange-brown suspensions after extrusion were clear as expected for predominantly unilamellar vesicles. More SPION than polymer are lost in the extrusion and for an initial input of 20%w/w 3.5nm SPION

we determined an incorporated weight fraction of around 10%w/w by TGA (rest mass after thermal decomposition relative to total organic content) and UV/VIS spectroscopy (characteristic wavelength at 350nm) (see Fig 26C-D).

The fluorescent dye calcein was encapsulated at self-quenching concentrations. Samples were purified from excess dye by size exclusion chromatography over a Superdex 75 FPLC column in ultrapure water and fractionated according to UV absorption and refractive index. The purification reduced the sample concentration to 0.5 mg/mL.

Release of encapsulated calcein to the bulk phase was quantified by recording the increase in fluorescence intensity as function of time and membrane actuation. The change in fluorescence intensity was obtained after subtraction of background fluorescence and normalizing to the total fluorescence after disruption of the vesicles by Triton. Magneto-thermal release was triggered by applying an alternating magnetic field (AMF) of variable duration and intensity. The resulting relative increase in fluorescence was compared to the passive release in absence of an applied field. The fluorescence resulting from triggered release of calcein from PI-*b*-PNIPAM polymersomes with 3.5nm SPIONs incorporated in the membrane is shown in Figure 27. The AMF causes heat to dissipate locally from the magnetic cores due to Néel relaxation. The generated heat causes dehydration of the hydrated PNIPAM comprising the outer part of the polymersome membrane when the local temperature exceeds its LCST. The resulting change in amphiphile packing parameter affects membrane integrity and hence alters permeability. It was found that only a long pulse duration of 10min led to significant release. Application of one pulse of 8min duration triggered only 3% release of entrapped calcein whereas application of one 10min pulse triggered release of 25% of encapsulated calcein as shown in Fig 27a. Similar release was achieved for 8min pulses only after 4 repetitions. This pulse length is significantly longer than required for release from liposomes with $T_m$ comparable to the LCST of the PI-*b*-PNIPAM and with similar nanoparticles incorporated in the membrane. As comparison, DPPC ($T_m$ = 41 °C) liposomes with 4% loading of 3.5nm SPION released 90% of encapsulated calcein after two 4-min pulses. For liposomes, the release has been demonstrated to be due to a change in membrane permeability by direct heating of the membrane by the nanoparticles without requiring bulk heating. The long pulse duration necessary for triggered release from the PNIPAM vesicles indicates that purely local heating of the PNIPAM to cause a thermal transition is not likely to have been achieved. This is further supported by that the bulk temperature at the end of the AMF pulse application exceeds the temperature required for thermal transition of the polymer (Figure 25).

Fig 27A shows that the release plateaued close to 50% of the encapsulated calcein set free. Since the chosen preparation method strongly favors formation of unilamellar vesicles, as supported by OD measurements, it is likely that an inhomogeneous distribution of SPIONs between different polymersomes is the main reason for that only half of the encapsulated calcein could be released. The lower contrast of some of the small polymersomes observed in TEM (Fig. 26B) could indicate low SPION loading in small vesicles and that high particle loading is required for efficient release. The passive release during the period leading to actuated release is negligible (Fig. 27A). However, after 5h storage the passive release reached close to 20% with a linear release profile. The relatively high passive leakage over long time scales might be caused by per-methylation of the hydrophobic core material which may render liposomes more permeable.

The PI-*b*-PNIPAM polymersomes showed reversible decrease in hydrodynamic size upon increased temperature rather than disintegration of the whole vesicles. This behavior was independent of the upper temperature (35°C, 45°C or 55°C). Also for extruded vesicles no significant change in scattering intensity or size was observed after reversible heating (Fig. 27B-C). We therefore attribute reversible, thermally induced vesicle shrinking to a reversible partial dehydration of the interfacial PNIPAM corona that changes the membrane integrity but does not alter the vesicle topology. Thus, permeability could be increased without disassembly of the vesicles. Similarly to magnetically actuated liposomes we also observe that the release could be dosed by application of multiple pulses, realizing a major advantage of field-triggered release. Although the release behavior of our polymeric vesicles parallels that of lipid analogues during magneto-thermal actuation we note that there are fundamental differences in the underlying mechanism due to different intermolecular interactions among the constituent amphiphiles. Lipid membrane dynamics are governed by collective behavior such as lateral mobility, while polymer actuation primarily proceeds intramolecularly through local chain dehydration of the hydrophilic block and concomitant changes in the packing parameter and preferred assembly structure.

**Example 17: Manufacture of fluorescent hybrid polymersome vesicle**

**Reagents and Materials**

Meldrum's acid (2,2-Dimethyl-1,3-dioxane-4,6-dione) 98%; 4-(Diethylamino)salicylaldehyde 98%; Piperidine ReagentPlus 99%; Glacial acetic acid ACS reagent >99.7%; %; 1,4-Diazabicyclo[2.2.2]octane ReagentPlus ≥99%; Succinic anhydride >99% (GC); Dicyclohexylcarbodiimide puriss ≥99% (GC); 4-(Dimethylamino)pyridine ReagentPlus ≥99; *N,N*-Diisopropylethylamine ReagentPlus ≥99%; *N,N*-Diethyldiethylenetriamine 98%;
Phosphate buffered saline tablets (0.01 M phosphate buffer, 0.0027 M potassium chloride and 0.137 M sodium chloride, pH 7.4), Milli-Q water (Millipore USA; R=18MΩcm); Ethanol anhydrous ≥99.8%; Acetone Chromasolv Plus for HPLC

≥99.9%; Dichloromethane anhydrous ≥99.8% (contains 40-150ppm amylene as stabilizer); Tetrahydrofuran Chromasolv Plus for HPLC ≥99.9% (inhibitor-free);

Polybutadiene(1200)-block-polyethyleneoxide(600) was obtained from Polymer Source Inc. 1,2-dioleoyl-sn-glycero-3-ethylphosphocholine chloride salt ($DOPC^+$) was obtained from Avanti Lipids Inc. Branched poly(ethylene imine) ($<M_w>$ ~ 800 g/mol by LS, $<M_n>$ ~ 600 g/mol by GPC) was purchased from Sigma Aldrich.

**TEM and analysis:** TEM studies were performed on a FEI Tecnai G2 20 transmission electron microscope operating at 160 kV. Samples were prepared by loading freshly cut ultrathin-sections onto 300-mesh carbon-coated copper grids and subsequently air drying them overnight.

**Confocal Microscopy:** All images were recorded on a Leica SP5 II Laser Scanning Confocal Microscope equipped with LCS software and a HCX APO L 40x/0.80 objective. Samples were excited at 405 nm (cw, 50 mW) and their emission was collected. Samples were imaged in transmission mode by placing one drop of the cell culture onto glass cover slips or into plastic wells mounted onto glass cover slips (manufacturer). The temperature of microscope stage was controlled with warmed platforms (manufacturer). All spectra were corrected for autofluorescence of the cells.

**Dynamic Light Scattering:** Hydrodynamic diameters and Zeta potentials were recorded on a Malvern Zetasizer Nano-ZS (Malvern UK) in PBS (1x; 10mM NaHPO4, 2.7mM KCl, 137mM NaCl, pH=7.4) at 25°C in 173° backscattering mode. Samples were equilibrated for 120 sec. each and the autocorrelation function was obtained by averaging 3 runs. Samples were measured at 100μg/ml.

**$^1$H- NMR measurements:** $^1$H -solution spectra were collected on a Bruker DPX spectrometer operating at 300MHz. Chemical shifts were recorded in ppm and referenced to residual protonated solvent ($CDCl_3$: 7.26 ppm ($^1$H).

**ESI-MS measurements:** Mass spectra were collected using a Q-Tof Ultima ESI (Waters, USA) mass spectrometer in positive ion mode (range 100-1500 Da). Samples were dissolved in MeOH and diluted to 100μg/ml.

**ATR-FTIR measurements:** Mid-IR powder spectra of the lyophilized samples were collected using a Bruker Tensor 37 FTIR spectrometer with a Bruker Platinum Diamond single reflection ATR equipment at a resolution of $4cm^{-1}$ by averaging 32 scans.

**UV-Vis measurements:** UV-Vis absorption spectra were collected at a scan speed of 400nm/min on a Hitachi UV-2900 spectrophotometer.

**Fluorescence measurements:** Fluorescence spectra were collected with a PerkinElmer LS 55 luminescence spectrometer with a scan speed of 400 nm/min and a slit width of 2.5 nm.

**TGA/DSC measurements:** Thermograms were recorded on a Mettler-Toledo TGA/DSC 1 STAR System in the temperature range 25-650°C with a ramp of 10K/min under 80mL/min synthetic air gas flow. The mass loss was evaluated by horizontal step setting.

**Synthesis of_N-palmityl-6-nitrodopamide capped superparamagnetic iron oxide nanoparticles (P-NDA SPIONs)**

Monodisperse 5 nm P-NDA capped SPIONs were prepared as above.

In a typical preparation 1ml of iron pentacarbonyl ($Fe(CO)_5$) was quickly injected at 100°C into a $N_2$-saturated solution of 50ml dioctylether ($Oct_2O$) containing different amounts of oleic acid (OA), e.g. 4 ml OA for 5 nm SPIONs. An equilibration period of 30min was employed to ensure homogenous formation of iron-oleate complexes. The solution was then gradually heated to 290°C with a ramp of 3K/min. The final temperature was held for 1h to obtain the desired particle sizes.

The as-synthesized magnetite nanoparticles were subsequently cooled to room-temperature, precipitated in excess EtOH, collected by magnetic separation and purified by repeated precipitation (toluene into EtOH) / magnetic decantation steps.

For irreversible grafting with N-palmityl-6-nitrodopamide 200mg of as-synthesized OA-NP were purified from excess physisorbed OA by repeated sonication with 50mg of Cetyltrimethylammoniumbromid (CTAB) in hot EtOH. SPIONs were collected by magnetic separation and residual CTAB was extracted with EtOH.

The purified OA-capped particles were taken up in 6ml $CHCl_3$ and mixed with 50mg P-NDA dissolved in 3ml DMF and 9ml of MeOH. The SPION-ligand mixture was sonicated for 3h under $N_2$. $CHCl_3$ was evaporated from the coating mix and the mixed-dispersant SPIONs were collected by magnetic precipitation from excess MeOH (40ml) and purified by three rounds of washing and magnetic separation from hot MeOH (20ml each).

Purified mixed-dispersant SPIONs were subjected to a post-coating step with 100mg P-NDA in 2,6-lutidine at 50°C for 48h under inert atmosphere and magnetic stirring. Lutidine was evaporated, the particles were washed three times with excess hot MeOH and lyophilized from THF:Milli-Q (1:5).

*ATR-FTIR ($cm^{-1}$) : 3600-3000 (b; CONH, OH), 2955 ($CH_3$), 2921 ($CH_2$), 2851 ($CH_2$), 1632 (CONH), 1546 (CONH), 1492 (C=C, $NO_2$), 1468 ($CH_2$), 1437 (C=C), 1374 ($CH_2$), 1320 ($NO_2$), 1276 (C=C, CO), 1226, 1186, 1117, 1098, 1048 (CO), 880 (PhH), 814 (PhH), 571 ($Fe_3O_4$), 385 ($Fe_3O_4$) TGA($O_2$, %w/w)*: -32; $\rho^{graft}$= 2.8/$nm^2$

**Synthesis of 7-(Diethylamino)-coumarin-3-carboxylic acid (DEAC-CA)**

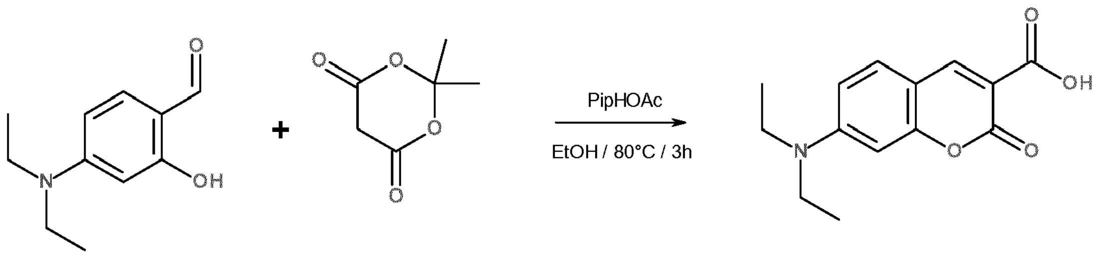


DEAC-CA was prepared by Knoevenagel condensation of para-substituted *ortho*-hydroxybenzaldehyde with *alpha*-C-H acidic Mel-drum's acid. Piperidinium acetate (PipHOAc) was prepared by dissolving 1eq of piperidine in acetone and dropwise adding 1eq. of glacial acetic acid under constant stirring. The white precipitate formed was collected by evaporation of the solvent and dried in vacuo.

A mixture of 4-(diethylamino)salicylaldehyde (20 mmol), Mel-drum's acid (2,2-dimethyl-1,3-dioxane-4,6-dione; 2.89 g, 20 mmol), piperidinium acetate (58 mg, 0.4 mmol) and ethanol (10 mL) was stirred at room temperature for 30 min and refluxed for 3 h. The reaction mixture was allowed to cool down to room temperature, followed by chilling in an ice bath for 1 h. The product was filtered, washed three times with and recrystallized from EtOH. DEAC-CA was obtained as bright orange crystals in ~ *80*% yield.

*$^1H$-NMR ($CDCl_3$, 300 MHz, ppm) : 8.65 (s, 1H, Ph-CH=C), 7. 46 (d, 1H, Ph), 6.72 (dd, 1H, Ph), 6.54 (d, 1H, Ph), 3.50 (q, 4H, $CH_2$), 1.26 (t, 6H, $CH_3$)*

*ESI-MS (MeOH, m/z) : $[M]H^+$ = 262.11, calc. 262.10; $[M]Na^+$ = 284.10, calc. 284.08*

*UV/VIS (MeOH, nm): 217, 259 sh, 423*

*fluor (MeOH, nm): 482 ($\lambda_{exc}$ = 420)*

**Synthesis of Poly(butadiene(1200)-block-ethyleneoxide(600))-O-(7-(Diethylamino)-coumarin-3-carboxylic ester) (PBD-b-PEO-DEAC)**

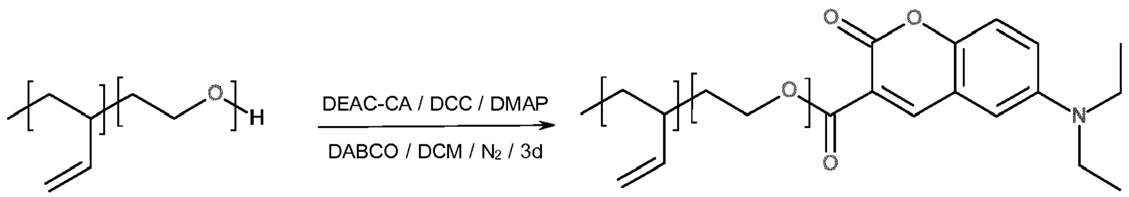


100mg PBD-b-PEO were dissolved in 10ml $N_2$-saturated, anhydrous $CH_2Cl_2$ (DCM) under sonication and subsequently activated for 15 min with 1 eq. of 1,4-Diazabicyclo[2.2.2]octane (DABCO). Next 1.5 eq. DEAC-CA and 0.2 eq. 4-Dimethylaminopyridine (DMAP) were added and the 10% polymer solution was purged with $N_2$ gas for 15min before cooling to 0°C in an ice-bath. N,N-Dicylcohexylcarbodiimide (DCC, 1.7 eq) in 5ml DCM was dropwise added to the magnetically stirred polymer solution at 0°C. The reaction mixture was allowed to slowly warm to room-temperature and reacted in the dark for 3 days under inert atmosphere.

The crude reaction mix was diluted with DCM, extracted thrice with 1M HCl, 5% $NaHCO_3$ and washed with Milli-Q water. The combined organic phases were dried over $Na_2SO_4$, reduced in volume to approx. 5ml and cooled to -20°C. Precipitated DCU was filtered off and the cooling-filtration procedure repeated. The organic phase was evaporated to dryness, taken up in $CHCl_3$, loaded onto a $SiO_2$-column (Silica 60) and washed with several volumes of MeCN to remove excess dye and by-products. The fluorescently labeled target compound was finally eluted in THF:MeOH=4:1. Lyophilization from THF:Milli-Q (1:10) yielded PBD-b-PEO-DEAC as a yellow viscous residue (dye content ~ 5%).

*$^1H$-NMR ($CDCl_3$, 300 MHz, ppm):*

*ATR-FTIR (powder, $cm^{-1}$): 3074, 2913, 2890, 1826, **1735,** 1640, **1622, 1589,** 1514, 1452, 1418, 1343, 1280, 1241, 1143, 1107, 1061, 993, 963, 907, 842, 673, 528*

*UV/VIS (MeOH, nm) : 223, 259 sh, 420*

*fluor (MeOH, nm)*: *474 ($\lambda_{exc}$ = 420)*

**Synthesis of Polybutadiene(1200)-block-Polyethyleneoxide(600)-carboxylic acid (PBD-b-PEO-COOH)**

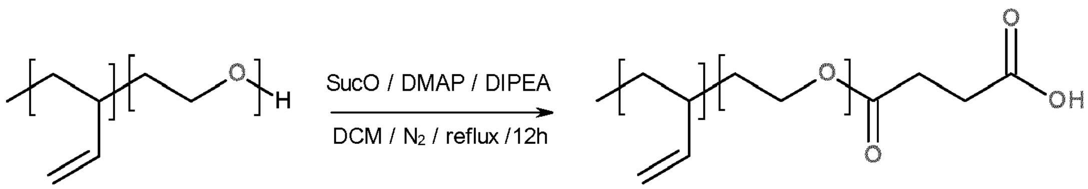


100mg PBD-b-PEO were dissolved in 10ml $CH_2Cl_2$ (DCM) under sonication and activated with 2eq. of N,N-Diisopropylethylamine (DIPEA) for 15 min. 0.2eq 4-Dimethylaminopyridine (DMAP) and 3eq succinic anhydride (SucO) in 2ml DCM were dropwise added to the above solution and purged with $N_2$ for 10min. The reaction mixture was refluxed overnight under inert atmosphere.

The crude product was diluted with DCM, extracted thrice with 1M HCl, 5% $NaHCO_3$, washed with Milli-Q and brine. The organic phases were dried over $Na_2SO_4$, evaporated and dried in high vacuum overnight to yield ~ 95 % of a transparent viscous residue.

*$^1$H-NMR ($CDCl_3$, 300 MHz, ppm):*

*ATR-FTIR (powder, $cm^{-1}$): **3680-3350** (b) 3074, 2913, 2890, 1826, **1735,** 1640, 1447, 1418, 1349, **1330, 1300,** 1249, 1101, 1039, 993, 951, 907, 860, 774, 675, 522*

**Synthesis of Polybutadiene(1200)-block-Polyethyleneoxide(600)-N-{2-[[2-(Diethylamino)ethyl]amino]ethaneamide} (PBD-b-PEO-DEDETA)**

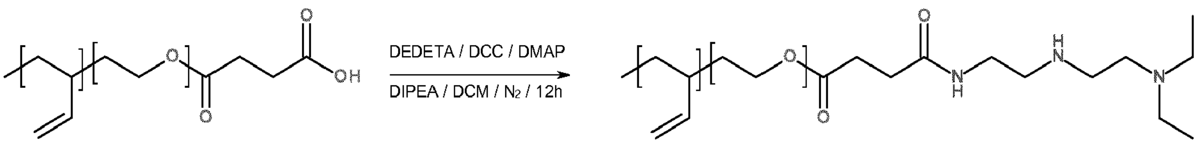


100mg PBD-b-PEO-COOH were dissolved in 15ml N-Methyl-2-pyrrolidone (NMP) under sonication and activated for 15 min at room temperature with 1.1eq. (1-Cyano-2-ethoxy-2-oxoethylidenaminooxy)-dimethylamino-morpholino-carbenium hexafluorophosphate (COMU) and 2eq of N,N-Diisopropylethylamine (DIPEA). The activated acid was dropwise added to a solution of 5eq. N,N-diethyldiethylenetriamine (DEDETA) in 10ml NMP at 4°C, slowly warmed to room temperature and reacted overnight under inert atmosphere.

The crude product was diluted with DCM, extracted thrice with 1M HCl, 5% $NaHCO_3$ and washed with Milli-Q and brine. The organic phases were pre-dried over $Na_2SO_4$, evaporated and dried in high vacuum overnight to yield ~ 95 % of a transparent to off-white viscous residue.

*$^1$H-NMR ($CDCl_3$, 300 MHz, ppm):*

*ATR-FTIR (powder, $cm^{-1}$): **3630-3150** (b;) 3074, 2913, 2890, 1826, **1735,** 1665, 1640, 1540, 1447, 1418, 1378, 1349, **1330, 1300,** 1249, **1219 (solv)**, 1101, 1039, 993, 951, 907, 860, **774 (solv),** 675, 563, 522*

**Example 18: Preparation polymeric magnetosomes by solvent inversion**

Large unilamellar vesicles (LUVs) were formed as above with modifications. Typically 4mg block co-polymers were mixed with the respective weight percentage of hydrophobic SPIONs and dissolved in 200μl THF. The mixture was dropwise added into 2ml aqueous medium (buffer or ultrapure water) under magnetic stirring. The solvent was evaporated at room-temperature under a constant $N_2$ stream for 3h and continuously refilled with Milli-Q to its initial level. To remove non-encapsulated nanoparticles and improve lamellarity, the as-prepared vesicle suspension was homogenized by extrusion through 100nm track-etched polycarbonate membranes (10-times) in a hand-held extruder (Avanti).

DLS size distributions of various nanoparticle-diblock copolymer assemblies are shown in Fig. 28 and ultrathin

sections of nanoparticle-diblock copolymer assemblies are shown in Fig. 29.

**Example 19: Fluorescent labeling**

Fluorescent polymersomes were created by employing the small, hydrophobic dye (7-diethylamino coumarin)-3-carboxyic acid (DEAC-CA). DEAC-CA adds a fluorescent modification to the amphiphilic diblock copolymer poly(butadiene-b-ethylene oxide) (PBD-b-PEO) without perturbing the block-copolymer physicochemical properties. Its small size importantly avoids morphological changes of the assemblies caused by the addition of a fluorescent group. Furthermore, its hydrophobicity causes the dye to locate within the membrane interior rather than being presented at the interface, thereby avoiding undesired interactions with biomolecules. Conjugates of DEAC to PEG are known to be only mildly cytotoxic and possess high photoluminescence quantum yields (PLQY). Coumarins can also serve as reporters for ROSderived high energy species and changes in the local pH. Fluorescent modification of PBD-b-PEO polymersomes with DEAC-CA was achieved by Steglich esterification for 3 days in the dark giving 5% dye content. The rather modest yield is likely to originate from the deactivated character of the terminal acid group which is conjugated to the ring system. A dye content below 10% is however desired to avoid self-quenching effects.

We did not detect any appreciable change in Zeta potential upon conjugation of DEAC-CA to the PEO-headgroup of the diblock copolymer. This is expected for properly purified samples that do not contain excess free dye but a few percent of conjugated entities that are linked via neutral ester bonds. Luminescence spectroscopy on the dye-labeled polymersomes in water exhibited dye emission profiles that are indicative of a low polarity microenvironment. The Stokes shift and PLQY of the assembled DEAC-copolymer-conjugates in water resembles those of the free dye dissolved in a low dielectric solvent (THF) rather than when dispersed in an aqueous phase (Milli-Q water or buffer). We therefore conclude that the majority of the conjugated dye molecules are located within the hydrophobic membrane interior rather than being presented at the vesicle interface. A loss in the overall quantum yield however suggests an equilibrium fraction in contact with water since excited (dialkylamino)coumarins are efficiently quenched in protic, high dielectric solvents by population of twisted intramolecular charge transfer states.

The incorporation of SPIONs generally lead to a drastic decrease in fluorescence intensity but the signal is still easily distinguishable from the cellular autofluorescence background for an employed 10% w/w SPION loading. This is in strong agreement with the result of complete quenching of dyes linked directly by spacers to the SPION and our expectation of the DEAC being localized in the membrane interior. A high concentration of SPIONs in the membrane means that conjugated dyes will be quenched by the close proximity to the nanoparticle acceptor.

**Example 20: Surface modification of polymeric vesicles**

Various surface modification approaches were tested for their potential to enhance the transfection efficiency of stealth polymersomes, with the aim of controlling delivery efficiency. We compare the efficiencies of surface adsorption of membrane-disruptive, low-Mw polymer to covalent modification of the scaffold diblock-copolymer with a short oligoamine sequence to a homogeneous supramolecular blend with cationic lipids ($DOPC^+$) as enhancers to promote cell uptake Branched poly(ethylene imine) or b-PEI is a commonly used non-viral transfection agent comprising a combination of primary, secondary and ternary amines that strongly interact with negatively charged species such as DNA or native cell membranes. Its transfection efficiency can be tuned via the molecular weight and constituent structure. Both high Mw and branching of the polymer increase transfection efficiencies in-vitro. Adsorption of these membrane-disruptive agents to polymeric delivery vesicles was recently exploited as a means of transfection because their mechanical robustness allows for direct coating without breaking down the vesicle's membrane integrity as seen for lipid carriers. Moreover b-PEI is a prerequisite to ensure endosomal escape from lytic organelles which is essential for active compounds to reach their intracellular target. In contrast to artificial liposomes which are inherently sensitive to osmotic changes due to deficiency of proton-pumps, polymer vesicles require a drastic driving force for endosomal escape. An osmotic proton sponge effect is thought to be responsible for endosomal escape of b-PEI coated vesicles while their neutral precursors were shown to be stably trapped in acidic cell compartments without releasing their cargo. Neutral polymersomes require hydrolytic cleavage of the bilayer forming amphiphile to develop lytic properties through a change of the hydrophilic-hydrophobic balance. This approach however displays slow uptake kinetics when PEG is used as non-degradable hydrophilic block. In a first step we increased the affinity of b-PEI to the vesicle surface by carboxylation of the hydrophilic PEO-block via esterification with succinic anhydride prior to adsorption of b-PEI to the modified vesicles. Quantitative endgroup modification was verified by 1H-NMR and FTIR spectroscopy (see SI x). The resulting acid terminated polymer vesicles (-40mV) exhibited a clear shift in Zeta potential of -36mV compared to the unmodified hydroxyl-functionalized diblock-copolymer assemblies (-4mV) in 0.1x PBS. The carboxylic acid modification is therefore operational as electrostatic linker. Moreover, as a weak electrolyte the terminal acid maximally accounts for 1 negative charge per polymer-chain upon dissociation thus maintains a high + to - ratio required for transfection and simultaneously minimizes polymersome membrane disruption by avoiding excessive electrostatic attraction among the modified scaffold

diblock-copolymer and countercharged b-PEI. The abundant pH of 5 - 5.5 in early endosomes is close to the pKa of the acid group hence slight pH-responsiveness is imparted through dissociation of ionic cohesion in an acidic environment. A weakening of the attraction to the polycationic surface coating is thought to increase membrane disruptive and lytic effects of b-PEI to facilitate endosome disruption.

The efficiency of coating with low-Mw b-PEI(800) was markedly influenced by the procedure. While addition of 1 mol-equivalent of b-PEI to preformed PBD(1200)-b-PEO(600)-COOH vesicles only yielded a modest change in Zeta potential independent of adsorption time (1-24h), input of 10x mol-excess drastically altered the surface charge. Subsequent syringe filtration through 0.2μm PVDF units however yielded negative Zeta potentials similar to those obtained for 1eq. b-PEI (-25mV) while purification via size exclusion chromatography over Sephadex G-75 lead to charge neutralization (-2mV). This finding implies that low-Mw b-PEI polyelectrolyte might only be adsorbed in patches to the vesicle surface rather than being quantitatively associated as seen in the case of high-Mw analogues that readily invert surface charge.

Although the Zeta potentials of neutral PBD-b-PEO polymersomes and those of the PBD-b-PEO-COOH/b-PEI(800) samples were very close, confocal microscopy showed markedly improved transfection for the latter upon 24h incubation with HeLa cells (see Fig 30). This is attributed to the direct accessibility of the cationic polymer coating on the vesicle surface leading to enhanced cell surface recognition. Subcellular co-localization studies were conducted by staining HeLa for specific cell compartments with CellLight® for 20h. The stain expresses a red-fluorescent protein (RFP) tag fused to a signaling peptide, here lamp1 (lysosomal associated membrane protein 1) which provides specific targeting of cellular lysosomes and reduces spectral overlap with the polymer-label. The resulting pattern after overnight incubation with cationic polymersomes preferentially exhibits concerted fluorescence near the nuclei (red - lysosomes and green - PBD(1200)-b-PEO(600)-DEAC in Fig 30) such that localization in lysosomes can be invoked. Lysosomal localization is inferred as terminal compartment after cellular uptake via an endocytotic pathway.

Other tested alternatives for mild surface modification of the vesicles involved covalent attachment of oligoamine sequences to the hydrophilic COOH terminated block. Diethyldiethylenetriamine (DEDETA) units are often employed in context with cationic lipid transfection agents. Its conjugation to a scaffold polymer constitutes a lenient option to vesicles that do not directly expose a recognizable strongly cationic motif at the vesicle surface. The Mw of the DEDETA fragment is low thus it is not expected to trigger a significant volume transition that disintegrates the vesicle but to provide membrane disruptive potential upon charging in low pH compartments. Reaction conversion with DEDETA was 80% complete based on 1H-NMR (broad singlet at 8.21ppm). Vesicles of PBD(1200)-b-PEO(600)-DEDETA depicted a Zeta potential of -6mV as compared to -40mV for the acid terminated precursor. As in the case of superficially adsorbed b-PEI(800) the surface charge could not be inverted but reset to around zero indicating either attenuation through co-existence of residual acid moieties and/or incomplete charging because of association of the oligoamine segments with the polymer occurs. Major differences in cellular fluorescence intensity despite similar Zeta potentials among b-PEI coated and DEDETA modified vesicles upon 24h incubation with HeLa cells is indicative of that accessibility of the charged entities plays a significant role in determining the overall transfection efficiency. Similar findings on antigen presentation were reported for RGD modified polymersomes (e.g. PBD-b-PEO-RGD). DEDETA modified vesicles yielded fluorescence intensities that were comparable to slightly above those of unmodified vesicles, signaling ineffective transfection. B-PEI in contrast exhibited improved performance compared to unmodified and DEDTEA conjugated vesicles despite that all three displayed close to neutral Zeta potentials under identical conditions. Again this suggests that b-PEI is immediately amenable to cellular recognition while the covalantly bonded oligoamine segments are shielded by the hydrophilic corona or not strong enough to cause interactions with the outer leaflet of cellular membrane. The former explanation is favored considering that the conjugated groups are dissociated, which is expected for a conjugate in a well-solvated polymer such as PEG in water. This would also account for statistical positioning of the charged entities elsewhere than at the interface.

**Example 21: Lipo-polymeric vesicles**

An elegant approach that avoids the need for chemical surface modification of diblock copolymers and its purification is by blending the polymeric assembly with cationic lipids. This approach is not only thought to largely avoid neutrophil recognition by disguising the lipid antigen underneath a superficial PEG layer but also to promote vesicle fusion in a pH independent way as quaternary ammonium groups are endowed. The admixture of lipids further allows for tuning of the PEG density, hence for regulating recognition of the lipid antigen at the cell surface and eases endosomal escape through hydrolytic degradation of lipids leading to altered lysis characteristics. Here we chose 1,2-dioleoyl-sn-glycero-3-ethylphosphocholine chloride salt ($DOPC^+$) to achieve a homogeneous lipopolymersome blend. The incorporation of doubly unsaturated lipids was previously shown to give a uniform lipid distribution within PBD-b-PEO vesicles of moderate Mw. This homogeneity is further improved in our case by additional dispersion through charge repulsion among the cationic lipids. The measured Zeta potential of the blended sample was rendered overall cationic (+16mV) in contrast to the above discussed modifications. Interestingly the polymer blend was considerable less cationic than

for respective DOPC$^+$ blended POPC liposome preparations (+50mV). This strongly suggests that the cationic lipids are likely to be located close to the hydrophilic-hydrophobic interface of the block-copolymers and are thus shielded by a hydrated, neutral polymer layer while the charge is directly exposed at the interface for liposomes.

Cell uptake studies of cationic lipopolymersomes lead to highest transfection efficiencies observed as seen in Fig.2. Similarly the subcellular localization of cationic lipopolymersomes paralleled that of b-PEI coated and DEDETA modified vesicles and showed containment within lysosomes (see Fig 31). We speculate that the improved efficiency of hybrid vesicles might be due to several rationales such as net cationic charge, improved antigen presentation and/or raised fusogenic potential of mixed-amphiphile vesciles. Net charge is undoubtedly a crucial factor that significantly accelerates uptake. Improved lipid antigen presentation by blending was advocated in context with accessibility of the cationic lipid by thinning the PEG density. Higher exchange rates for lipids are inherent due to their lower Mw, respectively lower hydrophobic adhesion than among polymers and therefore higher effective solution concentration (critical micelle concentration). The latter might account for increased fusion tendency of lipopolymersomes with the native lipids of the cell membrane while cationic polymer conjugates are relatively immobile and non-fusiogenic.

**Example 22: Magneto(lipo)polymersomes**

Monodisperse, irreversibly grafted superparamagnetic iron oxide nanoparticles (SPIONs) of maximal ligand density were prepared as above. Figure 29 shows TEM micrographs of the synthesized SPIONs with a sharp size distribution of 5 $\pm$ 0.4 nm. Successful high density membrane embedding of hydrophobic SPIONs by solvent inversion is demonstrated by ultra-thin sectioning of the loaded polymersomes in Fig. 29 exhibiting nanoparticle localizations exclusively in the bilayer region.

The obtained size distribution and overall lamellarity of the prepared sample however varies with aqueous phase composition and polymer concentration. At low amphiphile concentrations (<1mg/ml) the formed polymersomes were predominately unilamellar while higher concentrated samples (>1mg/ml) gave some mulitlamellar vesicles.

**Example 23: Cell uptake of polymeric and lipopolymeric vesicles**

Overnight incubation of human cervical adenocarcinoma cells (HeLa line) with differently prepared DEAC-labeled polymersomes gave rise to only a minor fluorescence signal within the cells in confocal microscopy. This indicates that unmodified PBD-b-PEO vesicles exhibit slow cellular uptake kinetics and significant stealth properties which is in line with earlier reports on uptake without irradiation.

Similar uptake behavior was observed among loaded vesicles prepared by solvent inversion or by rehydration plus extrusion despite different weight fractions of SPIONs incorporated in the membrane. This indicates that even at elevated SPION content, the non-fouling and stealth properties of the polymersomes are not compromised. In other words, either the adsorption of proteins to the polymersomes is not increased, that is, membrane embedded SPIONs do not act as sites for non-specific adsorption, or additionally adsorbed proteins remain well shielded by the PEO blocks. The former is the more plausible explanation and supported by that no associated proteins could be detected electrophoretically after incubation in cell culture media.

TEM confirmed modest uptake of unmodified mangetopolymersomes with a neutral, non-zwitterionic outermost PEG corona. Polymeric vesicles are easily identified in TEM by positive staining of the unsaturated PBD-block with OsO4. A rare event is shown in Fig. 31 which depicts an internalized, multilamellar SPION-loaded polymersome after 24h of incubation. The ingested SPION-loaded polymersomes were structurally intact and vesicle integrity was retained without any visual signs of decomposition denoting that lytic enzymes within the endosome or lysosome do not readily recognize the ingested vesicles. Cellular ultrastructure was highly conserved (pool of around 100 samples). HeLa cells embedded after 24h of incubation with surface modified PBD(1200)-b-PEO(600) on the contrary show clearly enhanced uptake.

Investigations on changes in the cellular ultrastructure after uptake of the supramolecular blend of diblock-copolymer with 30%n/n of the cationic lipid 1,2-dioleoyl-sn-glycero-3-ethylphosphocholine chloride salt (DOPC$^+$) in the hybrid vesicle exhibited not only high uptake efficiency but also an increased amount of apoptotic cells. Ingested lipopolymersomes were of reduced lamellarity and resulted in more flexible structures than compared to plain polymersomes. A change in the elastic modulus of these hybrid systems was suggested previously and is supported by our findings. A high amount of intactvesicles in healthy cells is shown in Fig 34a. Features of cellular stress are absent for these samples despite the high amount of ingested vesicles. Cellular apoptosis is testified by a sudden occurrence of membrane blebs, cytoplasm condensation, organelle packaging, extended vacuolation and nuclear pyknosis. We often observed dispersed high contrast areas attributed to iron-polymer complexes within vacuoles of apoptotic cells (see Fig 34b) indicating that lipopolymersomes are also degraded over time. Characteristics indicative of necrosis such as loss of membrane integrity and nuclear fragmentation were however only rarely observed. It is suggested in literature that cationic liposomes activate several cellular pathways like pro-apoptotic and pro-inflammatory cascades. In this view it seems plausible that an

increased fusogenic potential of lipopolymersomes facilitates transfer of cationic lipids leading to elevated apoptosis.

In contrast to polymersomes, hybrid lipopolymersomes were readily rendered cationic by incorporation of lipids and raised the intracellular iron content significantly. Co-localization studies showed that all surface-modified vesicles were preferentially located in lysosomes which is consistent with an endosomal uptake pathway. For cationic vesicles cellular ultrastructure showed an increased frequency of apoptotic features while neutral vesicles did not induce a conspicuous cellular stress response.

## Claims

1. A method of preparing a vesicular particle having at least in part a lipid and/or polymeric membrane that is a barrier between the interior and exterior of said vesicular particle, wherein said membrane comprises at least one magnetic nanoparticle embedded in said membrane, said method comprises the steps of

    i) providing a first dispersion with one or more inorganic core particles having a hydrophobic dispersant shell in a solution of membrane forming lipids and/or polymers in a non-aqueous solvent; and
    ii) introducing the first dispersion into a fluid that is a non-solvent for the membrane forming lipids and/or polymers, wherein the volume of the non-solvent exceeds the volume of the first dispersion and the non-aqueous solvent and the non-solvent are miscible, thereby forming the vesicular particles.

2. The method of claim 1, wherein said non-aqueous solvent comprises tetrahydrofuran.

3. The method of claim 1 or 2, wherein the introducing step ii) is turbulent, preferably by stirring, shaking or sonication of the non-solvent or by injection or dripping of the non-aqueous solvent into the non-solvent, and/or wherein the introducing step ii) is under agitation so that vesicles with an average diameter of 20 nm to 400 nm form, preferably vesicles with an average diameter of 30 nm to 200 nm, especially preferred 35 nm to 100 nm, form.

4. The method of any one of claims 1 to 3, wherein in step ii) the introduced volume of the non-aqueous solvent is less than half of the volume of the non-solvent.

5. The method of any one of claims 1 to 4, wherein the inorganic core particles are of an average size between 1 nm to 15 nm in diameter.

6. The method of any one of claims 1 to 5, wherein step i) is providing a first dispersion with one or more inorganic core particles having a hydrophobic dispersant shell in a solution of membrane forming lipids in a non-aqueous solvent, preferably wherein the lipids comprise a fatty acid ester group selected from palmitoyl-, lauryl-, myristoyl-, oleoyl-, stearoyl- groups and/or wherein at least one of the lipids has a melting transition above 38° C.

7. The method of any one of claims 1 to 6, comprising the steps of

    i) providing a first dispersion with one or more inorganic core particles having a hydrophobic dispersant shell and an inorganic paramagnetic or superparamagnetic core of between 1 to 15 nm in diameter, in a solution of membrane forming lipids in tetrahydrofuran; and
    ii) mixing the first dispersion into an aqueous fluid under rapid conditions and/or with agitation, thereby forming the vesicular particles.

8. The method of any one of claims 1 to 7, wherein the inorganic core particles comprise dispersant molecules bound to the particle surface, that

    (a) are at an average density of at least 1.1, preferably at least 3.0, dispersant molecules per $nm^2$ of the inorganic core surface, and/or
    (b) form a shell of constant dispersant density and a further shell of gradually reduced dispersant density with increasing distance from the inorganic core surface.

9. The method of any one of claims 1 to 8, further comprising sonicating the vesicular particles of step ii).

10. The method of any one of claims 1 to 9, comprising adding an amphiphilic polymer to the solution of step i) or to the forming vesicular particles of step ii).

**11.** The method of claim 10, wherein said amphiphilic polymer comprises a hydrophilic block of 20-60%v/v.

**12.** A composition of a plurality of vesicular particles each having at least in part a lipid and/or polymeric membrane that is a barrier between the interior and exterior of said vesicular particle, wherein said membrane comprises inorganic core nanoparticles embedded in said membrane, **characterized in that**

A) said embedded nanoparticles are in a concentration of at least 0.5 % (w/w per lipid and/or polymer), and wherein said concentration is constant or decreases by less than 25 % (percentage of w/w concentration) at least during 24 hours at standard conditions in an aqueous dispersion with physiological buffer; and/or
B) said vesicular particles are formed by a method of any one of claims 1 to 11.

**13.** The composition of claim 12, wherein the inorganic core nanoparticles comprises a magnetic core, preferably a superparamagnetic core of between 1 to 15 nm in diameter, and a hydrophobic dispersant shell.

**14.** The composition of claim 12 or 13, wherein a pharmaceutical agent is contained in the lumen or in the membrane of the vesicular particles.

**15.** Use of the composition of any one of claims 12 to 14 for administration to a subject or to a cell or tissue culture, preferably wherein the composition is administered to a subject and said subject is irradiated so that the inorganic core nanoparticles are excited and/or heated.

Fig. 1

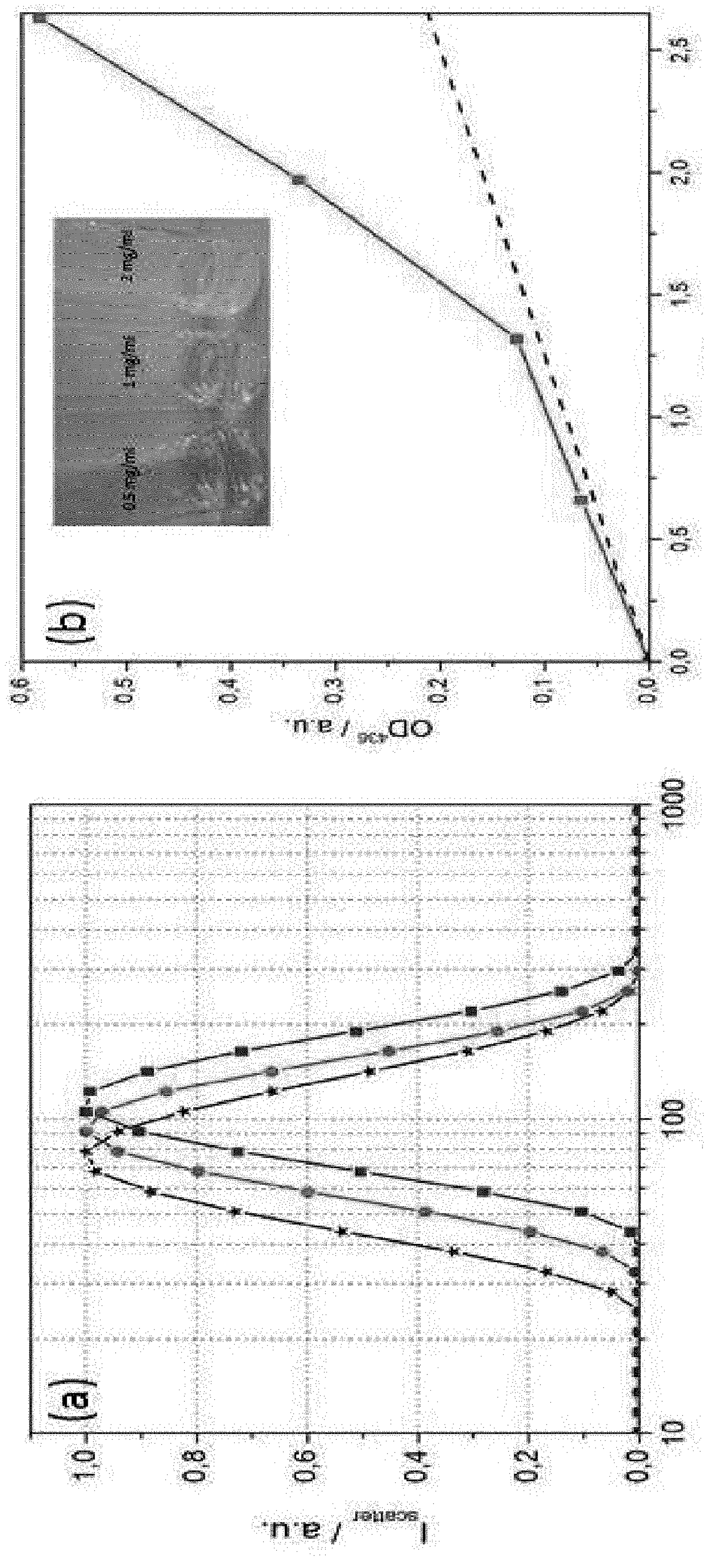

Fig. 2

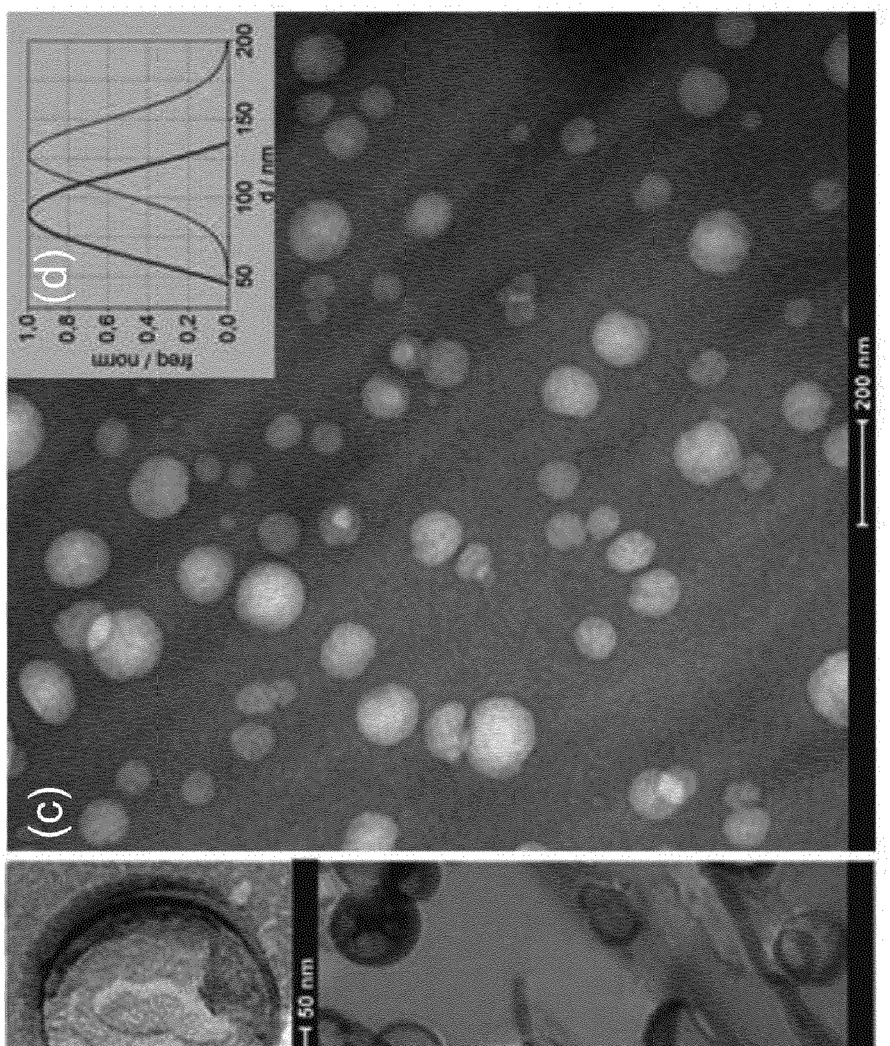
(a)
(b)
50 nm
200 nm
(c)
200 nm
(d)
freq / norm
1.0
0.8
0.6
0.4
0.2
0.0
50
100
150
200
d / nm

Fig. 3

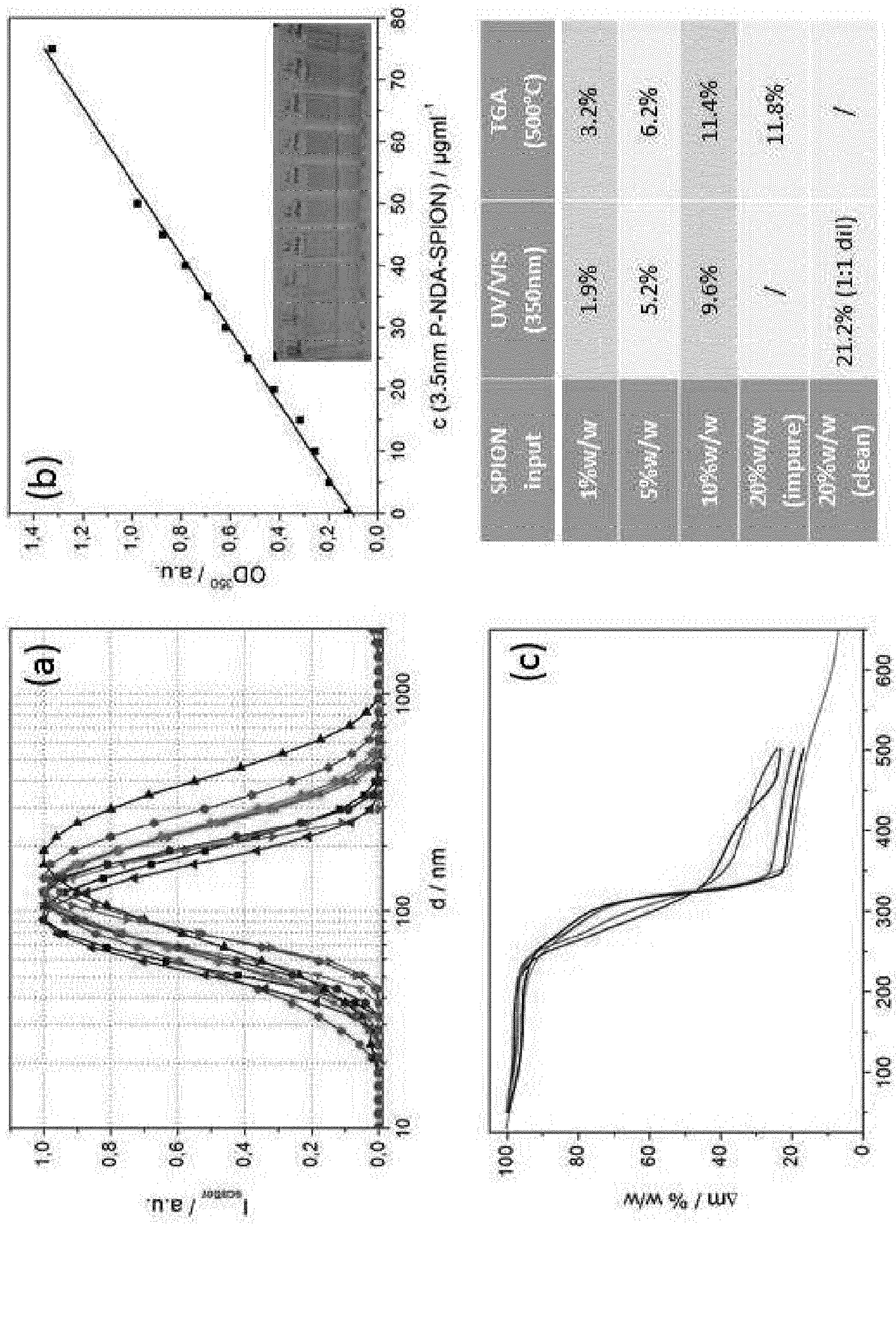


| SPION input | UV/VIS (350nm) | TGA (500°C) |
|---|---|---|
| 1%w/w | 1.9% | 3.2% |
| 5%w/w | 5.2% | 6.2% |
| 10%w/w | 9.6% | 11.4% |
| 20%w/w (impure) | / | 11.8% |
| 20%w/w (clean) | 21.2% (1:1 dil) | / |

Fig. 4

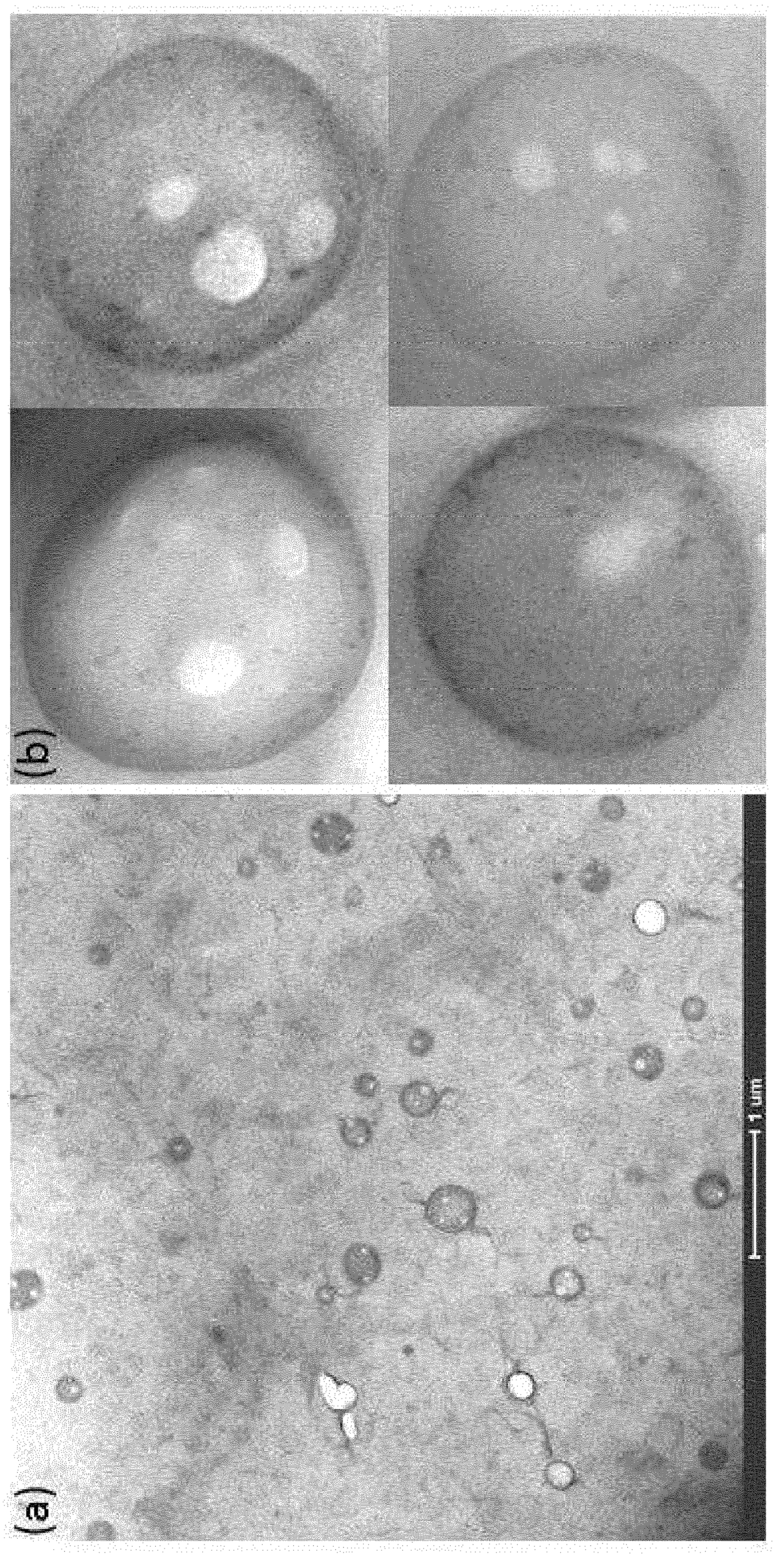

Fig. 5

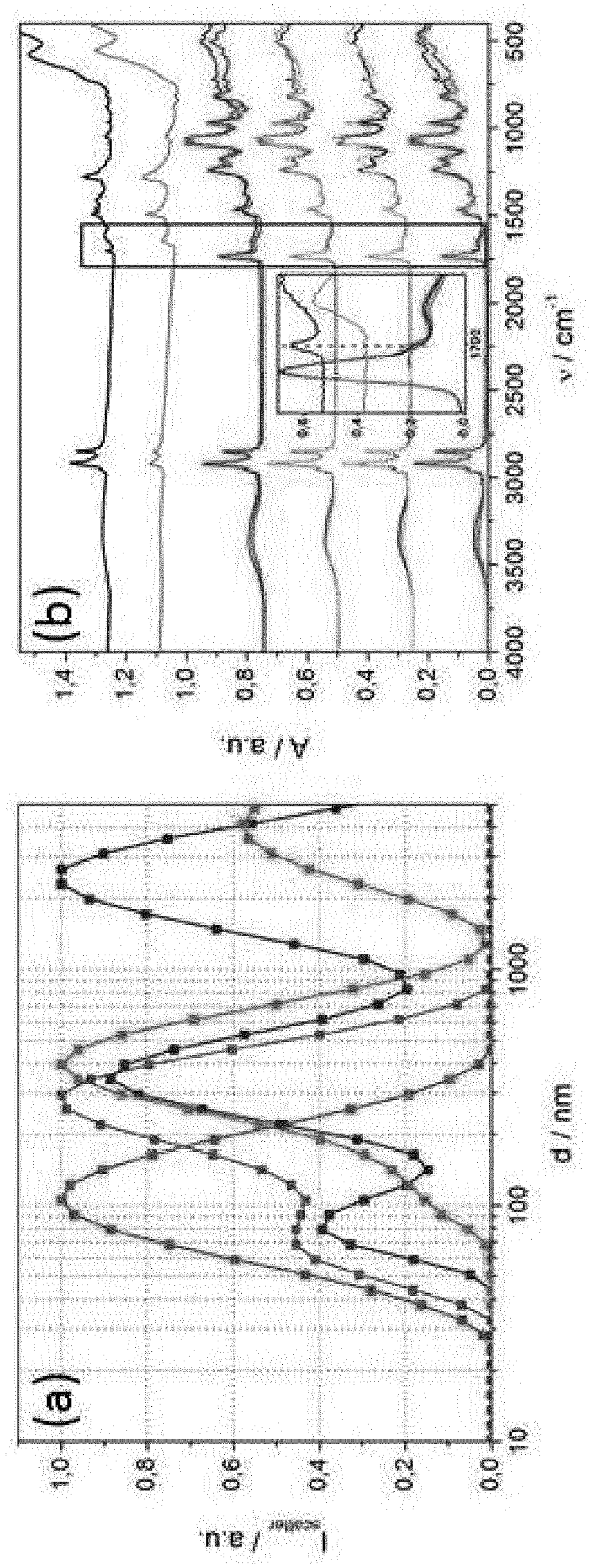

Fig. 6

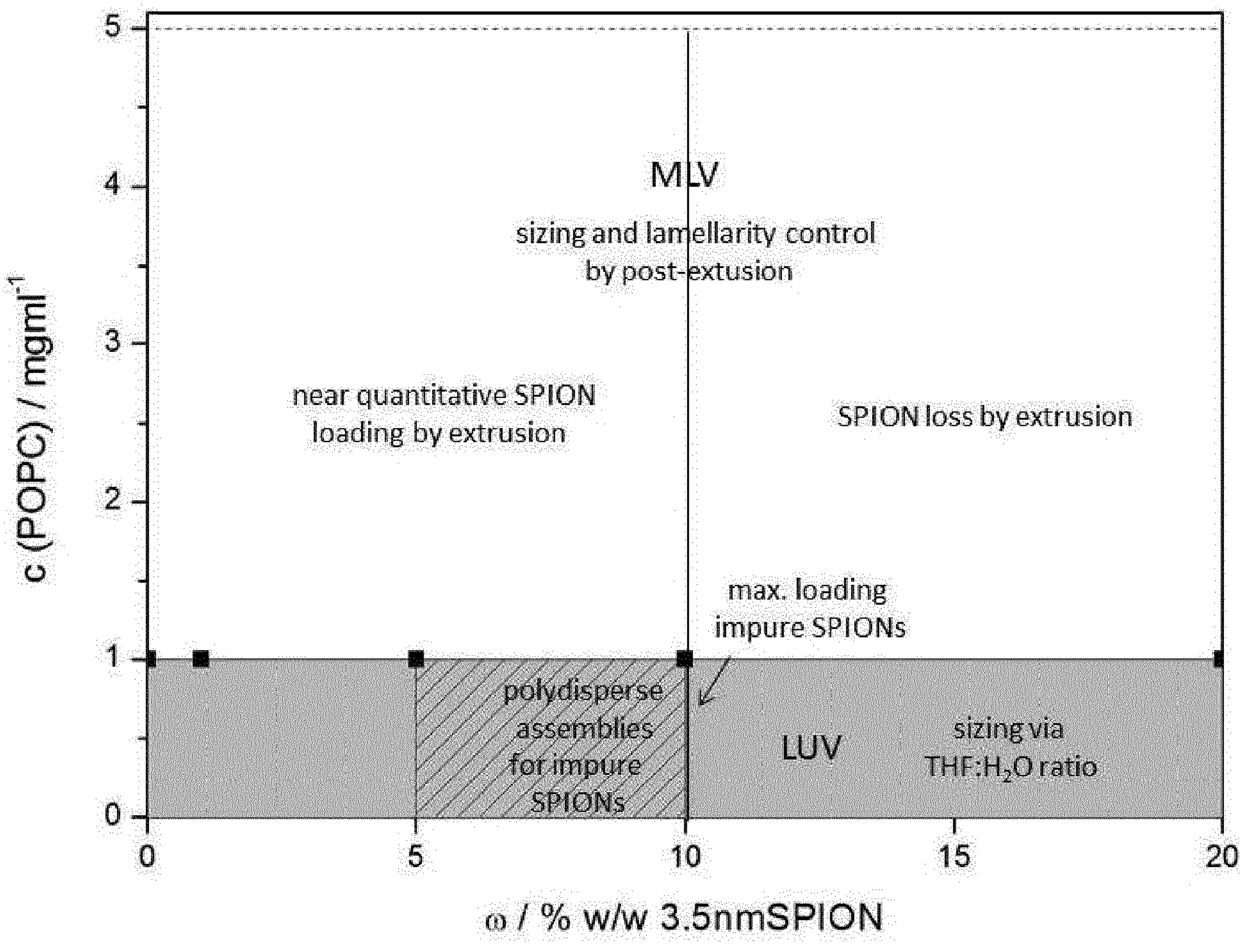

5
4
3
2
1
0
c (POPC) / mgml-1
MLV
sizing and lamellarity control
by post-extusion
near quantitative SPION
loading by extrusion
SPION loss by extrusion
max. loading
impure SPIONs
polydisperse
assemblies
for impure
SPIONs
LUV
sizing via
THF:H2O ratio
0
5
10
15
20
ω / % w/w 3.5nmSPION

Fig. 7

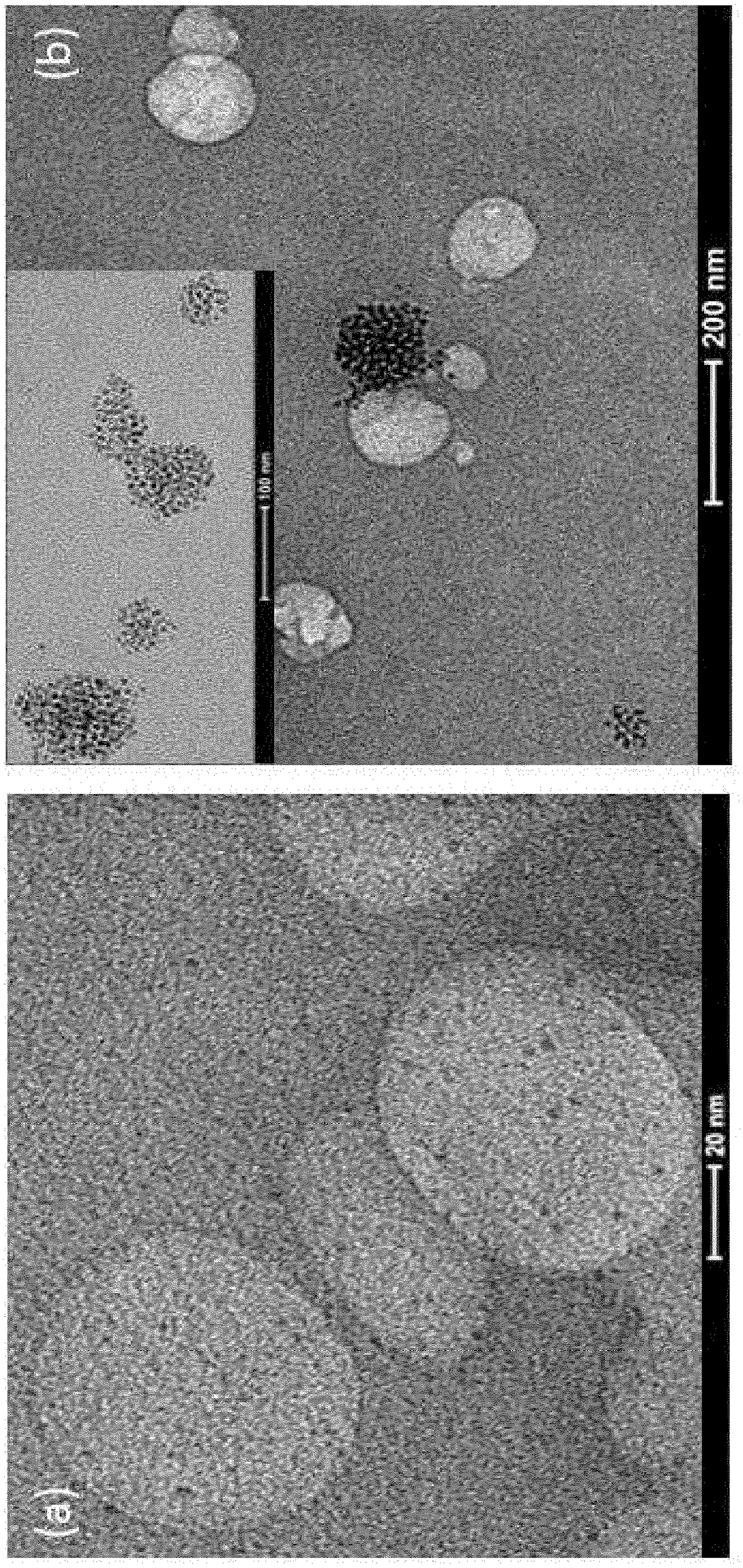

Fig. 8

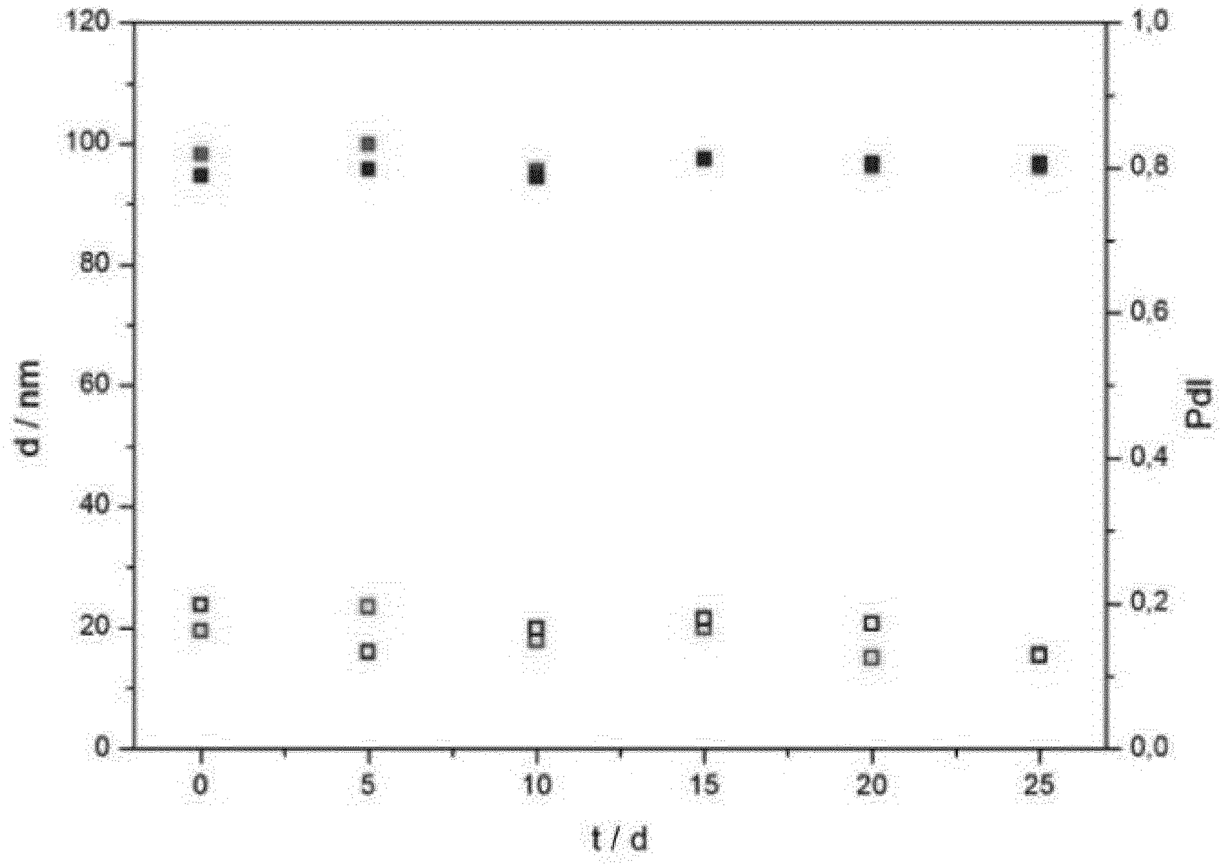

Fig. 9a

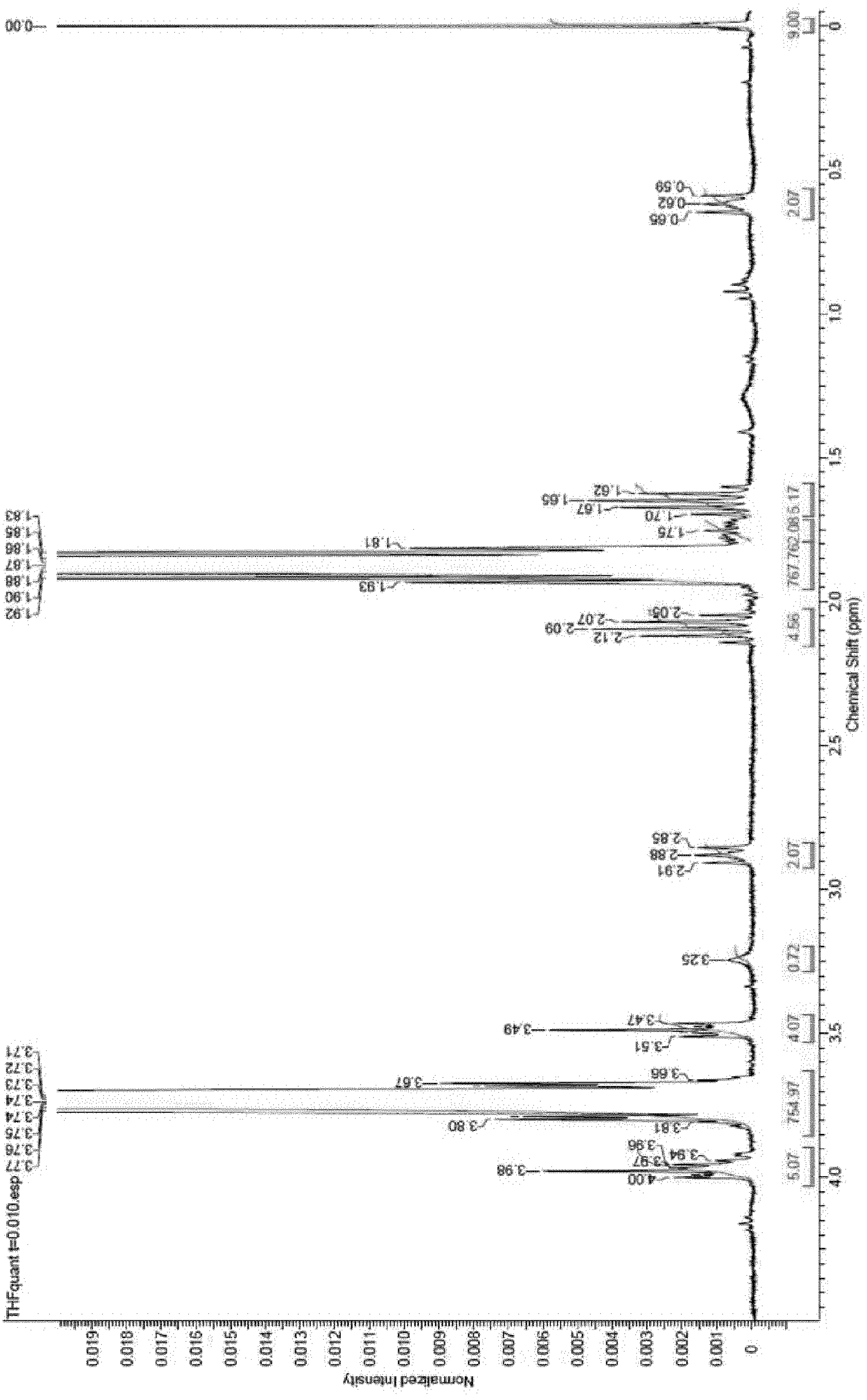
THFquant t=0.010.esp
Chemical Shift (ppm)
Normalized Intensity

Fig. 9b

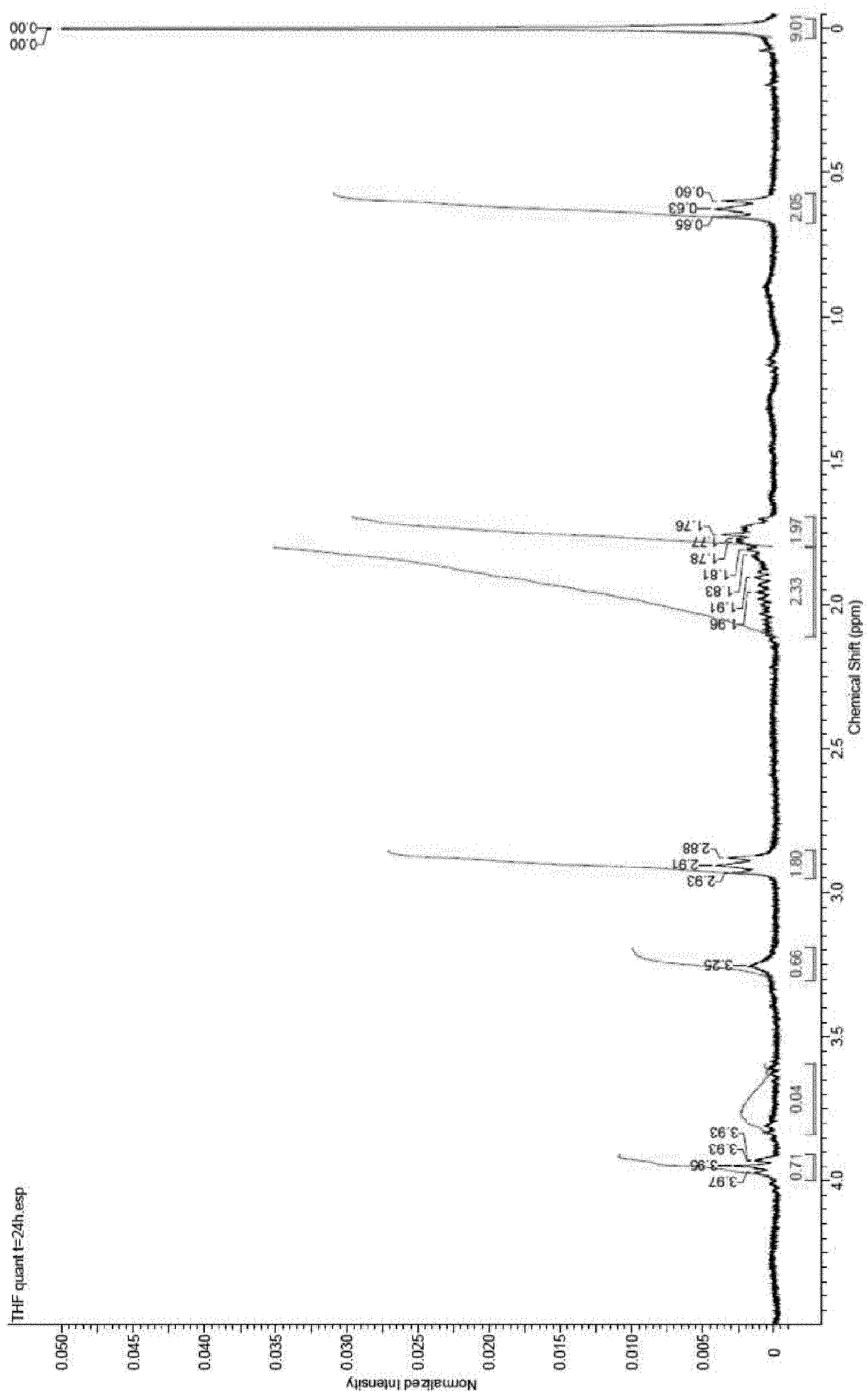
THF quant t=24h.esp
Normalized Intensity
Chemical Shift (ppm)

Fig. 10

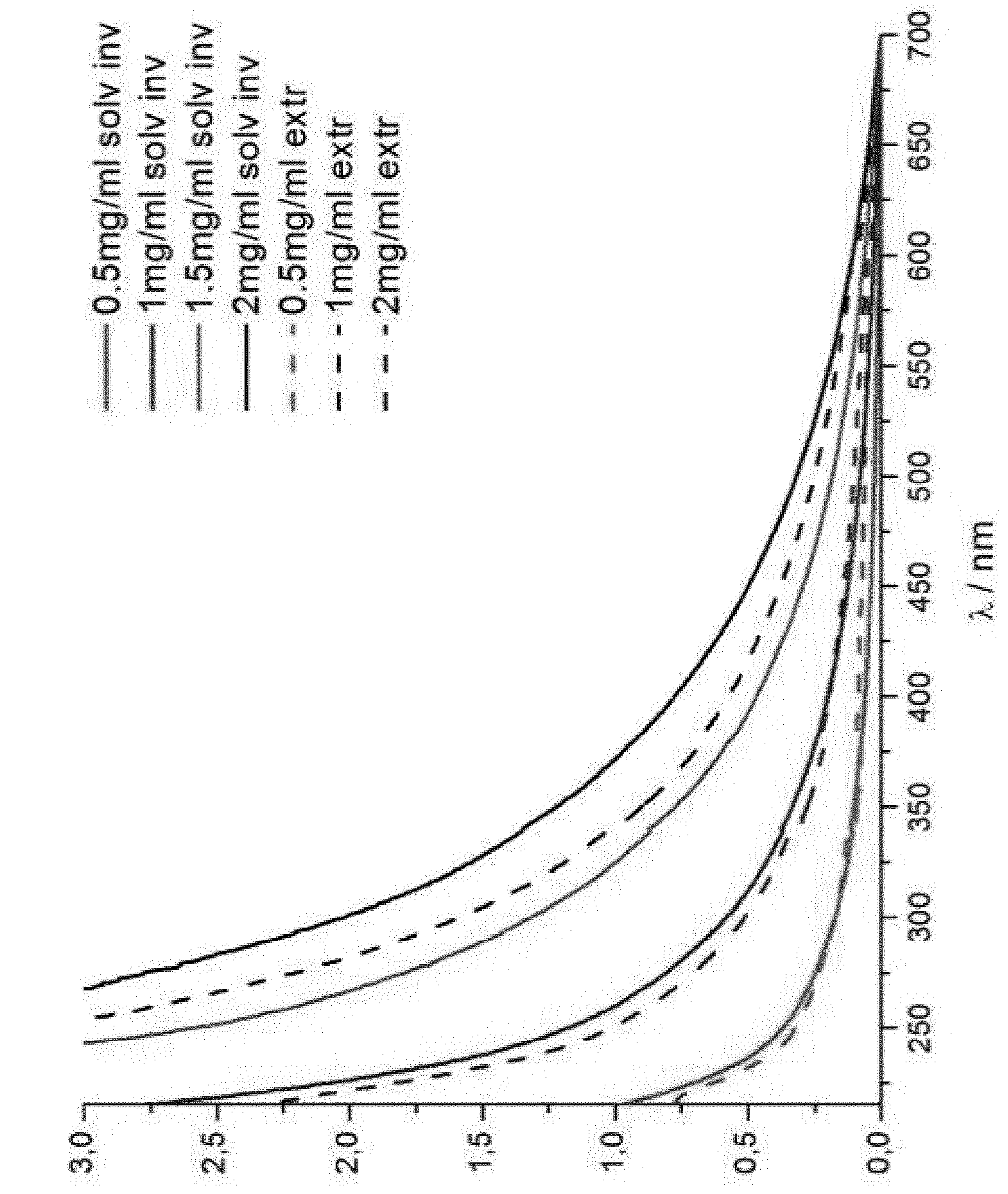
0.5mg/ml solv inv
1mg/ml solv inv
1.5mg/ml solv inv
2mg/ml solv inv
0.5mg/ml extr
1mg/ml extr
2mg/ml extr
O.D. / a.u.
λ / nm
3,0
2,5
2,0
1,5
1,0
0,5
0,0
250
300
350
400
450
500
550
600
650
700

Fig. 11

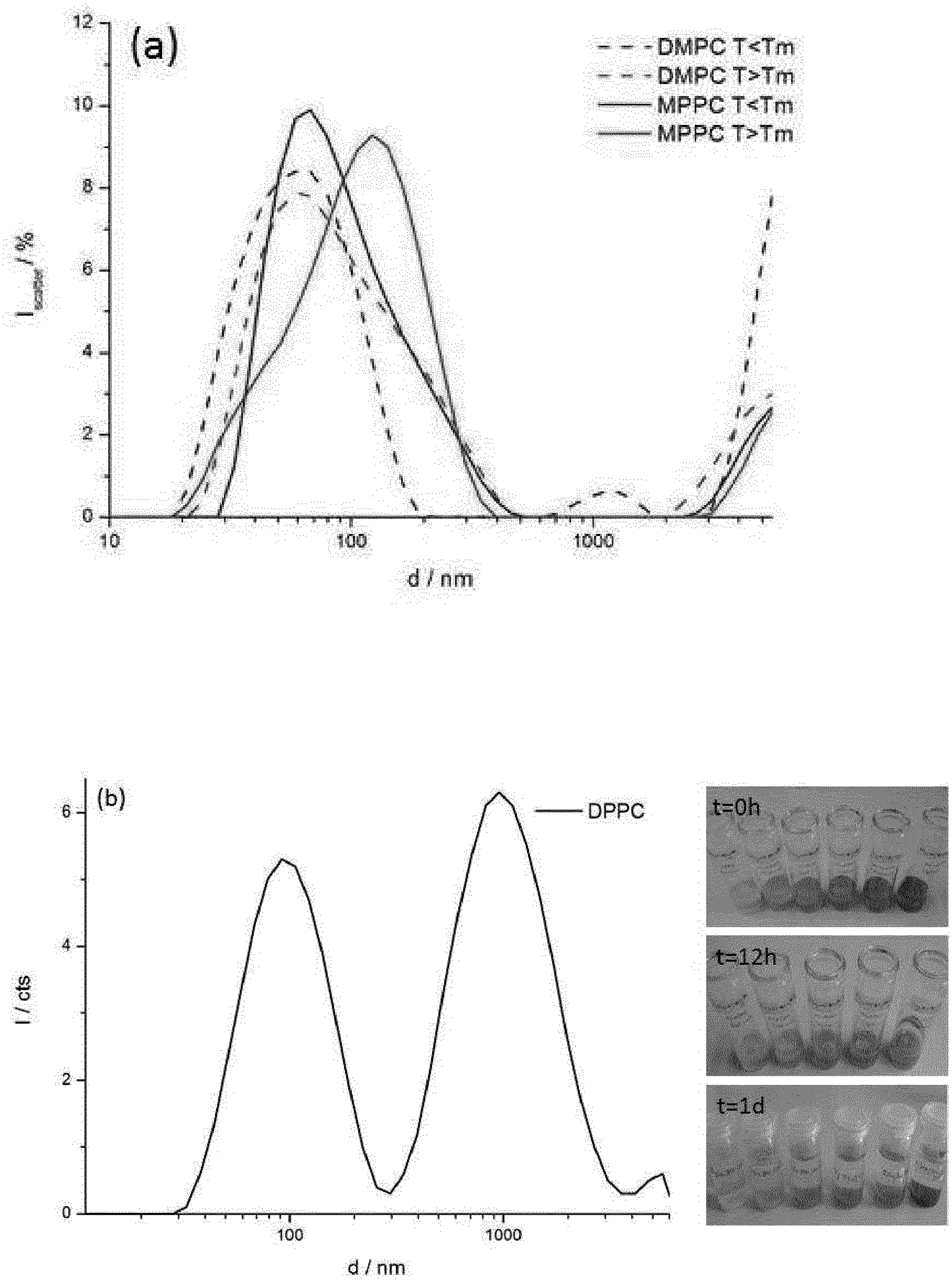
(a)
DMPC T<Tm
DMPC T>Tm
MPPC T<Tm
MPPC T>Tm
I_scatter / %
d / nm
(b)
DPPC
I / cts
d / nm
t=0h
t=12h
t=1d

Fig. 12

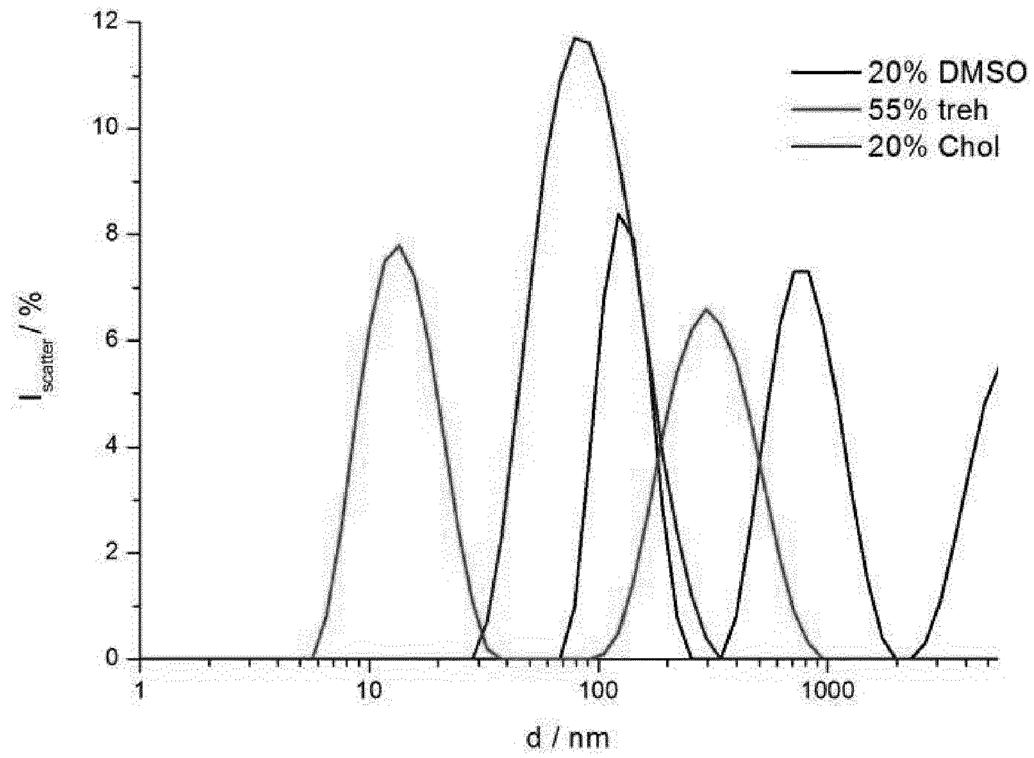

Fig. 13

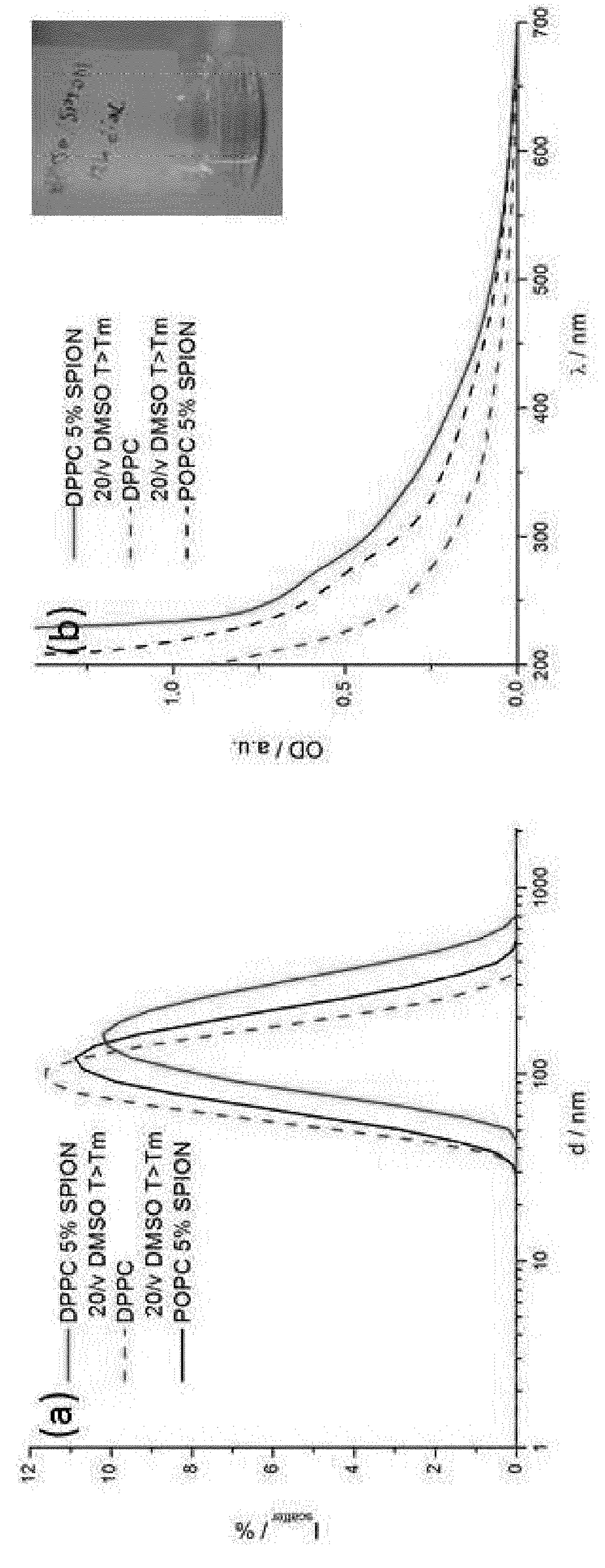
(a)
DPPC 5% SPION
20/v DMSO T>Tm
DPPC
20/v DMSO T>Tm
POPC 5% SPION
d / nm
I scatter / %
(b)
DPPC 5% SPION
20/v DMSO T>Tm
DPPC
20/v DMSO T>Tm
POPC 5% SPION
λ / nm
OD / a.u.

Fig. 14

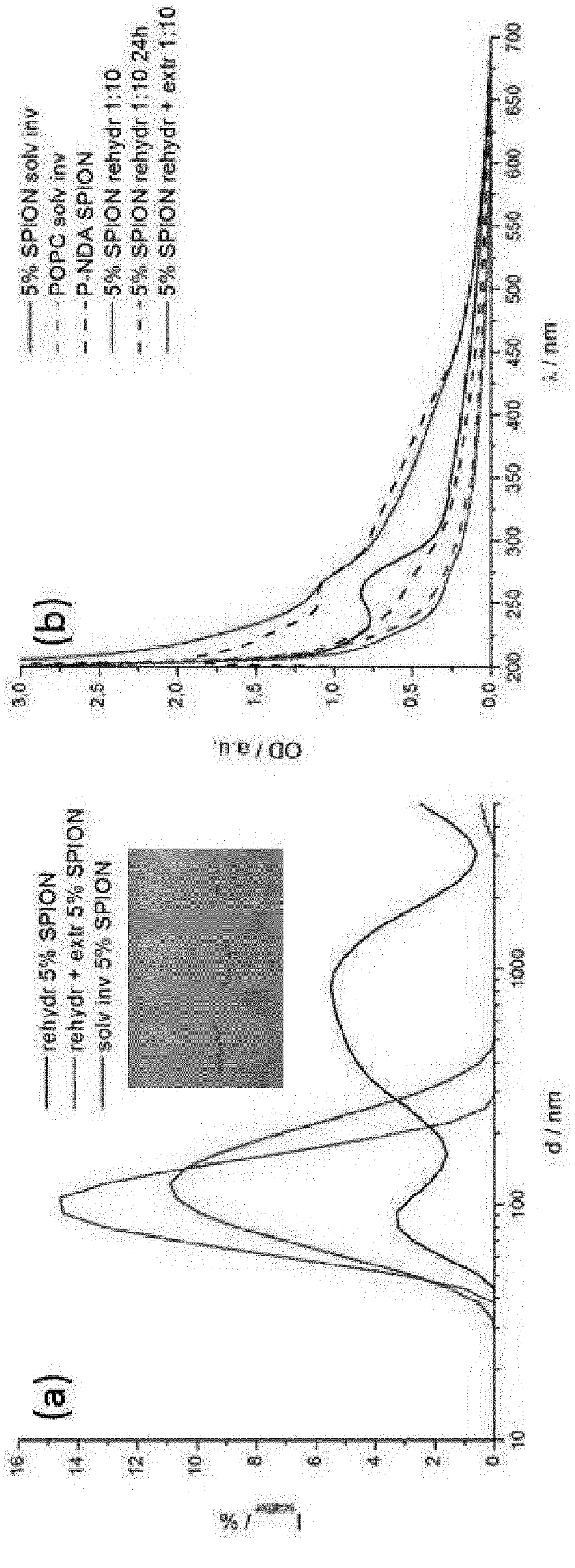
(a)
rehydr 5% SPION
rehydr + extr 5% SPION
solv inv 5% SPION
$I_{scatter}$ / %
d / nm
(b)
5% SPION solv inv
POPC solv inv
P-NDA SPION
5% SPION rehydr 1:10
5% SPION rehydr 1:10 24h
5% SPION rehydr + extr 1:10
OD / a.u.
λ / nm

Fig. 15

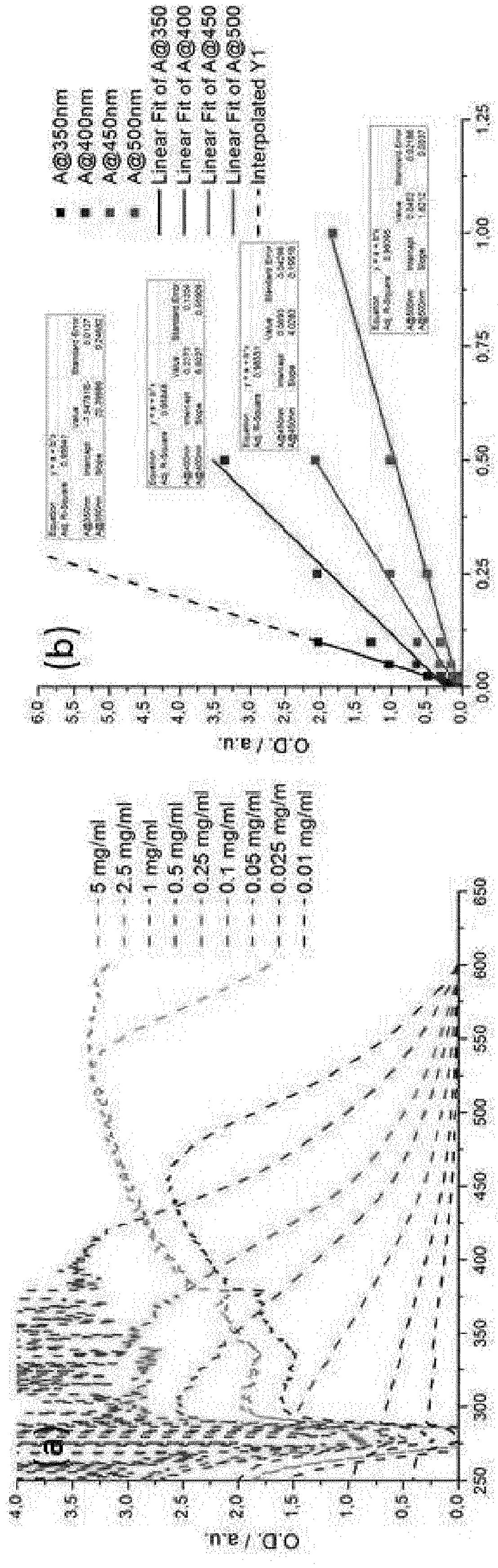
(a)
O.D. / a.u.
λ / nm
5 mg/ml
2.5 mg/ml
1 mg/ml
0.5 mg/ml
0.25 mg/ml
0.1 mg/ml
0.05 mg/ml
0.025 mg/m
0.01 mg/ml
(b)
O.D. / a.u.
c(P-NDA-NP) / mgml[-1]
A@350nm
A@400nm
A@450nm
A@500nm
Linear Fit of A@350
Linear Fit of A@400
Linear Fit of A@450
Linear Fit of A@500
Interpolated Y1

Fig. 16

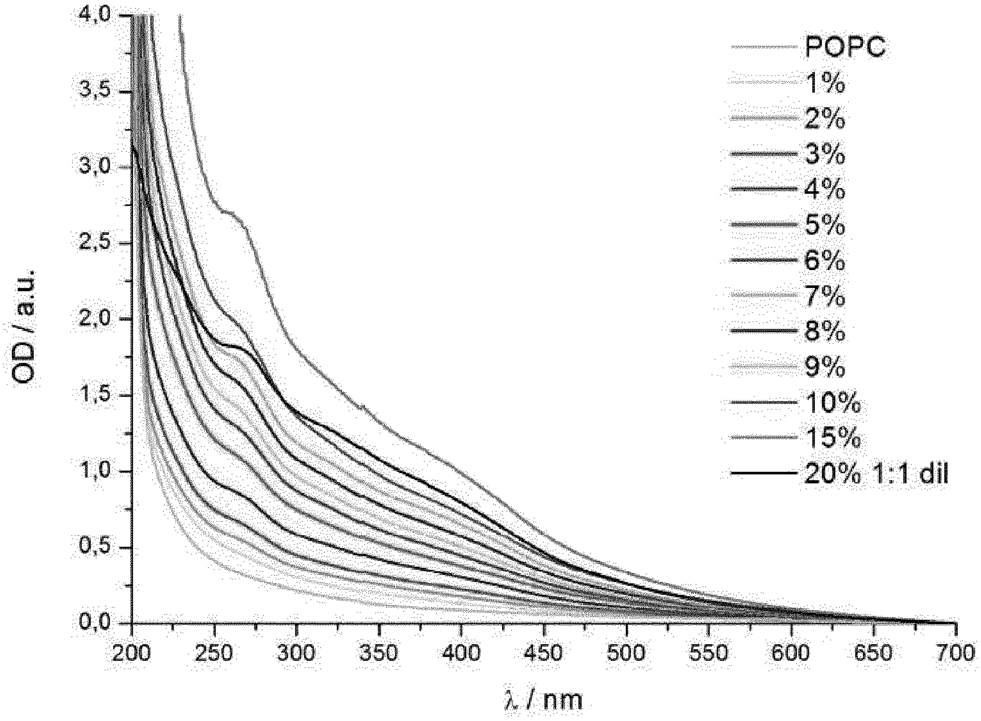
POPC
1%
2%
3%
4%
5%
6%
7%
8%
9%
10%
15%
20% 1:1 dil
OD / a.u.
λ / nm
4,0
3,5
3,0
2,5
2,0
1,5
1,0
0,5
0,0
200
250
300
350
400
450
500
550
600
650
700

Fig. 17

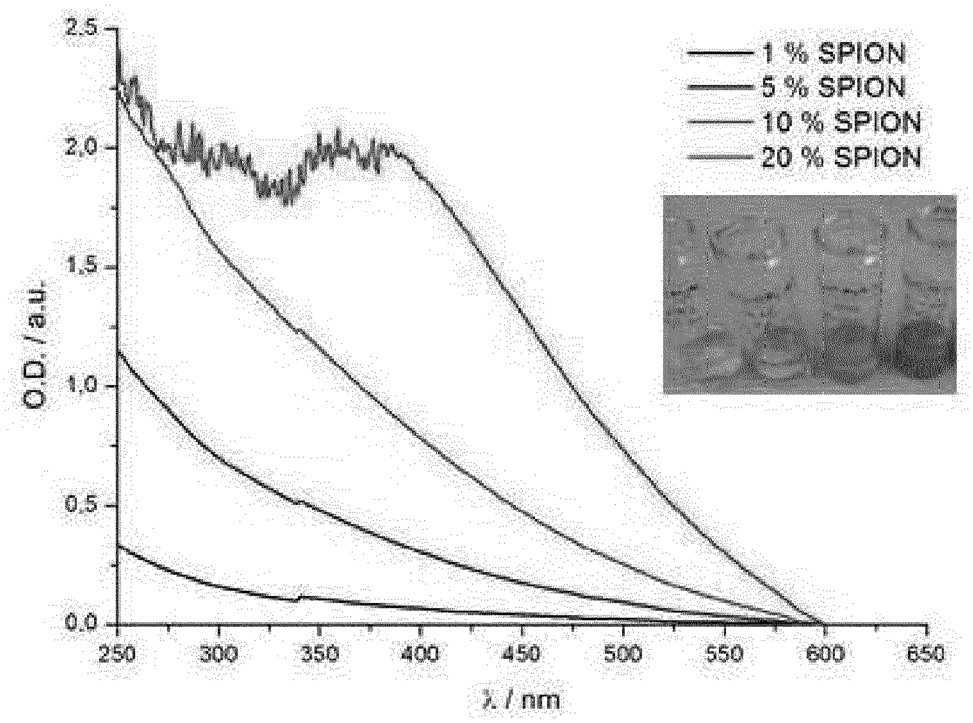
1 % SPION
5 % SPION
10 % SPION
20 % SPION
O.D. / a.u.
λ / nm
2,5
2,0
1,5
1,0
0,5
0,0
250
300
350
400
450
500
550
600
650

Fig. 18

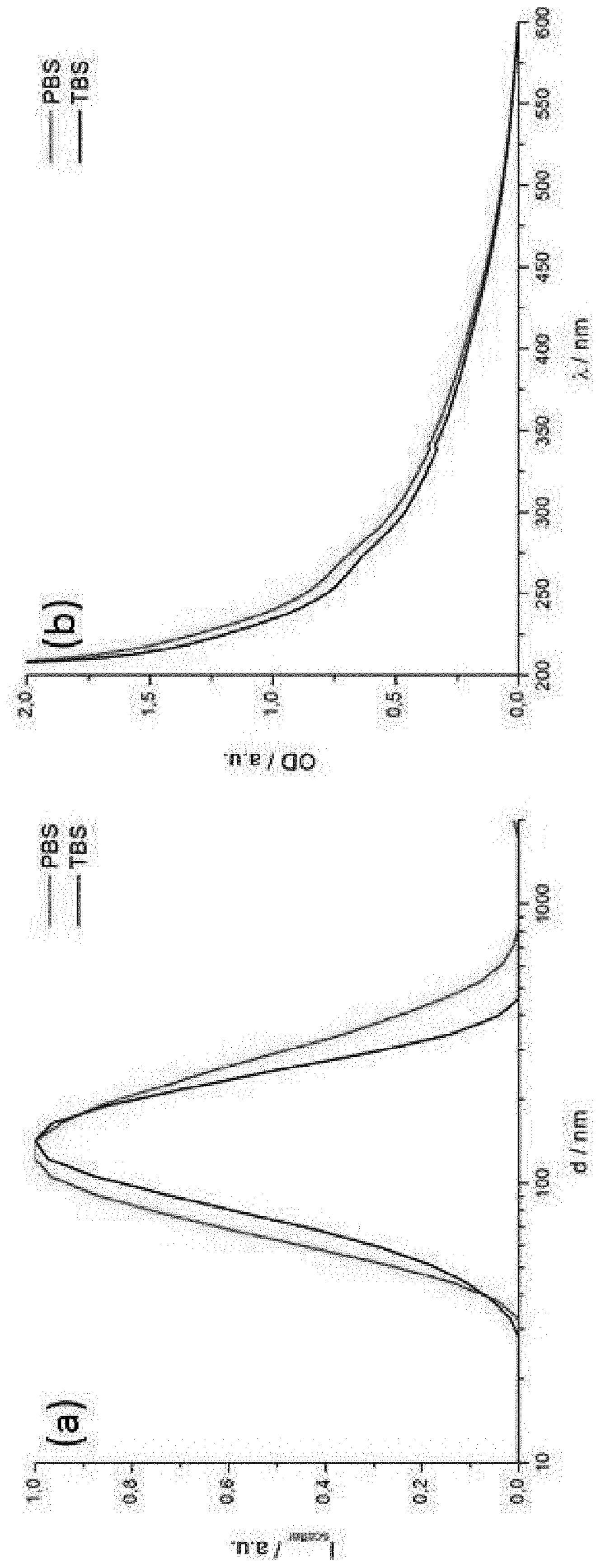

Fig. 19

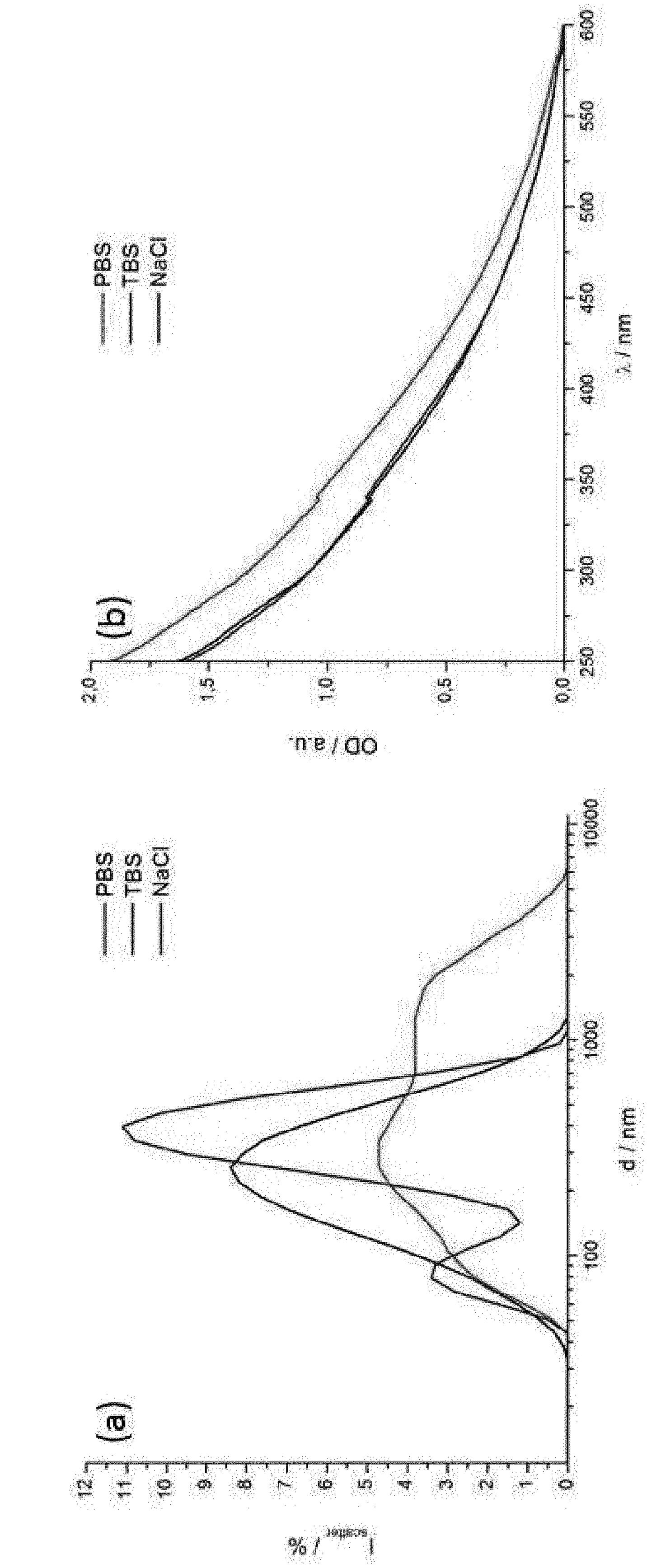
(a)
PBS
TBS
NaCl
I scatter / %
0 1 2 3 4 5 6 7 8 9 10 11 12
100
1000
10000
d / nm
(b)
PBS
TBS
NaCl
OD / a.u.
0,0 0,5 1,0 1,5 2,0
250 300 350 400 450 500 550 600
λ / nm

Fig. 20

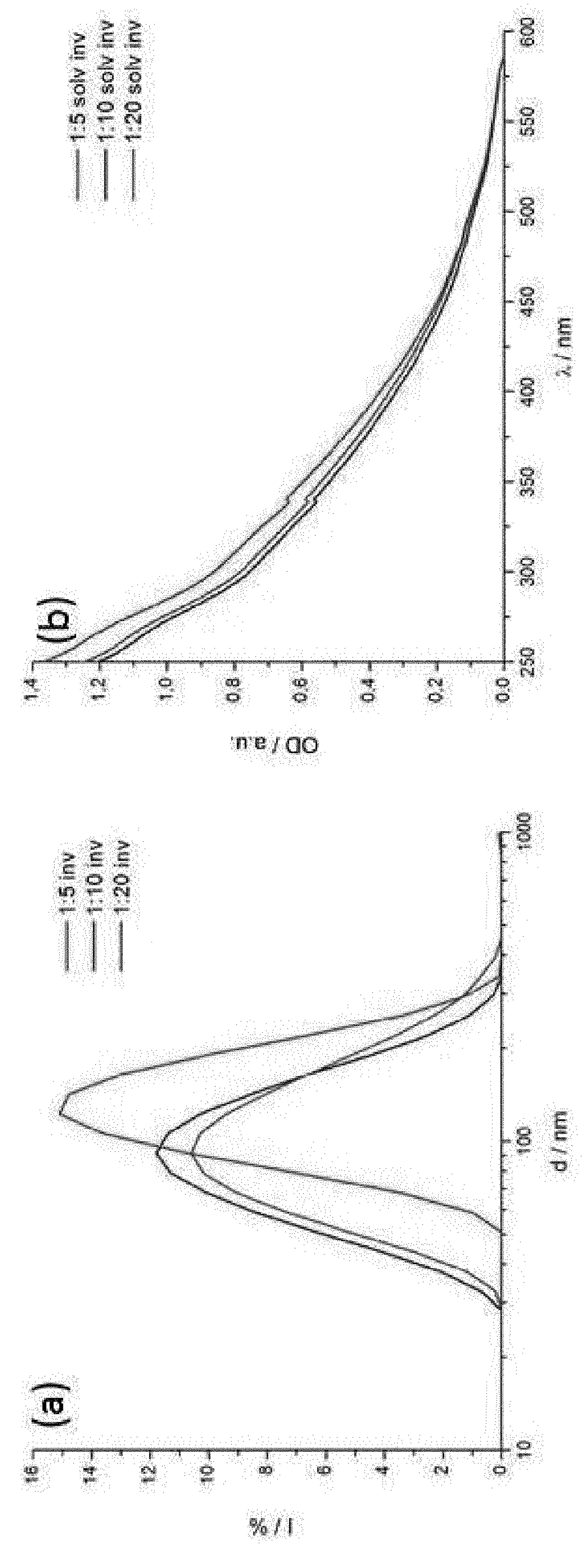
(a)
1:5 inv
1:10 inv
1:20 inv
I / %
d / nm
(b)
1:5 solv inv
1:10 solv inv
1:20 solv inv
OD / a.u.
λ / nm

Fig. 21

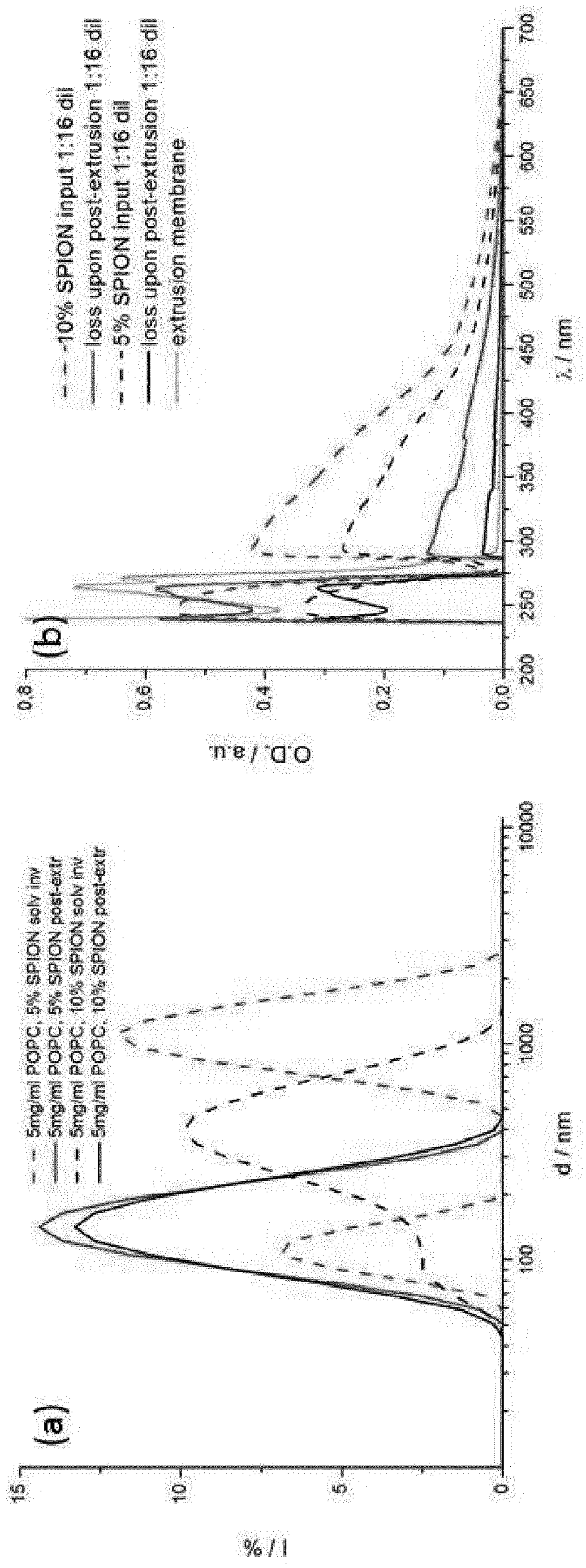
(a)
5mg/ml POPC, 5% SPION solv inv
5mg/ml POPC, 5% SPION post-extr
5mg/ml POPC, 10% SPION solv inv
5mg/ml POPC, 10% SPION post-extr
I / %
d / nm
(b)
10% SPION input 1:16 dil
loss upon post-extrusion 1:16 dil
5% SPION input 1:16 dil
loss upon post-extrusion 1:16 dil
extrusion membrane
O.D. / a.u.
λ / nm

Fig. 22

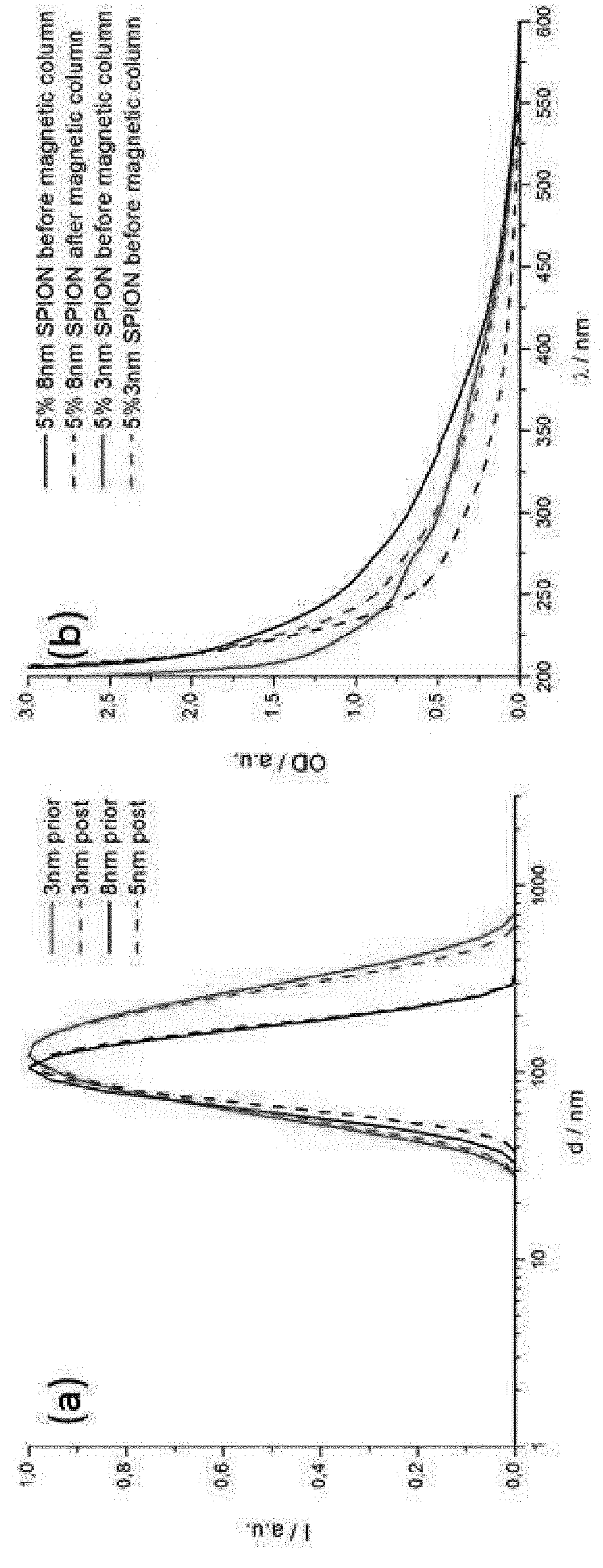
(a)
3nm prior
3nm post
8nm prior
5nm post
I / a.u.
d / nm
(b)
5% 8nm SPION before magnetic column
5% 8nm SPION after magnetic column
5% 3nm SPION before magnetic column
5%3nm SPION before magnetic column
OD / a.u.
λ / nm

Fig. 23

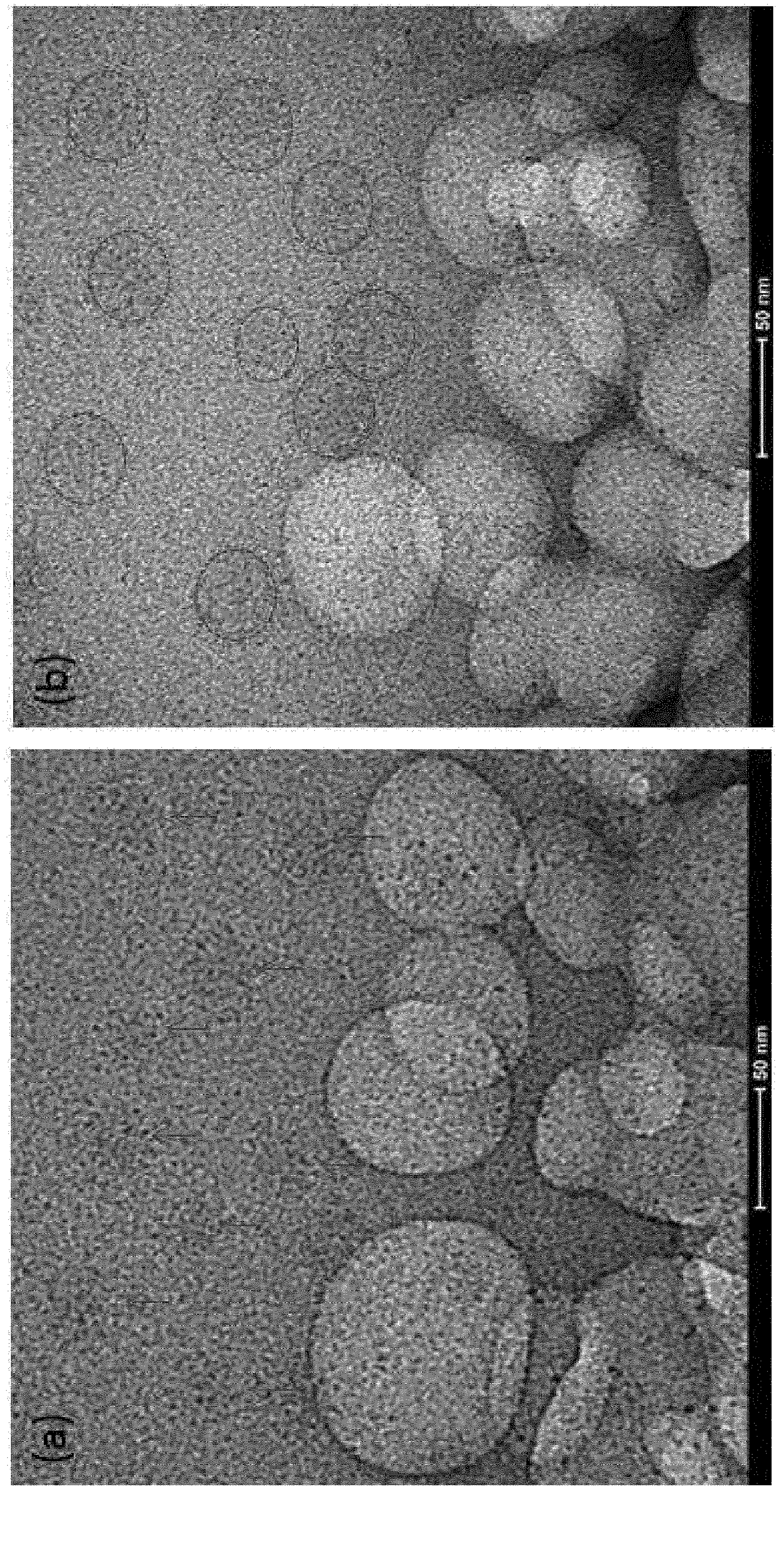

Fig. 24

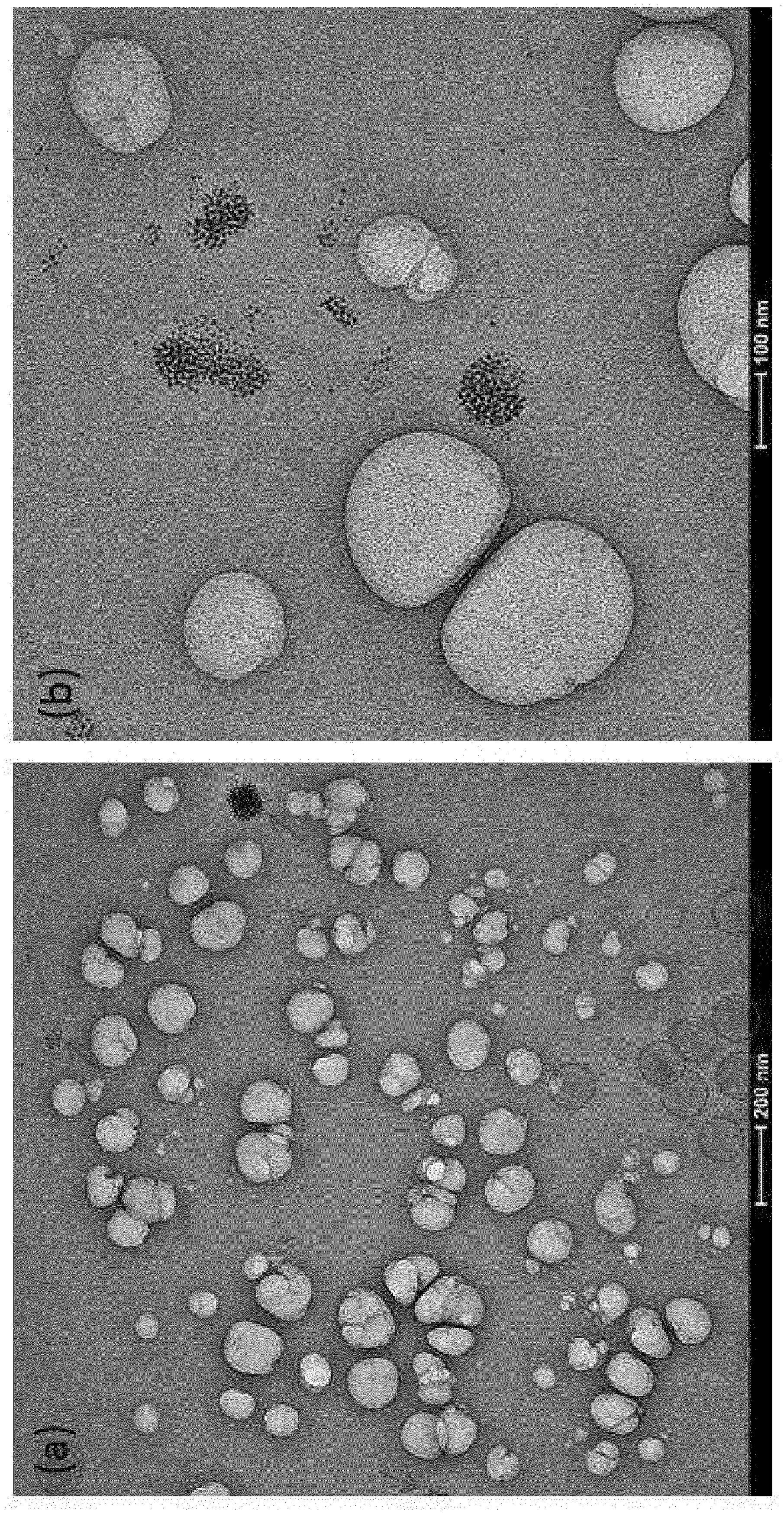

Fig. 25

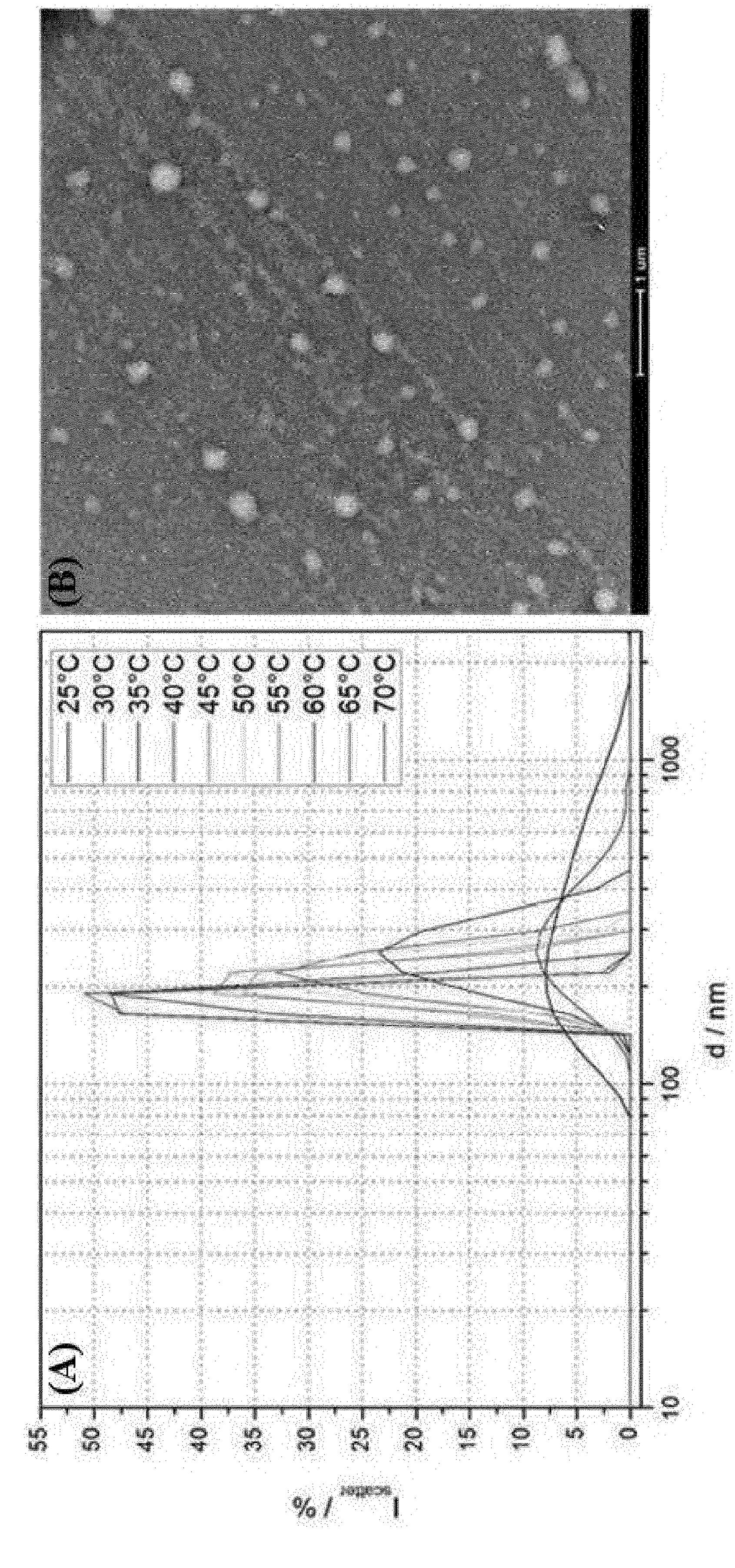

Fig. 26

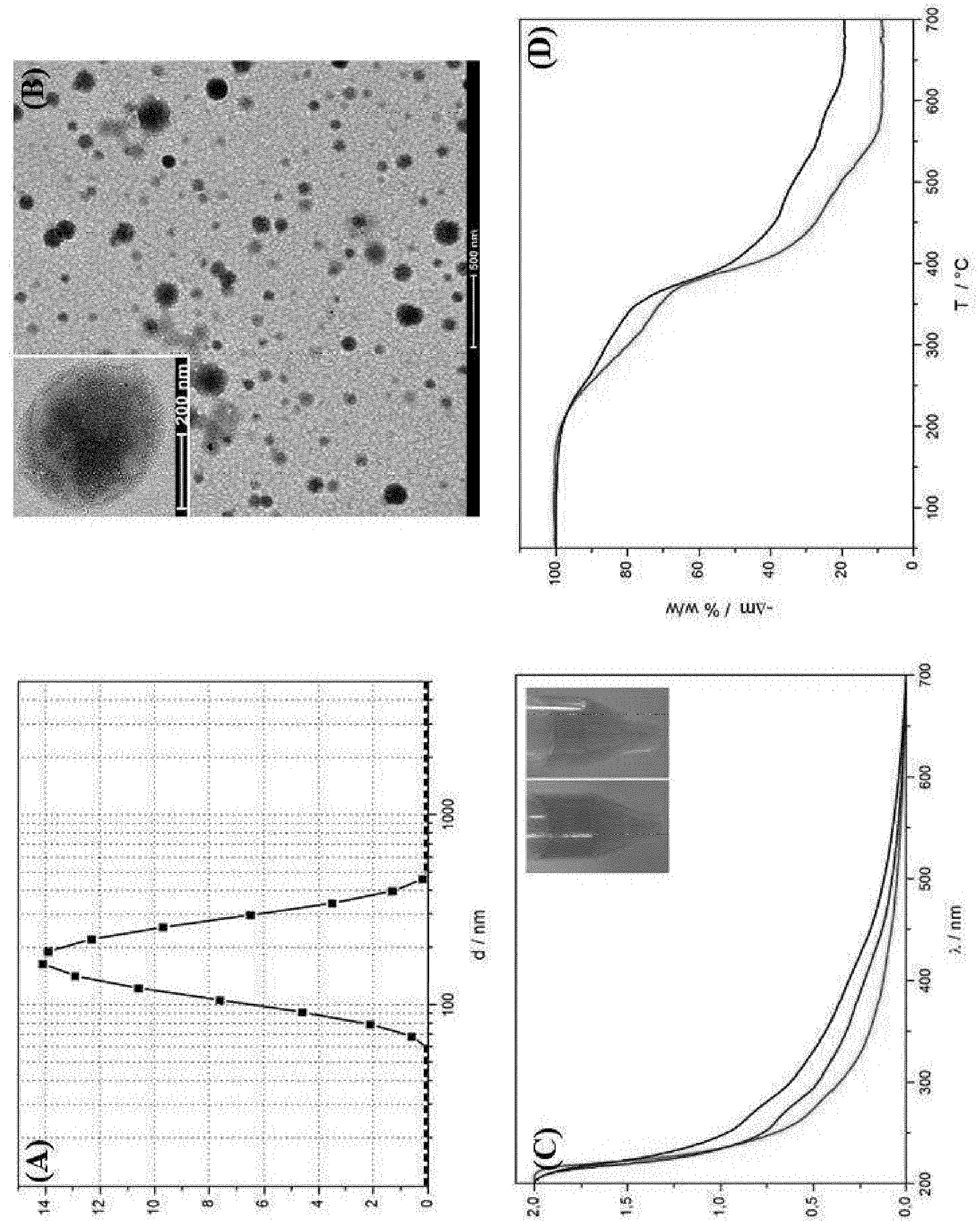

Fig. 27

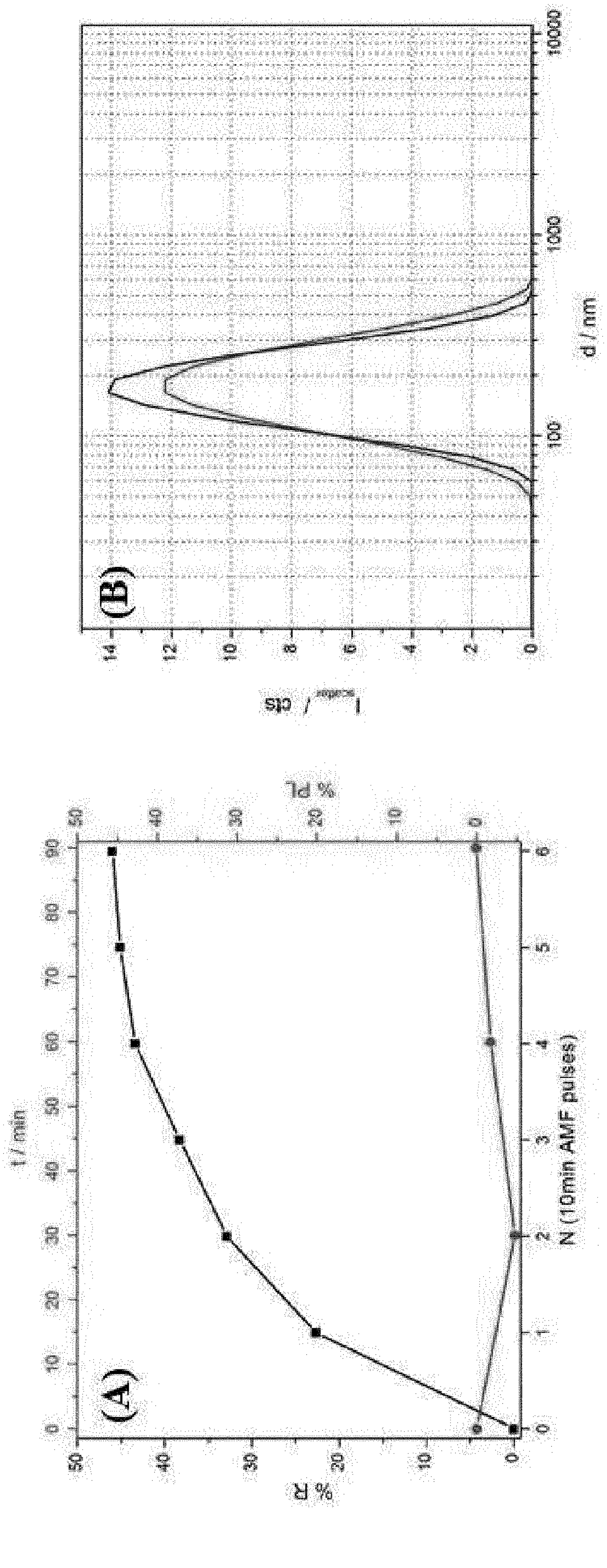

Fig. 28

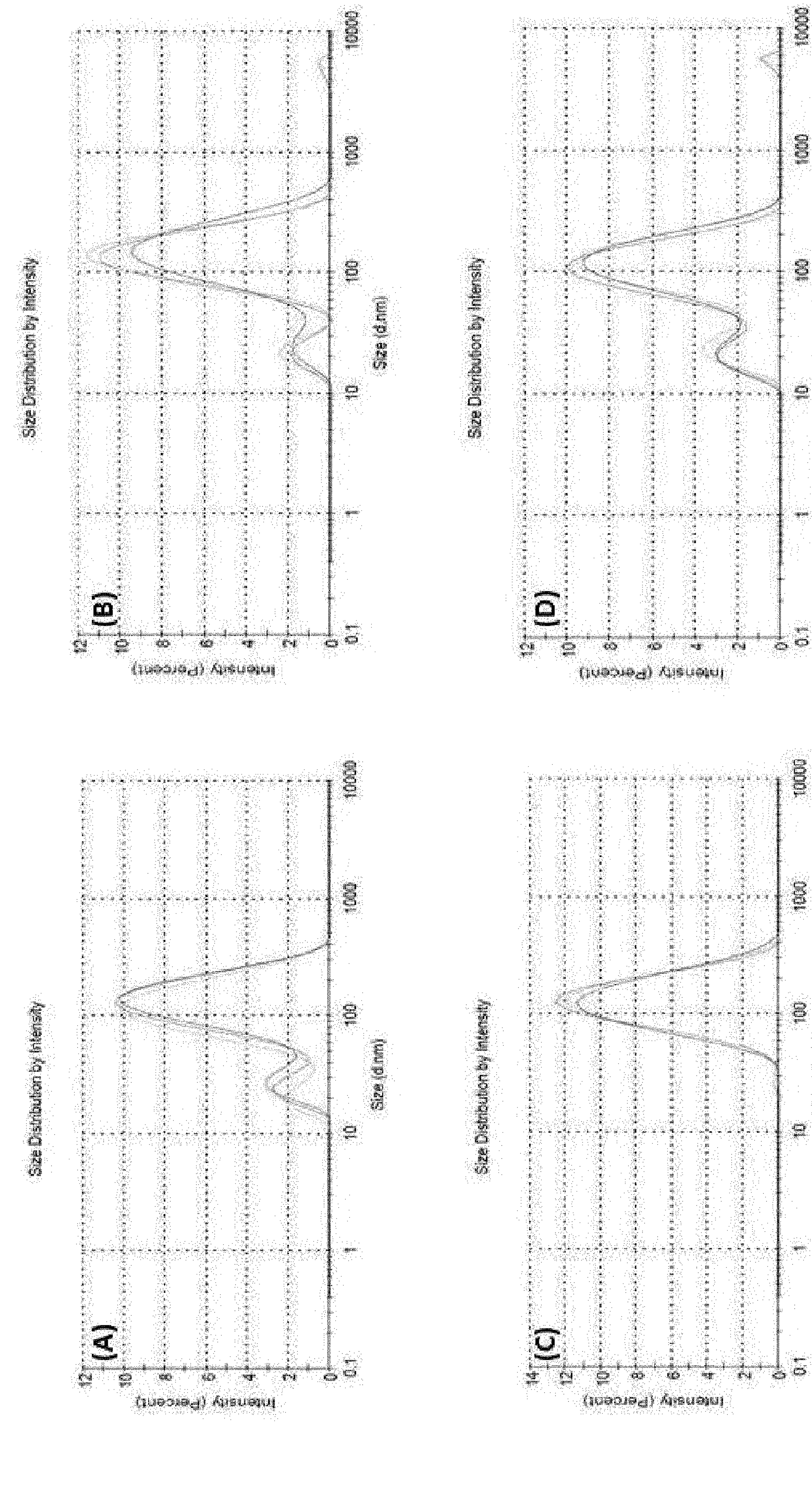
(A)
(B)
(C)
(D)
Size Distribution by Intensity
Intensity (Percent)
Size (d.nm)

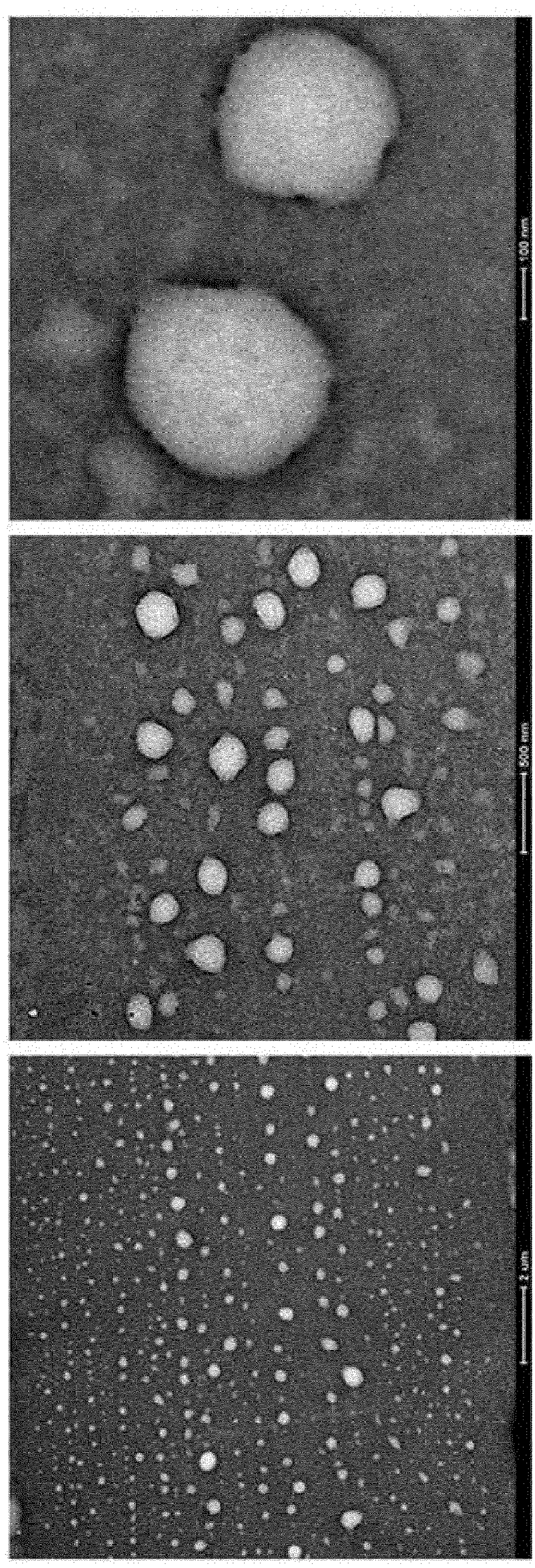


Fig. 29

Fig. 30

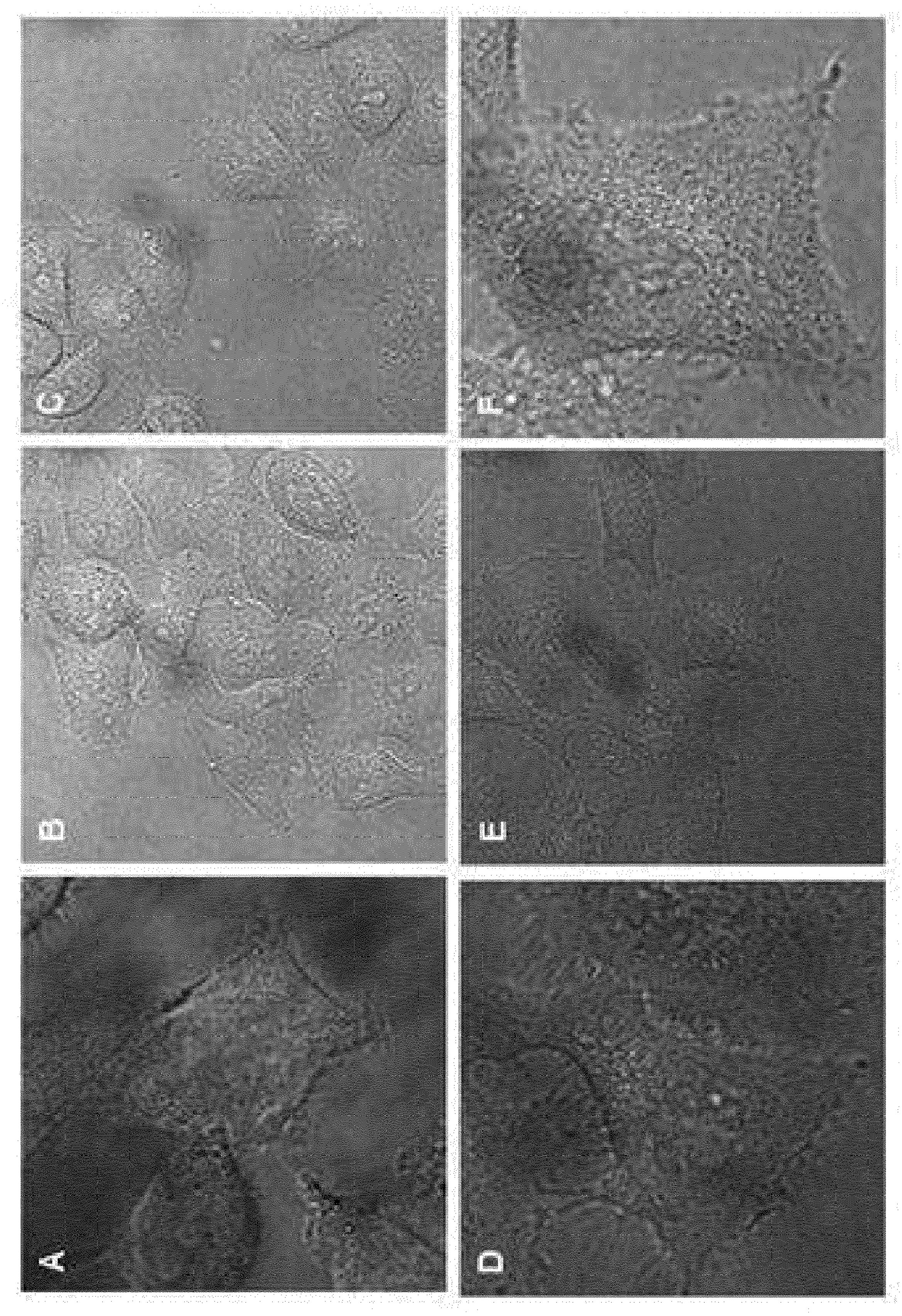

Fig. 31

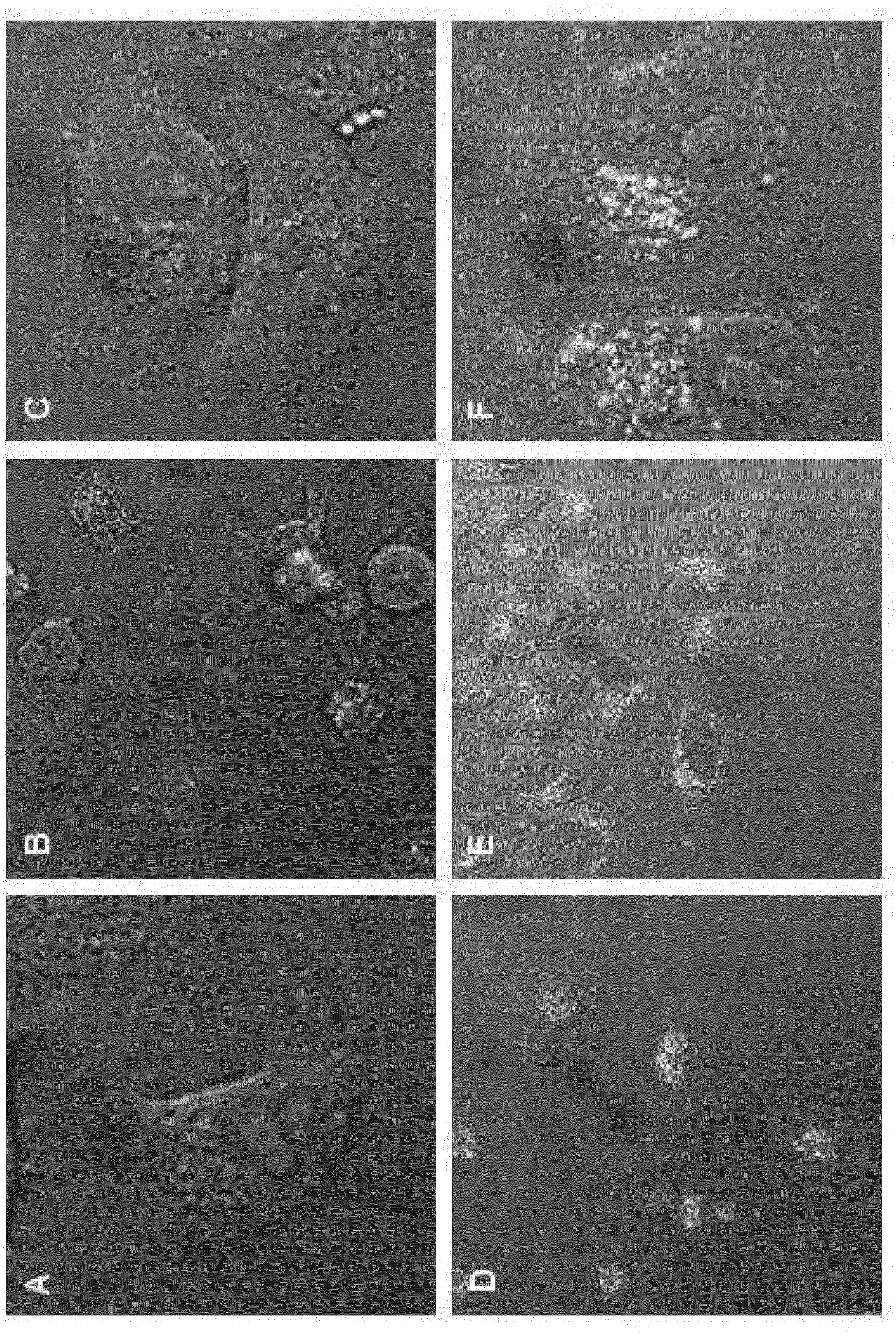

Fig. 32

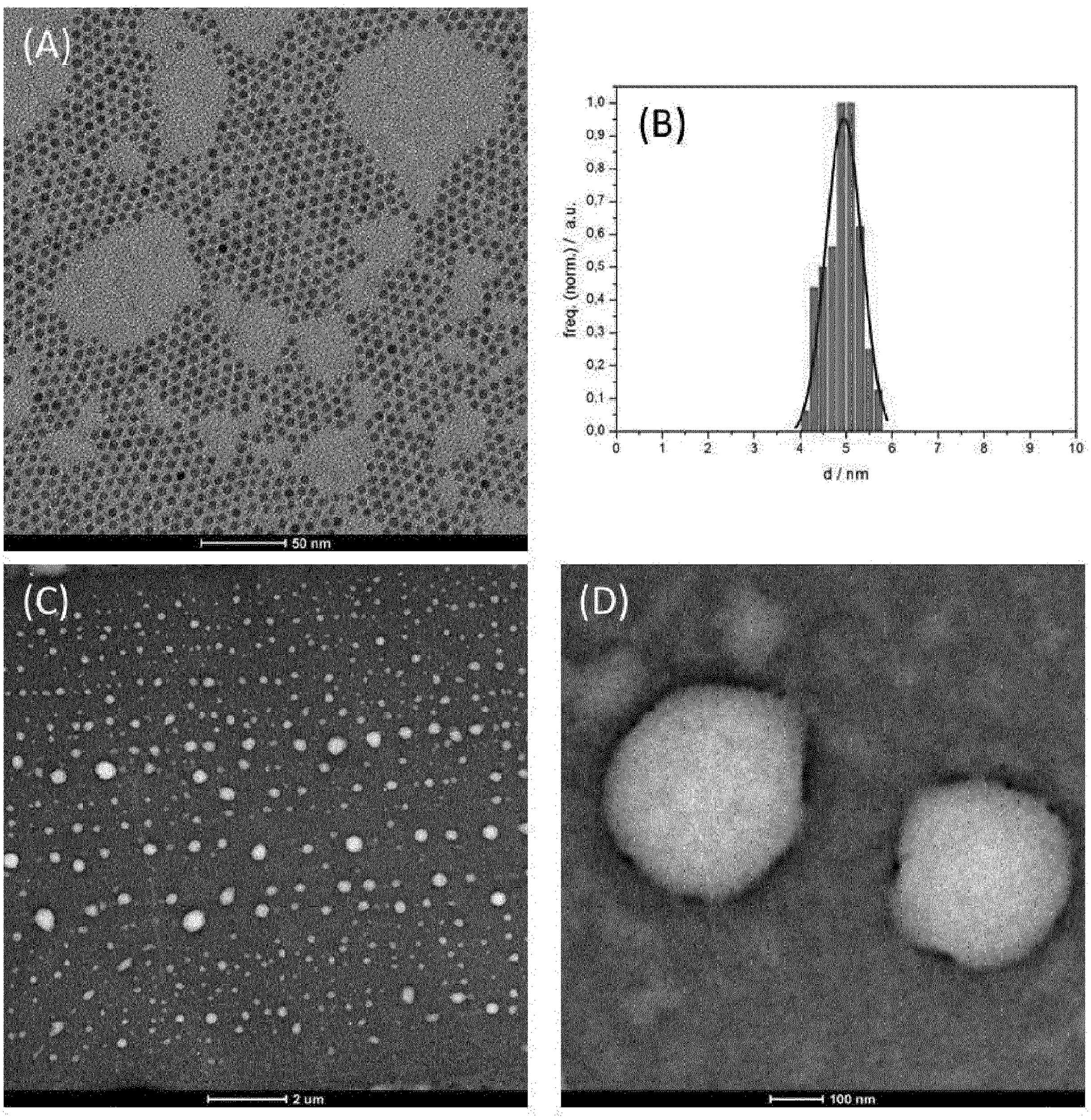
(A)
50 nm
(B)
freq. (norm.) / a.u.
1,0
0,9
0,8
0,7
0,6
0,5
0,4
0,3
0,2
0,1
0,0
0
1
2
3
4
5
6
7
8
9
10
d / nm
(C)
2 um
(D)
100 nm

Fig. 33

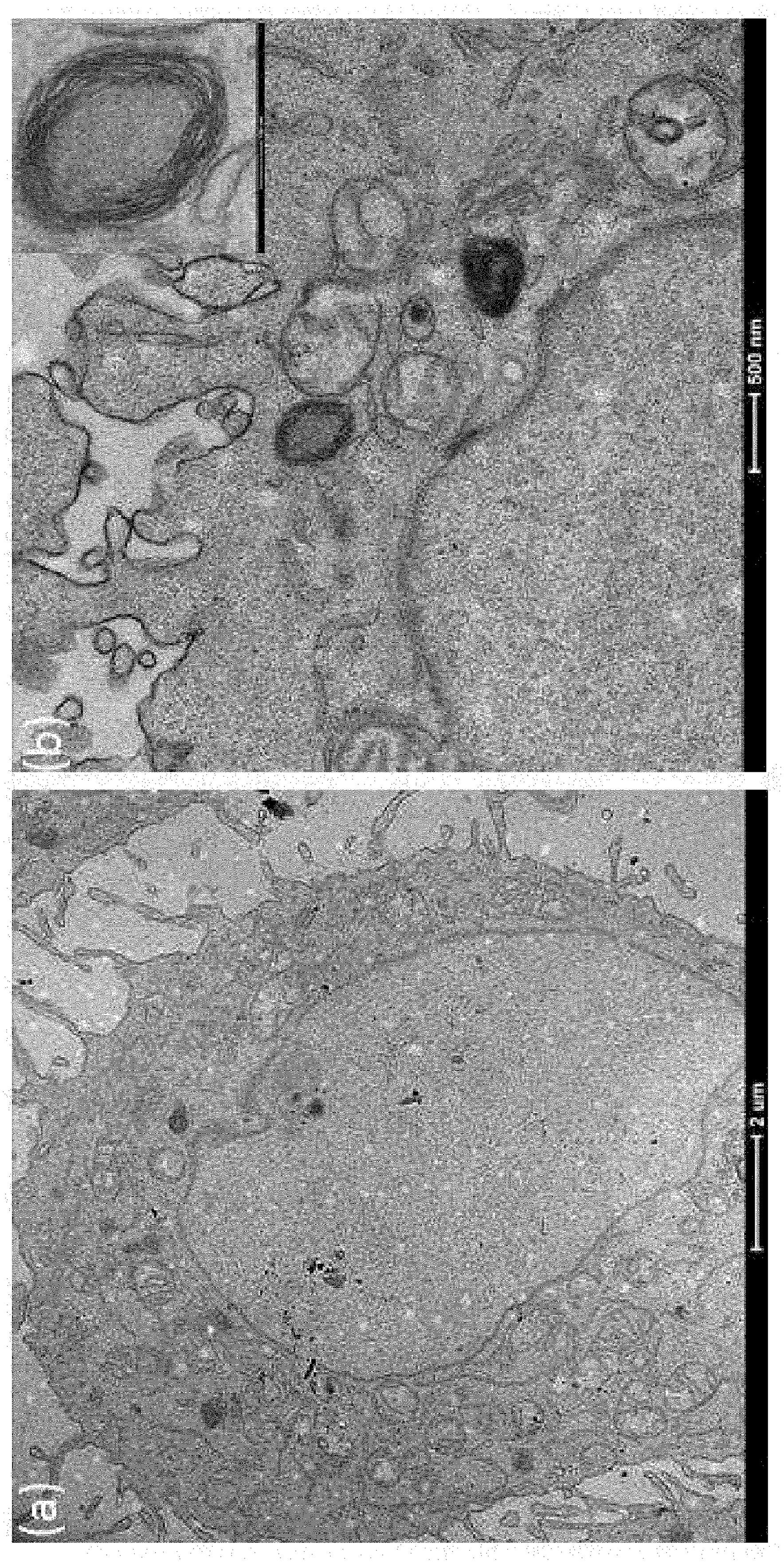

Fig. 34

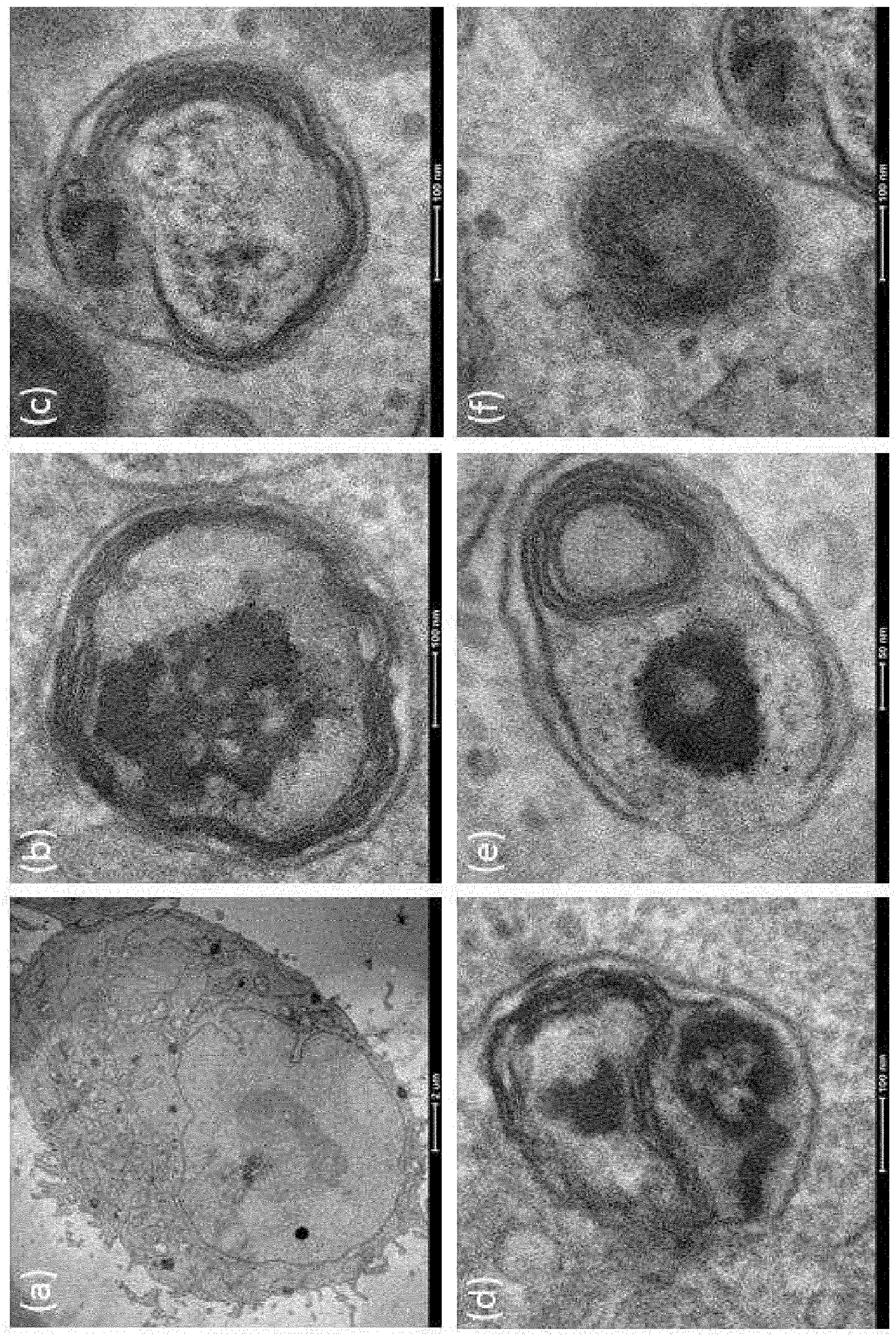

Fig. 35

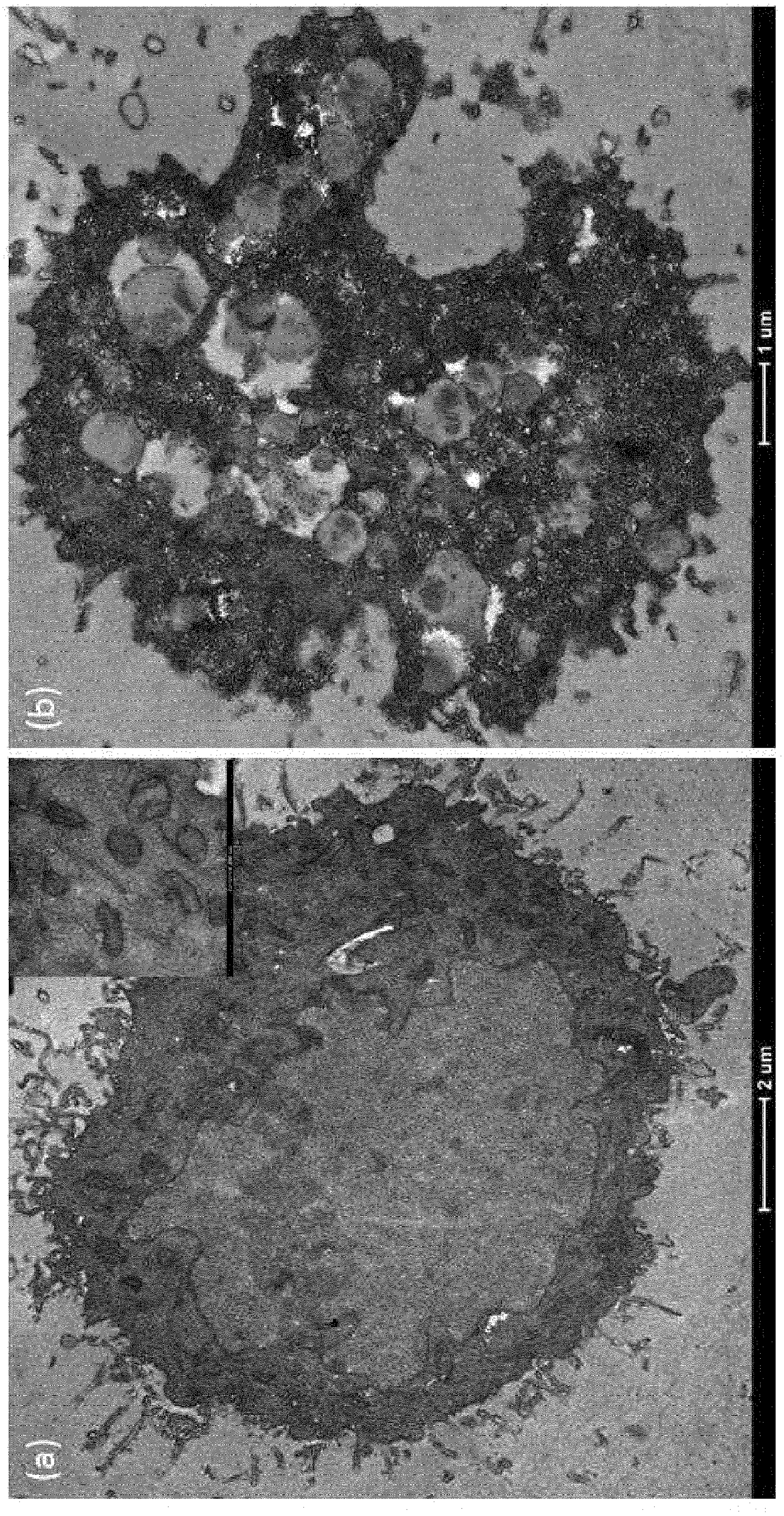

Europäisches Patentamt
European Patent Office
Office européen des brevets

# EUROPEAN SEARCH REPORT

Application Number
EP 15 19 2570

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ROBERT J. HICKEY ET AL: "Size-Controlled Self-Assembly of Superparamagnetic Polymersomes", ACS NANO, vol. 8, no. 1, 28 January 2014 (2014-01-28), pages 495-502, XP55264257, US ISSN: 1936-0851, DOI: 10.1021/nn405012h * fig. 1-3; par. "Self-assembly" on p. 500 * ----- | 12-15 | INV. A61K9/127 |
| X | CHARLES SANSON ET AL: "Doxorubicin Loaded Magnetic Polymersomes: Theranostic Nanocarriers for MR Imaging and Magneto-Chemotherapy", ACS NANO, vol. 5, no. 2, 22 February 2011 (2011-02-22), pages 1122-1140, XP55263931, US ISSN: 1936-0851, DOI: 10.1021/nn102762f * table 1; first par. on p. 1124; par. "Experimental details" on p. 1134 * ----- | 1-15 | TECHNICAL FIELDS SEARCHED (IPC) |
| | | | A61K |
| X | US 2006/099145 A1 (TAKEYAMA TOSHIHISA [JP]) 11 May 2006 (2006-05-11) * paragraph [0164] - paragraph [0166]; claim 1; table 2 * ----- | 1-7,9-15 | |
| X | WO 2011/147926 A2 (REIMHULT ERIK [AT]; AMSTAD ESTHER [CH]; TEXTOR MARCUS [CH]) 1 December 2011 (2011-12-01) * example 4 * ----- | 12-15 | |

The present search report has been drawn up for all claims

1

| Place of search | Date of completion of the search | Examiner |
|---|---|---|
| The Hague | 12 April 2016 | Frelichowska, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 19 2570

This annex lists the patent family members relating to the patent documents cited in the above-mentioned European search report. The members are as contained in the European Patent Office EDP file on
The European Patent Office is in no way liable for these particulars which are merely given for the purpose of information.

12-04-2016

| Patent document cited in search report | Publication date | Patent family member(s) | Publication date |
|---|---|---|---|
| US 2006099145 A1 | 11-05-2006 | AU 2005303251 A1 | 18-05-2006 |
| | | EP 1810688 A1 | 25-07-2007 |
| | | JP 5082446 B2 | 28-11-2012 |
| | | US 2006099145 A1 | 11-05-2006 |
| | | US 2008260648 A1 | 23-10-2008 |
| | | US 2009238764 A1 | 24-09-2009 |
| | | WO 2006051732 A1 | 18-05-2006 |
| WO 2011147926 A2 | 01-12-2011 | EP 2575896 A2 | 10-04-2013 |
| | | WO 2011147926 A2 | 01-12-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2002103517 A1
- WO 2006072943 A
- US 2007154397 A
- US 6251365 B
- WO 2007021236 A
- US 2009004258 A
- WO 2012001577 A
- WO 2011147926 A
- EP 2015068253 W, Bixner


### Non-patent literature cited in the description


- *Langmuir,* 2015, vol. 31, 9198-9204
- **KITA-TOKARCZYK et al.** *Polymer,* 2005, vol. 46, 3540-3563
- **SCHULZ et al.** *Soft Matter,* 2012, vol. 8, 4849
- **BIXNER et al.** *Langmuir,* 2015, vol. 31, 9198-9204
- **AMSTAD et al.** *Nano Lett.,* 2011, vol. 11, 1664-1670
- **SHAN et al.** *Macromolecules,* 2009, vol. 42, 2696
- **ROTH et al.** *Macromolecules,* 2008, vol. 41, 8316
- **DORN et al.** *Macromol. Biosci.,* 2011, vol. 11, 514